# EUROPEAN PATENT APPLICATION

(11) **EP 4 843 974 A2**
(43) Date of publication of application: **25.11.2026**
(21) Application number: 26199732.4
(22) Date of filing: 09.07.2024
(51) Int. Cl.: C07C 17/25

(54) **A PROCESS TO PRODUCE 1252ZC FROM 1230XA OR 252DC AND COMPOSITIONS THEREOF**

(30) Priority: 17.07.2023 US 202363527144 P
(62) Document number(s) of the earlier application(s) in accordance with Art. 76 EPC: 24748207.8 / 4 747 225
(71) Applicant: The Chemours Company FC, LLC, Wilmington, Delaware 19801 (US)
(72) Inventor: SUN, Xuehui, Kennett Square, 19348 (US); JACKSON, Andrew, Newark, 19711 (US); LOUSENBERG, Robert Daniel, Wilmington, 19810 (US)
(74) Representative: Mewburn Ellis LLP

(57) **Abstract**

Provided herein are processes for producing difluoroolefins, compositions and uses thereof, such difluoroolefins including HFO-1252zc.

## Description

The present invention is directed to processes for producing difluoroolefins, as well as to compositions and uses thereof.

### CROSS REFERENCE TO RELATED APPLICATIONS

This application claims the benefit of priority of U.S. Provisional Application 63/527,144, filed July 17, 2023, the disclosure of which is incorporated herein by reference it its entirety.

### BACKGROUND

Many industries have been working for the past few decades to find replacements for the ozone depleting chlorofluorocarbons (CFCs) and hydrochlorofluorocarbons (HCFCs). The CFCs and HCFCs have been employed in a wide range of applications, including their use as aerosol propellants, refrigerants, cleaning agents, expansion agents for thermoplastic and thermoset foams, heat transfer media, gaseous dielectrics, power cycle working fluids, polymerization media, particulate removal fluids, carrier fluids, buffing abrasive agents, and displacement drying agents. In the search for replacements for these versatile compounds, many industries have turned to the use of hydrofluorocarbons (HFCs).

Although HFCs do not contribute to the destruction of stratospheric ozone, they contribute to the "greenhouse effect", i.e., global warming. As a result of their contribution to global warming, HFCs have come under scrutiny, and their widespread use may also be limited in the future.

This regulatory landscape is continuously evolving, taking into consideration properties beyond just ODP and GWP. More particularly, there is a need for refrigerant compositions that not only meet low ODP standards and have low global warming potentials, but that also exhibit low or no flammability, provide superior performance in a variety of applications and which meet the standards of evolving regulations.

There is a need in this art for new refrigerants that meet evolving regulations as well as provide heat transfer and refrigerant characteristics that meet or exceed the effectiveness of conventional refrigerants, as well as processes for making these refrigerants and compositions thereof.

### SUMMARY

The present invention relates to processes for producing (making) 1,1-difluoropropene (HFO-1252zc, CF₂=CHCH₃) and compositions thereof.

One embodiment of the invention disclosed herein relates to processes of making HFO-1252zc by:
(a) contacting 1,2-dichloro-1,1-difluoropropane (HCFC-252dc, CClF₂CHClCH₃) with hydrogen in the vapor phase, or
(b) contacting HCFC-252dc in the liquid phase in the presence of a metal comprising Zn or Mg,
to form a product mixture comprising HFO-1252zc.

The present invention also relates to one or more of the following processes:
(a) contacting 1,1,2,3-tetrachloro-1-propene (HCO-1230xa, CCl₂=CClCH₂Cl) and HF in the vapor phase to form a product mixture comprising 2,3-dichloro-3,3-difluoropropene (CClF₂CCl=CH₂, HCFO-1232xf), or a product mixture comprising co-produced HCFO-1232xf and 2-chloro-3,3,3-trifluoropropene (HCFO-1233xf, CF₃CCl=CH₂).
(b) contacting HCFO-1233xf and HCl to form HCFO-1232xf,
(c) contacting HCFO-1232xf and hydrogen in the liquid or vapor phase to form a product mixture comprising HCFC-252dc,
(d) contacting co-produced HCFO-1232xf and HCFO-1233xf with hydrogen in the liquid or vapor phase to form a product mixture comprising HCFC-252dc,
(e) contacting the HCFC-252dc and hydrogen in the vapor phase or with a metal in the liquid phase, wherein metal comprises Zn or Mg, to form a product mixture comprising HFO-1252zc,
(f) a combination of (a), (c) and (e),
(g) a combination of (b), (d) and (e),
(h) a combination of (c) and (e),
(i) a combination of (d) and (e), or
(j) a combination of (b), (c) and (e).

A still further embodiment disclosed herein relates to a process of making HFO-1252zc comprising the following steps:
(a) contacting HCO-1230xa and HF to form a first product mixture comprising HCFO-1232xf,
(b) contacting the first product mixture comprising HCFO-1232xf and hydrogen to form a second product mixture comprising at least HCFC-252dc and/or 2-chloro-1,1,1-trifluoropropane (HCFC-253db, CF₃CHClCH₃), and
(c) subjecting the second product mixture comprising HCFC-252dc and/or 253db to reaction with a reactive metal to form a third product mixture comprising at least HFO-1252zc.

A still further embodiment disclosed herein relates to a process of making HFO-1252zc comprising the following steps:
(a) contacting HCFO-1232xf and hydrogen in the vapor or liquid phase to form a product mixture comprising HCFC-252dc, and
(b) subjecting the product mixture comprising HCFC-252dc to hydrodehalogenation in the vapor or liquid phase to form a final product mixture comprising HFO-1252zc.

A still further embodiment disclosed herein relates to a process of making HFO-1252zc comprising the following steps:
(a) contacting HCO-1230xa and HF to form a first product mixture comprising HCFO-1232xf and unreacted HCO-1230xa,
(b) contacting the first product mixture and hydrogen to form a second product mixture comprising HCFC-252dc and unreacted HCFO-1232xf, and
(c) subjecting the second product mixture to hydrodehalogenation for form a third product mixture comprising HFO-1252zc and unreacted HCFC-252dc.

A still further embodiment disclosed herein relates to a process of making HFO-1252zc comprising the following steps:
(a) contacting HCO-1230xa and HF to form a first product mixture comprising HCFO-1232xf and unreacted HCO-1230xa,
(b) contacting the first product mixture and hydrogen to form a second product mixture comprising HCFC-252dc and unreacted HCFO-1232xf,
(c) subjecting the second product mixture to reaction with H₂ or an active metal to form a third product mixture comprising HFO-1252zc and unreacted HCFC-252dc, and
(d) optionally separating the first, second, and/or third product mixtures,
(e) optionally recovering HCFO-1232xf prior to reaction with hydrogen,
(f) optionally recovering HCFC-252dc prior to hydrodehalogenation and optionally recycling unreacted HCO-1230xa, HCFO-1231 isomers, HCFO-1232xf and/or HCFC-252dc to increase the respective production of HCFO-1232xf, HCFC-252dc and/or HFO-1252zc.

In all process embodiments disclosed herein, product streams comprising (1) HCFO-1232xf, (2) HCFC-252dc and (3) HFO-1252zc, respectively, include additional members including unreacted precursor materials, e.g., HCO-1230xa, HCFO-1231 isomers for HCFO-1232xf, HCFO-1232xf for HCFC-252dc and HCFC-252dc for HFO-1252zc.

One embodiment disclosed herein relates to making HFO-1252zc sequentially through intermediate product mixtures (1) HCFO-1232xf and (2) HCFC-252dc, wherein each product mixture is optionally treated to a separation process to recover one of HCFO-1232xf, HCFC-253db, or HCFC-252dc, preferably one of HCFO-1232xf or HCFC-252dc, prior to being fed to the next downstream reaction.

In one embodiment, the process for making HFO-1252zc comprises producing a product mixture comprising HCFO-1232xf, optionally isolating and recovering HCFO-1232xf, and then reacting the HCFO-1232xf with hydrogen to produce HCFC-252dc, preferably in the presence of a catalyst. The process preferably further comprises converting HCFC-252dc to HFO-1252zc.

In one embodiment, the process for making HFO-1252zc proceeds through the intermediates HCFO-1233xf, HCFO-1232xf, HCFC-252dc, HCFC-253db to produce HFO-1252zc.

In certain embodiments disclosed herein, the amount of the HCFC-252dc or HFO-1252zc produced is greater than 10% based on the total amount of the composition.

Certain embodiments of the invention disclosed herein relate to compositions comprising, consisting essentially of, or consisting of at least one of HFO-1252zc, HCFO-1232xf, HFC-252dc, and one or more additional compounds selected from one HFC-263fb, HFC-253db, 1233xf, 272fb, 262fc, 152a and 1242xc.

Certain embodiments of the invention disclosed herein relates to compositions comprising, consisting essentially of, or consisting of HCFC-252dc and one or more of HCFO-1233xf, HCFO-1232xf, HFO-1243zf, HCFO-1242zf, HCFO-1242xc, HCFO-1242xf, HCFO-1241xb, HFO-1252zc, HCFO-1251zb (Z and E isomers), HCFO-1251xe, HCFO-1223xd, HCFC-262fc, HFC-263fb, HCFC-253 isomer, HCFC-253fb, HCFC-252dc, HCFC-253db, HCFC-262db, HFC-272fb, HCFC-243 isomer, propane and isopropanol.

Certain embodiments of the invention disclosed herein relate to compositions comprising, consisting essentially of, or consisting of one or more of HCFO-1233xf, HCFO-1232xf, HCO-1230xa, HCFO-1242xc, isopropanol, HCFC-252dc, HFO-1252zc, HFC-272fb, HCFC-253db, HFC-263fb, HCFC-262fc, HCFC-243 isomer, HCFO-1223xd, methane, HFO-E-1261ze, HFO-Z-1261ze, HFO-1243zf, HCFO-1242zf , HCFO-1251zb, HFC-262db, HCFC-253 isomer, HCO-1260, HCFO-1251zd, E-HFO-1241xb, Z-HFO-1241xb, HCFO-1250xd and HCFO-1251 isomer, wherein the main component comprises at least one of HCFO-1232xf, HCFO-1233xf, HFO-1252zc and HCFC-252dc. Examples of such compositions include, but are not limited to:
(a) HCFO-1233xf, HCFO-1242xc, isopropanol, HCFO-1232xf, and HCFC-252dc,
(b) HFO-1252zc, HFC-272fb, HCFO-1233xf, HCFC-253db, HCFO-1242xc, HFC-263fb, HCFC-262fc, HCFC-243 isomer, HCFO-1223xd, HCFO-1232xf, and HCFC-252dc,
(c) methane, E-HFO-1261ze, Z-HFO-1261ZE, HFO-1252zc, HFO-1243zf, HCFC-262fc, HCFO-1242zf, HCFO-1251zb, HFO-1242xf, HFC-272fb, HCFC-253db, HCFO-1232xf, HCFC-252dc, E-HFO-1241xb, Z-HFO-1241xb, and HCFC-262db,
(d) HFO-1261ze, HFO-1252zc, HFO-1243zf, HCFC-262fc, HCFO-1242zf, HCFC-253 isomer, HCFC-253fb, HCFC-252dc, and HFO-1241xb,
(e) HFO-1252zc, E-HFO-1261ze, Z-HFO-1261ze, HCO-1260, HCFO-1251zd, HCFC-252dc, E-HFO-1241xb isomer, Z-HFO-1241xb isomer, and HFO-1250xd,
(f) HFO-1243zf, HFO-1252zc, HFO-1242xf, HCFO-1242zf, HFO-1251, HCFC-253db, HCFO-1232xf, HCFC-252dc, and HFO-1241xb, or
(g) HFO-1243zf, HFC-272fb, HCFC-253 isomer, HCFO-1233xf, HFO-1251, HCFC-253db, HCFO-1232xf, HCFC-252dc, and HFO-1241xb,
wherein the main component comprises at least one of HCFO-1232xf, HCFO-1233xf, HFO-1252zc and HCFC-252dc.

Certain embodiments of the invention disclosed herein relates to compositions comprising, consisting essentially of, or consisting of one of at least two of HC-50, HFC-152a, HCFC-243 isomer, HCFC-252dc, HCFC-253, HCFC-253db, HCFC-262db, HCFC-262fc, HFC-263fb, HFC-272fb, HCFO-1223xd, HCO-1230xa, HCFO-1231 isomer (a), HCFO-1231 isomer (b), HCFO-1231 isomer (c), HCFO-1232xf, HCFO-1233xf, HCFO-1242xc, HCFO-1242xf, HCO-1250xd, E-HCFO-1251zb, Z-HCFO-1251zb HFO-1252zc, HCO-1260, E-HFO-1261ze, Z-HFO-1261ze (a), HFO-1261 isomer (b) and C₃H₇OH, wherein the main component comprises at least one of HCFO-1232xf, HCFO-1233xf, HFO-1252zc and HCFC-252dc.

Certain embodiments of the invention disclosed herein relates to compositions comprising, consisting essentially of, or consisting of HFO-1252zc which further include one or more additional members comprising hydrofluorocarbons (HFCs), hydrochlorocarbons (HCC's), hydrofluorochlorocarbons (HCFCs), hydrofluoroolefins (HFOs) and hydrofluorochloroolefins (HFCOs).

Certain embodiments of the invention disclosed herein are compositions comprising, consisting essentially of, or consisting of HFO-1252zc identified in Table 1.

A further embodiment of the invention disclosed herein is a composition comprising, consisting essentially of, or consisting of HFO-1252zc, and at least one additional member(compound), wherein the total amount of the additional members is between greater than 0 and less than about 90%, 85%, 80%, 75%, 70%, 65%, 60, 55%, 50%, 40%, 35%, 30,%, 25%, 20% or 15% GC/FID area, and all values and ranges therebetween.

A still further embodiment of the invention disclosed herein is a composition comprising, consisting essentially of, or consisting of HFO-1252zc, and at least one an additional member, wherein the total amount of the additional members or each member is between greater than 0 and less than about 50%, between greater than 0.001% and less than 50%, 40%. 30%, 20%, 10%, 9%, 8%, 7%, 6%, 5%, 4%, 3%, 2% or 1%, and all values and ranges therebetween provided the total amount of the composition is 100%.

One embodiment of the invention disclosed herein is a composition comprising, consisting essentially of, or consisting of HFO-1252zc, wherein HFO-1252zc is present in an amount greater than greater than 20%, greater than 30%, between 30% and greater than 99% and less than 100%, between greater than 30% and greater than 99.5%, between greater than 30% and greater than 99.6%, between greater than 30% and greater than 99.7%, between greater than 30% and greater than 99.8% or between greater than 30% and greater than 99.9% and all values and ranges therebetween.

Unless otherwise defined, all technical and scientific terms used herein have the same meaning as commonly understood by one of ordinary skill in the art to which this invention belongs. In case of conflict, the present specification, including definitions, will control. Although methods and materials similar or equivalent to those described herein can be used in the practice or testing of embodiments of the present invention, suitable methods and materials are described below. In addition, the materials, methods, and examples are illustrative only and not intended to be limiting.

### BRIEF DESCRIPTION OF THE DRAWINGS

**Fig. 1** illustrates a first embodiment of the invention.
**Fig. 2** illustrates second embodiment of the invention.

### DETAILED DESCRIPTION

The foregoing summary and the following detailed description and drawings are exemplary and explanatory only and are not restrictive of the invention, as defined in the appended claims. Other features and benefits of any one or more of the embodiments will be apparent from the following detailed description, drawings and from the claims.

Before addressing details of embodiments described herein, certain terms are defined or clarified as follows.

The term "hydro(halo)alkane," as used herein means a molecule containing hydrogen, carbon, and optionally fluorine and/or chlorine and/or bromine and/or iodine, with no carbon-carbon double bond (halo- fluoro, chloro, bromo, iodo). Examples are described throughout the instant specification. The term hydro(halo)alkane encompasses both alkanes and halogen substituted alkanes.

Reactors suitable for either liquid phase reactions or for vapor phase reactions can be used. In the vapor phase a heated reactor is used and the reactor is provided with suitable heat control. A number of reactor configurations are possible including packed bed tube or column reactors, operated in batch, semi-batch or continuous modes. Liquid phase reactor can be provided with suitable agitation equipment to increase contact between fluids and can be similarly operated in batch, semi-batch and continuous modes. In addition to the reactors disclosed herein, preheaters and vaporizers, heat exchangers, feed and effluent lines, units associated with mass transfer, contacting vessels (pre-mixers), distillation columns, and valving associated with reactors, heat exchangers, vessels, columns, and units that are used in the processes of various embodiments disclosed herein should be constructed of materials resistant to corrosion.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B is true (or present).

The transitional phrase "consisting essentially of" is used to define a composition, method that includes materials, steps, features, components, or elements, in addition to those literally disclosed provided that these additional included materials, steps, features, components, or elements do not materially affect the basic and novel characteristic(s) of the claimed invention, especially the mode of action to achieve the desired result of any of the processes of the present invention. The term 'consisting essentially of' occupies a middle ground between "comprising" and "consisting of."

The transitional phrase "consisting of" excludes any element, step, or ingredient not specified. If in the claim such would close the claim to the inclusion of materials other than those recited except for impurities ordinarily associated therewith. When the phrase "consists of" appears in a clause of the body of a claim, rather than immediately following the preamble, it limits only the element set forth in that clause; other elements are not excluded from the claim as a whole.

Where applicants have defined an invention or a portion thereof with an open-ended term such as "comprising," it should be readily understood that (unless otherwise stated) the description should be interpreted to also include such an invention using the terms "consisting essentially of" or "consisting of."

Also, use of "a" or "an" are employed to describe elements and components described herein. This is done merely for convenience and to give a general sense of the scope of the invention. This description should be read to include one or at least one and the singular also includes the plural unless it is obvious that it is meant otherwise.

When an amount, concentration, or other value or parameter is given as either a range, preferred range or a list of upper preferable values and/or lower preferable values, this is to be understood as specifically disclosing all ranges formed from any pair of any upper range limit or preferred value and any lower range limit or preferred value, regardless of whether ranges are separately disclosed. Where a range of numerical values is recited herein, unless otherwise stated, the range is intended to include the endpoints thereof, and all integers and fractions within the range.

As used herein GC/FID peak area correlates to the amount of a compound present as a proportion of the total area of all detected peaks. FID area% can be converted to mol% using response factors either calculated or measured. See https://www.chromatographytoday.com/news/gc-mdgc/32/breaking-news/what-is-a-response-factor/31169.

As used herein, the term "about" is meant to account for variations due to experimental error (e.g., plus or minus approximately 10% of the indicated value. ±1%, ± 2%, ± 3, ... ±10% or between ±1% of a stated value and ±10% of the stated value and all ranges therebetween). All measurements reported herein are understood to be modified by the term "about," whether or not the term is explicitly used, unless explicitly stated otherwise.

Compounds referred to in this disclosure may be referred to by code, based on fluorochemical naming convention, chemical structure and/or chemical name. For convenience and reference, selected compounds with codes, structures and chemical names are provided in Table 1.

**TABLE 1**

| **Code** | **Name-ASHRAE Designation** | **Formula** |
|---|---|---|
| HC-50 | methane | CH₄ |
| HFC-152a | 1,1,difluoroethane | CHF₂CH₃ |
| HC-270 | propane | C₃H₆ |
| HCFC-243 isomer | Dichlorotrifluoropropane isomer | C₃H₃Cl₂F₃ |
| HCFC-252dc | 1,2-dichloro-1,1-difluoropropane | CClF₂CHClCH₃ |
| HCFC-253 | chlorotrifluoropropane | C₃H₄ClF₃ |
| HCFC-253db | 2-chloro-1,1,1-trifluoropropane | CF₃CHClCH₃ |
| HCFC-262db | 2-chloro-1,1-difluoropropane | CHF₂CHClCH₃ |
| HCFC-262fc | 1-chloro-1,1-difluoropropane | CClF₂CH₂CH₃ |
| HFC-263fb | 1,1,1- trifluoropropane | CF₃CH₂CH₃ |
| HFC-272fb | 1,1- difluoropropane | CHF₂CH₂CH₃ |
| HCFO-1223xd | 1,2-dichloro-3,3,3-trifluoropropene | CF₃CCl=CHCl |
| HCO-1230xa | 1,1,2,3-tetrachloro-1-propene | CCl₂=CClCH₂Cl |
| HCFO-1232xf | 2,3-dichloro-3,3-difluoropropene | CClCF₂CCl=CH₂ |
| HCFO-1233xf | 2-chloro-3,3,3-trifluoropropene | CF₃CCl=CH₂ |
| HFO-1241xb | | |
| HCFO-1242xc | 1,1-difluoropropene- 2-chloropropene | CH₃CCl=CF₂ |
| HCFO-1242zf | 3-chloro-3,3-dufluoropropene | CClF₂CH=CH₂ |
| HCFO-1242xf | 2-chloro-3,3-difluoropropene | CHF₂CCl=CH₂ |
| HCFO-1251zb | 1-chloro-1-fluoropropene | CH₃CH=CClF |
| HCFO-1251xe | 1-fluoro-2-chloropropene | CH₃CCl=CHF |
| HFO-1252zc | 1,1-difluoropropene | CH₃CH=CF₂ |
| HCO-1260 | chloropropene | C₃H₅Cl |
| E-HFO-1261ze | E-1-fluoropropene | CFH=CH-CH₃ |
| Z-HFO-1261ze | Z-1-fluoropropene | CFH=CH-CH₃ |
| C₃H₈O | isopropanol | CH₃CH(OH)CH₃ |

Some of the compounds present in the compositions of the present invention may exist as different configurational isomers or stereoisomers. The present invention is intended to include all single configurational isomers, single stereoisomers or any combination or mixture thereof. Single isomers or multiple isomers of the same compound may be used in any proportion. For example, as used herein, the term HFO-1261ze is defined to mean E-HFO-1261ze (aka HFO-E-1261ze), Z-HFO-1261ze (aka HFO-Z-1261ze) and mixtures of E-HFO-1261ze and Z-HFO-1261ze.

### Fluorination of HCO-1230xa

In some embodiments, the present invention also relates to processes of producing HCFO-1232xf or co-producing HCFO-1232xf and HCFO-1233xf, and compositions thereof.

In some embodiments, the present invention relates to methods of making HCFO-1232xf and compositions thereof. In some embodiments, the present invention relates to methods of co-producing HCFO-1232xf and HCFO-1233xf, and compositions thereof. In some embodiments, the present invention relates to methods of making HCFO-1233xf and compositions thereof.

More particularly, one embodiment of the invention disclosed herein relates to a process of contacting HCO-1230xa and HF in the vapor phase to produce a product mixture comprising HCFO-1232xf.

Another embodiment disclosed herein relates to a process of contacting HCO-1230xa and HF in the vapor phase to produce a product mixture comprising HCFO-1232xf and HCFO-1233xf.

More particularly, in some embodiments of the invention disclosed herein, there is provided a process comprising contacting HCO-1230xa and HF in the vapor phase, in the absence or presence of a catalyst, to form a product mixture comprising HCFO-1232xf. In another embodiment, the reaction of HCO-1230xa and HF may be conducted in the vapor phase, in the absence or presence of a catalyst, to form a product mixture comprising HCFO-1232xf and HCFO-1233xf. In another embodiment, the reaction of HCO-1230xa and HF may be conducted in the vapor phase in the presence of a catalyst, to form a product mixture comprising HCFO-1233xf.

Typically, a heated reactor is used for the vapor phase hydrofluorination of HCO-1230xa. A number of reactor configurations are possible including horizontal or vertical orientation of the reactor as well as the sequence of reaction of the HCO-1230xa with HF. In one embodiment of the invention, the HCO-1230xa may be initially vaporized and fed to the reactor as a gas.

In another embodiment of the invention, HCO-1230xa may be contacted with HF in a pre-reactor prior to reaction in the vapor-phase reactor. In one embodiment, the pre-reactor may be empty. In another embodiment, the pre-reactor is filled with a suitable packing such as nickel-based alloys such as Hastelloy^{®}, nickel-chromium alloys commercially available from Special Metals Corp. under the trademark Inconel^{®} (hereinafter Inconel^{®}) or nickel-copper alloys commercially available from Special Metals Corp. (New Hartford, N.Y.) under the trademark Monel^{®} or other nickel alloy turnings or wool, or other material inert to HCl and HF which allows efficient mixing of HCO-1230xa and HF vapor.

In some embodiments, a diluent gas is used as a carrier gas for HCO-1230xa. In one embodiment, the carrier gas is an inert gas selected from, for example, nitrogen, argon, helium or carbon dioxide. In one embodiment, the carrier gas is a hydrofluorocarbon, such as HFC-263fb. In some embodiments, the carrier gas is mixed and vaporized with the HCO-1230xa and HF in the pre-reactor.

In one embodiment, the HCO-1230xa is vaporized, optionally in the presence of HF, and fed to a pre-reactor or to a vapor-phase reactor along with HF.

In some embodiments, the molar ratio of HF to HCO-1230xa for the vapor-phase reaction is from about 10:1 to about 60:1, preferably from about 15:1 to about 50:1, or more preferably about 20:1 to about 40:1.

Suitable temperatures for the vapor-phase reaction are from about 150°C to about 350°C, preferably from about 180°C to about 300°C, most preferably from about 250°C, about 260°C, about 270°C, about 280°C, about 290°C, about 300°C, up to about 320°C, about 330°C, about 340°C or about 350°C. In certain embodiments, vapor phase contact of HCO-1230xa and HF is conducted at a temperature of about 150°C, about 155°C, about 160°C, about 165°C, about 170°C, about 175°C, about 185°C, about 195°C, about 200°C, about 205°C, about 210°C, about 215°C, about 220°C, about 225°C, about 230°C, about 235°C, about 240°C, about 245°C, about 250°C, about 255°C, about 260°C, about 265°C, about 270°C, about 275°C, about 280°C, about 285°C, about 290°C, about 295°C, about 300°C, about 305°C, about 310°C, about 315°C, about 320°C, about 325°C, about 330°C, about 335°C, about 340°, about 345°C or about 350°C where about is ±1% of the stated temperature.

Suitable reactor pressures for the vapor-phase reactor may be from about 0 to about 200 psig, preferably about 20 to about 100 psig, more preferably about 30 to about 80 psig. Suitable reaction times may vary from about 5 to about 100 seconds, preferably from about 10 to about 80 seconds, more preferably from about 15 to about 60 seconds.

In one embodiment, the vapor-phase fluorination of HCO-1230xa to form HCFO-1232xf is carried out in the absence of a catalyst. In one embodiment, the vapor-phase fluorination of HCO-1230xa to co-produce HCFO-1232xf and HCFO-1233xf is carried out in the absence of a catalyst, preferably at a high HCFO-1232xf:HCFO-1233xf ratio.

In one embodiment, the vapor-phase fluorination of HCO-1230xa is carried out in the presence of a catalyst, to co-produce HCFO-1232xf and HCFO-1233xf at a high HCFO-1232xf:HCFO-1233xf ratio. More particularly, the HCFO-1232xf:HCFO-1233xf ratio is greater than 1:1. The HCFO-1232xf may then be isolated and hydrogenated to form HCFC-252dc, which in turn may be converted to HFO-1252zc, or the co-produced HCFO-1232xf and HCFO-1233xf may be hydrogenated to form HCFC-252dc and HCFC-253db, which in turn may be converted to HFO-1252zc, as disclosed herein.

The catalyst for the reaction to co-produce HCFO-1232xf and HCFO-1233xf at a high HCFO-1232xf:HCFO-1233xf ratio may be, for example, a supported or unsupported partially fluorinated metal oxide (e.g., fluorinated Al₂O₃), nickel-based alloy or nickel-chromium-based alloy (e.g., Hastelloy^{®} or Inconel^{™} packing).

In one embodiment, the vapor-phase fluorination of HCO-1230xa is carried out in the presence of a catalyst, to co-produce HCFO-1232xf and HCFO-1233xf at a low HCFO-1232xf:HCFO-1233xf ratio. More particularly, the HCFO-1232xf:HCFO-1233xf ratio is less than or equal to 1:1. The HCFO-1233xf may then be hydrohalogenated to form HCFO-1232xf, which may then be hydrogenated to form HCFC-252dc, which in turn may be converted to HFO-1252zc, as disclosed herein.

In some process embodiments disclosed herein relating to contacting HCO-1230xa and HF to produce HCFO-1232xf or HCFO-1233xf or to co-produce HCFO-1232xf and HCFO-1233xf at a low HCFO-1232xf:HCFO-1233xf ratio, the contacting is conducted in the vapor phase in the presence of a metal oxide catalyst which has been partially activated with HF. The metal is selected from one of chromium, iron, cobalt, nickel, ruthenium, rhodium, osmium, iridium, zinc and manganese, which has been partially fluorinated. The partially fluorinated metal oxide can be unsupported to support on alumina or carbon.

Examples of a suitable catalyst include a partially fluorinated catalyst such as chromium, aluminum, cobalt, manganese, nickel, zinc and iron oxides or their halides, including but not limited to Cr₂O₃, CrCl₃/C, Cr₂O₃/Al₂O₃, Cr₂O₃/AlF₃, Cr₂O₃/carbon, CoCl₂/Cr₂O₃/Al₂O₃, NiCl₂/Cr₂O₃/Al₂O₃, CoCl₂/AlF₃, Zn/Al₂O₃, or NiCl₂/AlF₃.

Optionally, the catalysts described above can be pretreated with HF. This pretreatment can be accomplished, for example, by placing the catalyst in a suitable container and thereafter, passing HF over the catalyst. In one embodiment, such container can be the reactor used to perform the hydrofluorination reaction. In one embodiment, the pretreatment time is from about 15 to about 300 minutes, and the pretreatment temperature is from about 180°C to about 450°C.

In certain embodiments, the vapor phase contacting of HCO-1230xa and HF is conducted at a temperature ranging from 150°C to 350°C in the absence of a catalyst.

In certain embodiments, the vapor phase contacting of HCO-1230xa and HF is conducted at a temperature ranging from 150°C to 350°C in the presence of a catalyst, more preferably in the presence of a metal oxide catalyst, preferably that has been activated with HF.

In certain embodiments, vapor phase contact of HCO-1230xa and HF is conducted at a temperature ranging from 150°C to 350°C, in the presence of a metal oxide catalyst, preferably that has been partially activated with HF starting at 180°C and ending at 450°C.

In all process embodiments disclosed herein, product streams comprising HCFO-1232xf, HCFO-1233xf or co-produced HCFO-1232xf and HCFO-1233xf also include the HCO-1230xa precursor starting material.

In some embodiments, under these conditions, for example, a mixture of HF and HCO-1230xa is converted by the vapor-phase fluorination process in the absence of a catalyst or in the presence of a catalyst such as fluorinated Al₂O₃, Hastelloy^{®} packing or Inconel^{™} packing to a reaction mixture comprising HCl and a composition comprising HCFO-1232xf and one or more additional compounds selected from HCFO-1233xf, HCO-1230xa, HCFO-1231 isomers and C₄H₆ClF. In some embodiments, the HCFO-1232xf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

In some embodiments, under these conditions, for example, a mixture of HF and HCO-1230xa is converted by the vapor-phase fluorination process in the presence of a catalyst, such as a partially fluorinated catalyst including but not limited to chromium, aluminum, cobalt, manganese, nickel, zinc and iron oxides or their halides, including but not limited to Cr₂O₃, CrCl₃/C, Cr₂O₃/Al₂O₃, Cr₂O₃/AlF₃, Cr₂O₃/carbon, CoCl₂/Cr₂O₃/Al₂O₃, NiCl₂/Cr₂O₃/Al₂O₃, CoCl₂/AlF₃, Zn/Al₂O₃, or NiCl₂/AlF₃, to a reaction mixture comprising HCl and a composition comprising HCFO-1233xf and one or more additional compounds selected from HCFO-1232xf, HCO-1230xa, HCFO-1231 isomers and C₄H₆ClF. In some embodiments, the HCFO-1233xf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

Certain embodiments of the invention disclosed herein relate to compositions comprising, consisting essentially of, or consisting of at least 30% percent HCFO-1232xf based on the total amount of the composition and including one or more additional compounds selected from one of HFCO-1233xf, HCO-1230xa. Such compositions may be formed by the vapor phase fluorination of HCO-1230xa disclosed herein.

### Hydrochlorination of HCFO-1233xf

A further embodiment of the invention discloses herein relates to a process of making HCFO-1232xf by contacting HCFO-1233xf and HCl, in the vapor phase, in the presence of a catalyst, to form a product mixture comprising HCFO-1232xf.

In one embodiment disclosed herein, the HCFO-1233xf starting material may be obtained from any commercially available source.

In another embodiment, the process comprises providing or obtaining the HCFO-1233xf starting material using the above-discussed process of hydrofluorination of HCO-1230xa, or using any other known method for the production of HCFO-1233xf, such as the methods disclosed in at least U.S. Patent Pub. No. 2014/0309462 or U.S. Patent Pub. No. 20210317055, the disclosure of each of which is incorporated herein by reference in its entirety.

In one embodiment disclosed herein, the process of making HCFO-1232xf relates to a process of providing HCFO-1233xf which is produced by contacting HCO-1230xa and HF in the vapor phase, and contacting the HCFO-1233xf with HCl in the vapor phase in the presence of a catalyst to form a product mixture comprising HCFO-1232xf.

The process comprises making HCFO-1232xf and compositions thereof, by contacting the HCFO-1233xf with HCl in the vapor phase, in the presence of a catalyst.

In some embodiments, the molar ratio of HCl to HCFO-1233xf for the hydrochlorination reaction is from about 10:1 to about 40:1, preferably from about 15:1 to about 35:1, or more preferably about 20:1 to about 20:1.

Suitable temperatures for the vapor phase hydrochlorination reaction of HCFO-1233xf are from about 180°C to about 350°C, preferably from about 200°C to about 320°C, more preferably from about 220°C to about 300°C.

Suitable reactor pressures for the hydrochlorination reaction may be from about 0 to about 200 psig, preferably about 20 to about 100 psig, more preferably about 30 to about 80 psig. Suitable reaction times may vary from about 5 to about 100 seconds, preferably from about 10 to about 80 seconds, more preferably from about 15 to about 60 seconds.

In one embodiment, the vapor phase hydrochlorination of HCFO-1233xf to form HCFO-1232xf is carried out in the presence of a catalyst.

For example, the catalyst may be selected from a fluorinated transition metal oxide or a transition metal halide catalyst. The catalyst may either be unsupported, or supported on a support such as activated carbon, graphite or alumina. The transition metals can be Cr, Ni, Co or combination of them which may further contains a co-catalyst selected from manganese or zinc.

In some embodiments, under these conditions, for example, a mixture of HF and HCFO-1233xf is converted by the hydrochlorination process in the presence of a catalyst to a reaction mixture comprising HCl and a composition comprising HCFO-1232xf and one or more additional compounds selected from HCFO-1233xf, HCO-1230xa, HCFO-1231 isomers and HCO-1230 isomers. In some embodiments, the HCFO-1232xf constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

Certain embodiments of the invention disclosed herein relate to compositions comprising, consisting essentially of, or consisting of at least 30% percent HCFO-1232xf based on the total amount of the composition and including one or more additional compounds selected from one of HFCO-1233xf, HCO-1230xa. Such compositions may be formed by the hydrochlorination of HCFO-1233xf disclosed herein.

### Hydrogenation of HCFO-1232xf to form HCFC-252dc

In some embodiments, the present invention also relates to making HCFC-252dc and compositions thereof. More particularly, in one embodiment, the present invention relates to a process for making HCFC-252dc by contacting HCFO-1232xf and hydrogen, in the liquid phase or vapor phase, and in the presence of a catalyst, to form a product mixture comprising HCFC-252dc.

In some embodiments, the molar ratio of H₂ to HCFO-1232xf for the vapor phase hydrogenation reaction, preferably vapor phase catalytic hydrogenation reaction, is from about 1:1 to about 15:1, preferably from about 1.5:1 to about 10:1.

That is, in certain embodiments, the reactants are exposed to sufficient temperature to effect hydrogenation, preferably vapor phase catalytic hydrogenation, of HCFO-1232xf to HCFC-252dc. In certain embodiments disclosed herein, hydrogenation of HCFO-1232xf to HCFC-252dc is conducted at a temperature between about 20°C to about 200°C, or about 20°C to about 150°C, preferably about 90°C to about 200°C, or about 40°C to about 120°C, or about 60°C to about 140°C, or greater than about 20°C, 30°C, 40°C, 50°C, 60°C, 70°C, 80°C, 90°,100°C, 110°C, 120°C, 130°C, 140°C, 160°C or 180°C to less than about 200°C.

In certain embodiments, the reactants are exposed to sufficient temperature to catalytically hydrogenate HCFO-1232xf to HCFC-252dc in the vapor phase, which temperature is selected from about 20°C, about 30°C, about 40°C, about 50°C, about 60°C, about 70°C, about 80°C, about 90°C, about 100°C, about 110°C, about 120°C, about 130°C to about 150°C.

In certain embodiments, reactants are exposed to sufficient temperature for vapor phase catalytic hydrogenation of HCFO-1232xf to HCFC-252dc, wherein the temperature is selected from between about 20°C, about 30°C, about 40°C, about 50°C, about 60°C, about 70°C, about 80°C, about 90°C, about 100°C, about 110°C, about 120°C or about 140°C and about 200°C.

In some embodiments, for vapor phase catalytic hydrogenation of HCFO-1232xf to HCFC-252dc, the reaction pressure is between atmospheric pressure and ≤ 300 psig, preferably between ≥ 20 psig and ≤100 psig.

In some embodiments, pressures for vapor phase catalytic hydrogenation of HCFO-1232xf to HCFC-252dc range from atmospheric to ≤ 300 psig, preferably ≥ 0 to ≤ 200 psig.

In some embodiments for the vapor phase hydrogenation of HCFO-1232xf to HCFC-252dc, the catalyst comprises a transition metal, preferably on a support. In some embodiments, the metal of the catalyst is selected from one of Pd, Pt, Cu, Ni, alone or in combination. In some embodiments, the support comprises one of carbon, silicon carbide (SiC) or alumina. In some embodiments, the catalyst loading ranges from about 0.005 wt.% to about 3.0 wt.%, preferably from about 0.01 wt.% to about 2 wt.%, more preferably from about 0.015 wt.% to about 0.5 wt.%.

In one embodiment, the Pd catalyst for hydrogenation of HCFO-1232xf to HCFC-252dc is activated by H₂ at an elevated temperature of greater than about 40°C before use.

In some embodiments, the vapor phase hydrogenation reaction of HCFO-1232xf to HCFC-252dc is conducted in the presence of a catalyst with loading ranging from about 0.005 wt.% to about 3.0 wt.%, preferably from about 0.01 wt.% to about 2 wt.%, more preferably from about 0.015 wt.% to about 0.5 wt.%, of a transition metal, such as Pd, Pt, Cu, Ni, or combinations thereof, on a support of carbon, silicon carbide or alumina, at a temperature of between about 20°C and about 150°C, and at a pressure of between atmospheric pressure and ≤ 300 psig.

In certain embodiments, reactants are exposed to sufficient temperature to convert HCFC-252dc to HFO-1252zc in the vapor phase at a temperature of about 160°C to about 300°C, and more preferably about 200°C to about 260°C, preferably about 200°C to about 240°C, and at a pressure of between ≥ 0 to ≤ 300psig, optionally in the presence of a diluent.

In certain embodiments disclosed herein, the process relates to making HCFC-252dc comprising contacting HCFO-1232xf in the vapor phase with hydrogen in the presence of a hydrogenation catalyst and optionally a diluent. In some embodiments, the catalyst is selected from a Ni, Pd, Cu or Pt catalyst with a support select from alumina, carbon or SiC which is optionally activated with hydrogen at temperatures between about 20°C and about 250°C. The reaction temperature ranges from about 20°C to about 200°C, preferably about 90°C to about 200°C, or about 40°C to about 120°C, or about 60°C to about 140°C. In one embodiment, the catalyst is Pd/C, and more preferably 0.5% Pd/C to 5% Pd/C, or is Pd/Al₂O₃ and more preferably 0.02% Pd/Al₂O₃ to 0.1% Pd/Al₂O₃.

In one embodiment disclosed herein, a process relates to making HCFC-252dc comprising contacting HCFO-1232xf in the liquid phase, in the presence of hydrogen, a catalyst and optionally a solvent.

In some embodiments, the molar ratio of H₂ to HCFO-1232xf for the liquid phase hydrogenation reaction, preferably liquid phase catalytic hydrogenation reaction, is from about 1:1 to about 5:1, preferably from about 1.2:1 to about 3:1.

In certain embodiments, the reactants are exposed to sufficient temperature to effect hydrogenation, preferably liquid phase catalytic hydrogenation, of HCFO-1232xf to HCFC-252dc. In certain embodiments disclosed herein, hydrogenation of HCFO-1232xf to HCFC-252dc is conducted at a temperature between about 20°C to about 150°C, or about 40°C to about 120°C, or about 60°C to about 140°C, preferably about 60°C.

In some embodiments, for liquid phase catalytic hydrogenation of HCFO-1232xf to HCFC-252dc, the reaction pressure is between atmospheric pressure and ≤ 500 psig.

In some embodiments for the liquid phase hydrogenation of HCFO-1232xf to HCFC-252dc, the catalyst comprises a transition metal, preferably on a support. In some embodiments, the metal of the catalyst is selected from one of Pd, Pt, Cu, Ni, alone or in combination. In some embodiments, the support comprises one of carbon, silicon carbide (SiC) or alumina. In some embodiments, the catalyst loading ranges from about 0.005 wt.% to about 3.0 wt.%, preferably from about 0.01 wt.% to about 2 wt.%, more preferably from about 0.015 wt.% to about 0.5 wt.%.

In some embodiments, the solvent is selected from an alcohol, dimethyl sulfoxide (DMSO), dimethyl acetamide (DMAC), dimethyl formamide (DMF), tetrahydrofuran (THF), dioxane, or other suitable protic or aprotic organic solvents.

In some embodiments, the liquid phase hydrogenation reaction of HCFO-1232xf to HCFC-252dc is conducted in the presence of a catalyst with loading ranging from about 0.005 wt.% to about 3.0 wt.%, preferably from about 0.01 wt.% to about 2 wt.%, more preferably from about 0.015 wt.% to about 0.5 wt.%, of a transition metal, such as Pd, Pt, Cu, Ni, or combinations thereof, on a support of carbon, silicon carbide or alumina, at a temperature of between about 20°C and about 150°C, and in the presence of a solvent and at a pressure of between atmospheric pressure and ≤ 500 psig.

In certain embodiments disclosed herein, the process relates to making HCFC-252dc comprising contacting HCFO-1232xf in the liquid phase with hydrogen in the presence of a hydrogenation catalyst and a solvent. In some embodiments, the catalyst is selected from a Ni, Pd, Cu or Pt catalyst with a support select from alumina, carbon or SiC which is optionally activated with hydrogen at temperatures between about 20°C and about 250°C. The reaction temperature ranges from about 20°C to about 150°C. In one embodiment, the catalyst is Pd/C, and more preferably 0.5% Pd/C to 5% Pd/C, or is Pd/Al₂O₃ and more preferably 0.02% Pd/Al₂O₃ to 0.1% Pd/Al₂O₃. In one embodiment, the solvent is selected an alcohol, DMSO, DMAC, DMF, THF, dioxane, or other suitable protic or aprotic organic solvent.

In one embodiment, the HCFO-1232xf starting material is obtained by the hydrofluorination of HCO-1230xa and/or the hydrochlorination of HCFO-1233xf, as disclosed herein. In one embodiment, the HCFO-1232xf is separated or isolated from the reaction mixture comprising co-produced HCFO-1232xf and HCFO-1230xa by one of the above-discussed processes, and utilized as a starting material for the hydrogenation reaction to form HCFC-252dc.

Another embodiment of the invention disclosed herein relates to a process of making HCFC-252dc by contacting co-produced HCFO-1232xf and HCFO-1233xf with hydrogen in the presence of a catalyst, in the vapor or liquid phase to produce a product mixture comprising HCFC-252dc, HCFC-253db, HCFO-1232xf and HCFO-1233xf.

Under these conditions, compositions may be produced which comprise, consist essentially of, or consist of HCFC-252dc and one or more additional compounds selected from HCFO-1233xf, HCFO-1232xf, HFO-1243zf, HCFO-1242zf, HCFO-1242xc, HCFO-1242xf, HCFO-1241xb, HFO-1252zc, HCFO-1251zb (Z and E isomers), HCFO-1251xe, HCFO-1223xd, HCFC-262fc, HFC-263fb, HCFC-253 isomer, HCFC-253fb, HCFC-252dc, HCFC-253db, HCFC-262db, HFC-272fb, HCFC-243 isomer, propane and isopropanol. In some embodiments, the HCFC-252dc constitutes about 0.1 wt% to about 99.9 wt.%, or about 40 wt.% to about 99.9 wt.%, or about 90 wt.% to about 99.9 wt.%, based on the total weight of the composition, inclusive of all integers and ranges therebetween.

### Hydrodehalogenation of HCFC-252dc

In some embodiments, the present invention relates to processes for producing (making) HFO-1252zc and compositions thereof.

In certain embodiments disclosed herein relating to making HFO-1252zc by hydrodechlorination of HCFC-252dc. In some embodiments, the HCFC-252dc starting material is obtained by one of the processes disclosed herein.

In some embodiments, the hydrodechlorination of HCFC-252dc to form HFO-1252zc can be conducted in the vapor phase.

That is, in certain embodiments, the reactants are exposed to sufficient temperature to effect hydrodehalogenation, preferably vapor phase catalytic hydrodechlorination, of HCFC-252dc to HFO-1252zc. In certain embodiments disclosed herein, the hydrodechlorination reaction can be conducted at temperatures between about 160°C and about 500°C, preferably between about 250°C and about 450°C, or between about 160°C to about 300°C, and more preferably about 200°C to about 300°C, preferably about 200°C to about 260°C or preferably about 200°C to about 240°C.

In some embodiments, for vapor phase catalytic hydrodechlorination of HCFC-252dc to HFO-1252zc, the reaction pressure is between ≥ 0 to ≤ 250 psig.

In some embodiments for the vapor phase hydrodechlorination of HCFC-252dc to HFO-1252zc, the reaction is in the presence of a catalyst with or without a support. In some embodiments, the metal of the catalyst is selected from one of Cu, Au, alone or in combination. In some embodiments, the support comprises one of carbon, silicon carbide (SiC) or alumina. In some embodiments, the catalyst loading ranges from about 0.1% to about 10%, preferably from about 0.5% to about 8%. In some embodiments, the catalyst is optionally activated with hydrogen.

In some embodiments, the vapor phase hydrodechlorination of HCFC-252dc to HFO-1252zc is conducted in the presence of a catalyst with loading ranging from about 0.1% to about 10%, preferably from about 0.5% to about 8%, of a metal, such as Cu, Au, or combinations thereof, on a support of carbon, silicon carbide or alumina, at a temperature of between about 160°C and about 500°C, preferably between about 250°C and about 450°C, and at a pressure of between ≥ 0 to ≤ 250 psig, optionally in the presence of a diluent.

### Dehalogenation of HCFC-252dc using Metal

Another embodiment of the invention disclosed herein relates to processes of making HFO-1252zc by reacting HCFC-252dc in the liquid phase in the presence of a metal, preferably a reactive metal, to form a product mixture comprising HFO-1252zc.

In some embodiments, the metal comprises Zn, Mg, Cu, Fe or combinations thereof.

In some embodiments, the present invention relates to a process of reacting of HCFC-252dc in the liquid phase in the presence of a reactive metal, optionally in the presence of an aprotic solvent (i.e., in the presence or absence of an aprotic solvent), to form a product mixture comprising HFO-1252zc, optionally in the presence of a catalyst.

In some embodiments, the catalyst is selected from a metal halide, a phase transfer catalyst, zinc salts, or combinations thereof.

As used herein, phase transfer catalyst is intended to mean a substance that facilitates the transfer of ionic compounds into an organic phase from an aqueous phase or from a solid phase. The phase transfer catalyst facilitates the reaction of these dissimilar and incompatible components. While various phase transfer catalysts may function in different ways, their mechanism of action is not determinative of their utility in the present invention provided that the phase transfer catalyst facilitates the dehydrochlorination reaction.

A phase transfer catalyst as used herein is a quaternary alkylammonium salt wherein the alkyl groups are alkyl chains having from four to twelve carbon atoms. In one embodiment, the quaternary alkyl ammonium salt is a tetrabutylammonium salt. The anions of the salt can be halides such as chloride or bromide, hydrogen sulfate, or any other commonly used anion.

In another embodiment, the quaternary alkylammonium salt is trioctylmethylammonium chloride (Aliquat 336). In another embodiment, the quaternary alkylammonium salt is tetraoctylammonium chloride. In yet another embodiment, the quaternary alkylammonium salt is tetraoctylammonium hydrogen sulfate.

In some embodiments, the liquid phase dechlorination of HCFC-252dc to HFO-1252zc is conducted in the presence of a metal and a solvent, such as an alcohol, or an organic liquid such as dimethyl sulfoxide (DMSO), dimethyl acetamide (DMAC), dimethyl formamide (DMF), tetrahydrofuran (THF), pyridine, dioxane, or other suitable protic or aprotic organic solvents.

In some embodiments, for the liquid phase dechlorination of HCFC-252dc to HFO-1252zc, the vessel contents are heated to a temperature of between about 50°C and about 160°C, or about 80°C to about 160°, and more preferably in the range of about 90° to about 140°C, including but not limited to 80°C, 90°C 100°C, 110°C, 120°C, 130°C, 140°C, 150°C, or 160°C.

The reaction is conducted under autogenous pressure up to about 1000 psig.

The HCFC-252dc liquid phase dechlorination can be conducted in batch, semi-batch or continuous modes.

The HCFC-252dc liquid phase dechlorination can be conducted in the presence of an inert diluent gas.

In certain hydrogenation embodiments disclosed herein, the process relates to making HFO-1252zc in the liquid phase by contacting HCFC-252dc with a reactive metal selected from Zn or Mg. Optionally the liquid phase process is conducted in the presence of a catalyst, such as zinc salt, phase transfer catalyst or combination, and/or a suitable solvent. The liquid phase hydrogenation of HCFC-252dc can be conducted at autogenous pressures and at temperatures of between about 50°C and about 160°C.

Certain embodiments disclosed herein relate to making HFO-1252zc by liquid phase reaction of HCFC-252dc with a reactive metal. HCFC-252dc is placed in a vessel, in the presence of a reactive metal such as Zn or Mg optionally in a solvent such as acetic acid or acetic anhydride or a protic or aprotic solvent. The vessel contents are heated to a temperature of between about 80°C to about 160°, and more preferably in the range of about 90° to about 140°C, under autogenous pressure. This liquid phase process can be conducted in batch, semi-batch or continuous modes.

In certain embodiments reactants are exposed to sufficient temperature to effect conversion of HCFC-252dc to HFO-1252zc in the presence of an alcohol and a zinc salt such as zinc chloride, zinc acetate, or a phase transfer catalyst such as TBAB at temperatures of about 80°C, 90°,100°C, 110°C, 120°C, 130°C, 140°C, 160°C, or between 80°C or 90°C and 110°C or 120°C, preferably about 100°C, for a period of between about 1 and about 25 hours, preferably about 20 hours. In certain embodiments, the alcohol is selected from methanol, ethanol, propanol, or isopropanol.

Another embodiment of the invention disclosed herein relates to processes of making HFO-1252zc by reacting HCFC-252dc and HCFC-253db in the liquid phase in the presence of a metal, preferably a reactive metal, to form a product mixture comprising HFO-1252zc.

More particularly, in an alternative embodiment, the process for making HFO-1252zc comprise reacting HCFC-252dc and HCFC-253db in the liquid phase, in the presence of a reactive metal selected from Zn, Mg or combination of them, at temperatures between 50°C and 160°C, more preferably about 80°C to about 160°, and more preferably in the range of about 90° to about 140°C, optionally in the presence an alcohol or an aprotic solvent and inert diluent gas, optionally in the presence of a zinc salt or phase transfer catalyst.

Another embodiment disclosed herein relates to a process for making HFO-1252zc by first producing HCFO-1233xf and catalytically hydrogenating the HCFO-1233xf to produce HCFC-253db. Then, the process comprises converting the HCFC-253db to HFO-1252zc by reaction with a reactive metal such as Zn or Mg.

### Integrated Processes

In some embodiments, the present invention relates to an integrated process for making HFO-1252zc, according to the following reaction scheme (A):
**Step 1:**
   CCl₂=CClCH₂Cl (HCO-1230xa) + HF → CClF₂CCl=CH₂ (HCFO-1232xf) + HCl
**Step 2:**
   CClF₂CCl=CH₂ + H₂ → CClF₂CHClCH₃ (HCFC-252dc)
**Steps 3A or Step 3B:**
   **3A:** CClF₂CHClCH₃ + H₂ → CF₂=CHCH₃ (HFO-1252zc) + HCl
   **3B:** CClF₂CHClCH₃ + Zn →CF₂=CHCH₃ + ZnCl₂

In some embodiments, the present invention relates to an integrated process for making HFO-1252zc, according to the following reaction scheme (B):
**Step 1:**
   CCl₂=CClCH₂Cl (HCO-1230xa) + HF → CF₃CCl=CH₂ (HCFO-1233xf) + HCl
**Step 2:**
   CF₃CCl=CH₂+ HCl → CClF₂CCl=CH₂ (HCFO-1232xf)
**Step 3:**
   CClF₂CCl=CH₂ + H₂ → CClF₂CHClCH₃ (HCFC-252dc)
**Steps 4A or Step 4B:**
   **4A:** CClF₂CHClCH₃ + H₂ + CF₂=CHCH₃ (HFO-1252zc) + HCl
   **4B:** CClF₂CHClCH₃ + Zn → CF₂=CHCH₃ + ZnCl₂

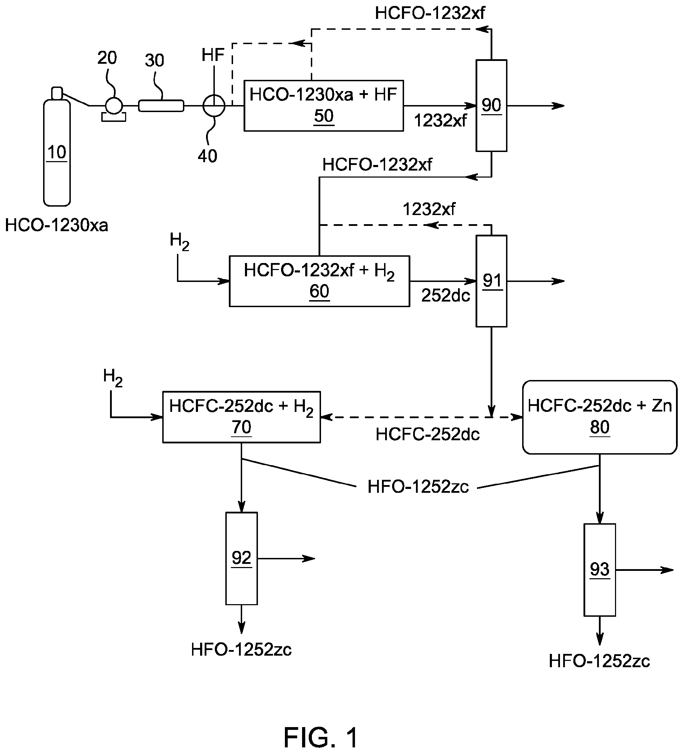

**Fig. 1** illustrates one embodiment for the conversion of HCO-1230xa. The HCO-1230xa feed flow is conveyed by pump **20** to and through optional vaporizer **30** in which the organic feed is heated and combined with HF feed in mixer **40** and then fed to reactor **50,** or alternatively the feed can be connected through valving directly to the reactor **50.** The combined HCO-1230xa/HF feed is fed into a heated, catalyst reactor **50** using any system suitable for heating, controlling and regulating temperatures ranging from between about 150°C and 350°C, at a temperature suitable for the hydrofluorination of HCO-1230xa. The product mixture from reactor **50** which comprises HCFO-1232xf and/or HCFO-1233xf is discharged and transferred into and through a treating system **90,** e.g., scrubbers (via scrubbing fluid), distillation columns (not shown), a decanter (not shown), etc. to purify and recover at least one of HCFO-1232xf or HCFO-1233xf for further processes in reactor **60** and treatment system **91.** For example, the HCFO-1232xf may be hydrogenated in reactor **60** to produce a reaction mixture comprising HCFC-252dc. Similarly, HCFC-252dc from reactor **60** and be further processed in reactors **70** or **80,** to produce HFO-1252zc.

Certain process embodiments described herein are illustrated in the drawings. For example, in one embodiment, referring to Fig. 1, three reaction zones **50, 60, 70/80** are depicted.

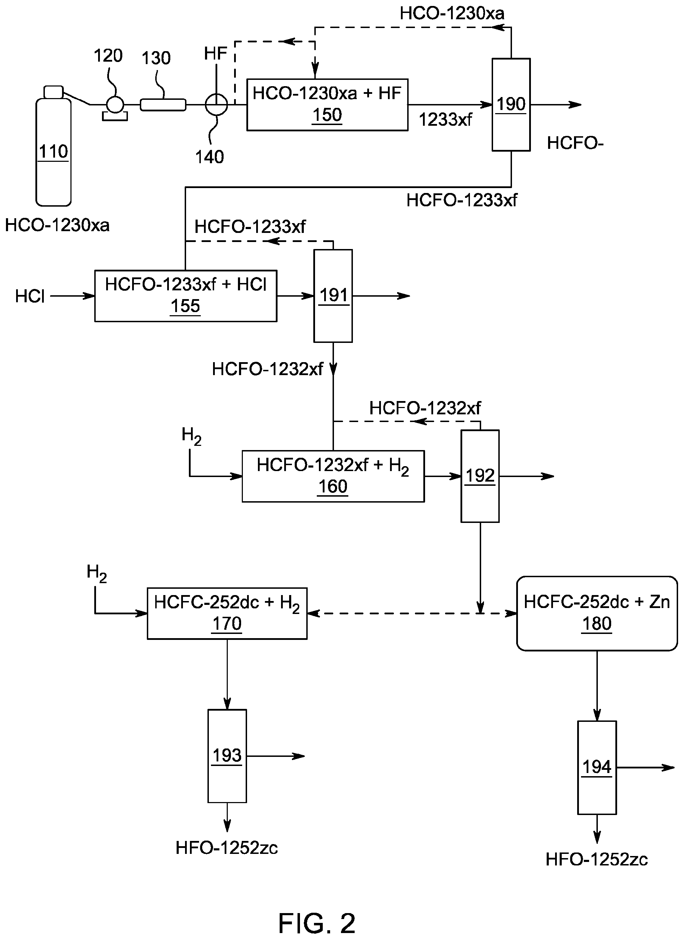

In another embodiment, referring to Fig. 2, four reactions zones **150, 155, 160** and **170/180** are depicted.

In certain embodiments, the reactors or reaction zones for HCFC-252dc conversion to HFO-1252zc can be configured for vapor phase reactions in reactors **60/170** or for liquid phase reactions in reactors **70/180.** The process can be operated in batch, semi-continuous or continuous modes.

In one embodiment, the starting feed includes HCO-1230xa which is pumped by a pump **20, 120** from feed vessel **10, 110** to the first reaction zone **50, 150** for contact with hydrogen fluoride (HF) to produce either HCFO-1232xf (see Fig. 1) or to co-produce HCFO-1232xf and HCFO-1233xf intermediates. The HCO-1230xa feed is preferably first vaporized in vaporizer **30, 130** and then mixed with HF in mixers **40, 140,** or may be vaporized together with HF in vaporizer **30, 130,** and then fed to the first reaction zone **50, 150** for contact with the catalyst bed. Alternatively, in the vapor phase, HF and HCO-1230xa can be mixed directly in the first reaction zone **50, 150** upstream of the catalyst bed (not shown). The first reaction zone **50, 150** is heated and the reaction proceeds and produces a product mixture containing either HCFO-1232xf (e.g,. Fig. 1, reactor **50**) or HCFO-1232xf and HCFO-1233xf (e.g., Fig. 2, reactor **150**). The product mixture comprising either HCFO-1232xf or HCFO-1232xf and HCFO-1233xf is withdrawn and preferably further processed (e.g., system **90, 190**) to upgrade the content of either HCFO-1232xf or HCFO-1232xf and HCFO-1233xf, such as using conventional separation techniques, recycling of unreacted HCO-1230xa and HCFO-1231 isomers to the first reaction zone, removal of HF and HCl. Removal of unreacted HCO-1230xa from the product mixture increases the relative concentration/amount of HCFO-1232xf or HCFO-1232xf and HCFO-1233xf which is then fed to a second reaction zone.

HCFO-1233xf can be fed to an intermediate reaction zone **155** suitable for conversion of the HCFO-1233xf to HCFO-1232xf, and the product mixture comprising HCFO-1232xf is withdrawn and preferably further processed in system **191** to upgrade the content of the HCFO-1232xf.

HCFO-1232xf and H₂ can be premixed and fed to the second reaction zone **60, 160** or mixed in the second reaction zone **60, 160** upstream of the catalyst bed. A product mixture of a second intermediate comprising HCFC-252dc is withdrawn from the second reaction zone **60, 160** and subjected to separation techniques in system **91, 192** to upgrade the HCFC-252dc content, and any unreacted HCFO-1232xf is optionally recycled as shown in Figs. 1 and 2. The upgraded HCFC-252dc intermediate can then be converted to HFO-1252zc using only one of the two flowlines illustrated in Figs. 1 and 2. That is, the HCFC-252dc may be contacted with hydrogen in the vapor phase in the presence of a catalyst to form a product mixture comprising HFO-1252zc in a third reaction zone **70, 170** configured for vapor phase reactions, or may be contacted in the liquid phase with a reactive metal, optionally in the presence of an aprotic solvent and optionally in the presence of a catalyst, to form a product mixture comprising HFO-1252zc in a third reaction zone **80, 180** configured for liquid phase reactions. A product mixture comprising HFO-1252zc is withdrawn from the third reaction zone **70, 170** and subjected to separation techniques in system **92, 193** or is withdrawn from the third reaction zone **80, 180** and subjected to separation techniques in system **93, 194** to upgrade the HFO-1252zc content.

Certain embodiments described herein relate to a system comprising supplies of HCO-1230xa, hydrogen, hydrogen chloride or hydrogen fluoride, a vaporizer, at least one mixer, at least first, second and third serially arranged reactors respectively producing first and second intermediate product mixtures, and the third reactor providing a final product mixture, and one or more separator systems, said first reactor containing a flow through bed of fluorination catalyst, said second reactor containing a flow through bed of hydrogenation catalyst, and said third reactor configured for one of a vapor phase reaction or a liquid phase reaction to convert a hydrochlorofluorocarbon, namely HCFC-252dc, produced in said second reactor to HFO-1252zc. Preferably, each of said reactors includes a discharge line, and at least the discharge line from one of said first and second reactors first conveys the respective first and second intermediate product mixtures to respective first and second separator systems to recover first and second intermediate products which are respectively conveyed to a downstream reactor.

Certain embodiments described herein relate to a system further comprising a vaporizer and a mixer connected in series and arranged downstream of said source and upstream of said first reactor.

Certain embodiments described herein relate to a system wherein said pressurized source of hydrogen fluoride is connected to a mixer.

Certain embodiments described herein relate to a system wherein a pressurized source of hydrogen fluoride is connected to directly to said first reactor.

Certain embodiments described herein relate to a system wherein unreacted HCFO-1230xa from the first separator system is recycled said first reactor.

Certain embodiments described herein relate to a system wherein a self-contained source of HCO-1230xa, pressurized source container of hydrogen and hydrogen fluoride, a vaporizer, at least one mixer, first, second, third, and fourth serially arranged reactors respectfully producing first, second and third intermediate product mixtures, and said fourth reactor providing a final product mixture, wherein separator systems can be arranged between the first and second reactors, second and third reactors, and third and fourth reactors to separate and purify the intermediate product of the respective first and third product mixture, said first reactor containing a flow through bed of hydrofluorination catalyst, said second reactor containing a flow through bed of hydrochlorination catalyst, and said third reactor containing a flow through bed of hydrogenation catalyst, sand said fourth reactor configured for one of a vapor phase reactor or a liquid phase reactor to convert a hydrogenation products of the third reactor to HFO-1252zc, wherein each of said reactors includes a discharge line, and at least the discharge line from one of said first, second or third reactors first conveys the respective first, second and third intermediate product mixtures to respective first, second and third separator systems to recover first, second and third intermediate products which are respectively conveyed to a downstream reactor.

Certain embodiments described herein relate to a system wherein unreacted starting material of each reactor separated in a separator system can be recycled to the corresponding upstream reactor.

Certain embodiments described herein relate to a system wherein unreacted HCO-1230xa, HCFO-1231 isomers, unreacted first intermediate product or unreacted second intermediate product is recycled.

Certain embodiments described herein relate to a system wherein said first reactor provides an intermediate product mixture comprising HCFO-1232xf, said second reactor provides a second intermediate product mixture comprising HCFC-252dc, and said third vapor phase or liquid phase reactor provides a final product mixture comprising HFO-1252zc.

Certain embodiments described herein relate to a system wherein a first reactor provides a first intermediate product mixture comprising HCFO-1233xf, a second reactor provides a second intermediate product mixture comprising HCFO-1232xf, a third reactor provides a second intermediate product mixture comprising HCFC-252dc, and a fourth vapor phase or liquid phase reactor provides a final product mixture comprising HFO-1252zc.

In certain embodiments disclosed herein, the compositions comprise (1) HFO-1252zc, (2) HCFC-252dc, and (3) one or more additional compounds selected from HFO-1233xf, HFO-1243zf, HCFC-253db, HFC-263fb, HFC-272fb, HCFC-262fc, E-HFO-1261ze, Z-HFO-1261ze, HFC-152a, HCFO-1241xb, HCFO-1242xf, HCFO-1242zf, HCFO-1232xf, Z-HCFO1251zb, E-HCFO-1251zb, and HCFO-1242xc.

### EXAMPLES

### Example 1: Hydrofluorination of HCO-1230xa to

### HCFO-1233xf and HCFO-1232xf without a catalyst

An empty 12-inches long Monel (0.5" OD and 0.43" ID) tube was used as a reactor with a 10-inch heating zone. The HCO-1230xa-HF reaction was tested at/under the conditions provided in Table 2. HCO-1230xa was fed by a pump and passed through a vaporizer at 200°C with N₂ and was then mixed with HF, and the mixture flowed through the reactor. The reactor effluent was analyzed by a GC-MS-FID. The analysis shows that HCFO-1233xf and HCFO-1232xf were co-produced at high concentrations at various conditions. All of the tests were done at atmospheric pressure. The test conditions are provided in Table 2 and the product analysis is provided in Table 3.

**Table 2: Example 1 Reaction Conditions**

| **Time Hr** | **T C** | **1230xa ml/hr** | **N2 sccm** | **HF sccm** | **HF/1230xa mol ratio** | **Contact Time Sec** |
|---|---|---|---|---|---|---|
| 1.7 | 203 | 0.11 | 10.16 | 11.51 | 30 | 17 |
| 3.3 | 203 | 0.11 | 9.98 | 11.51 | 30 | 17 |
| 5.0 | 223 | 0.11 | 10.02 | 11.48 | 30 | 16 |
| 6.7 | 223 | 0.11 | 10.01 | 11.49 | 30 | 16 |
| 8.3 | 242 | 0.11 | 10.01 | 11.48 | 30 | 16 |
| 10.0 | 243 | 0.11 | 10.01 | 11.43 | 30 | 16 |
| 11.7 | 263 | 0.11 | 10.01 | 11.49 | 30 | 15 |
| 13.3 | 262 | 0.11 | 10.01 | 11.44 | 30 | 15 |
| 15.0 | 283 | 0.11 | 10.01 | 11.48 | 30 | 14 |
| 16.7 | 278 | 0.11 | 10.01 | 11.43 | 30 | 15 |
| 18.3 | 303 | 0.11 | 9.98 | 11.53 | 30 | 14 |
| 20.0 | 298 | 0.10 | 10.02 | 11.47 | 33 | 14 |
| 21.7 | 323 | 0.10 | 10.01 | 11.47 | 33 | 14 |
| 23.3 | 320 | 0.10 | 10.01 | 11.47 | 33 | 14 |
| 25.0 | 343 | 0.10 | 10.01 | 11.37 | 33 | 13 |
| 26.7 | 342 | 0.10 | 10.01 | 11.46 | 33 | 13 |
| 28.3 | 363 | 0.10 | 10.01 | 11.38 | 33 | 13 |
| 30.0 | 363 | 0.10 | 10.00 | 11.46 | 33 | 13 |
| 33.3 | 198 | 0.07 | 10.00 | 11.42 | 47 | 17 |
| 35.0 | 199 | 0.07 | 10.00 | 11.46 | 47 | 17 |
| 36.7 | 224 | 0.07 | 10.00 | 11.46 | 47 | 16 |
| 38.3 | 223 | 0.07 | 10.00 | 11.46 | 47 | 16 |
| 40.0 | 243 | 0.07 | 10.00 | 11.60 | 48 | 16 |
| 41.7 | 243 | 0.07 | 10.00 | 11.40 | 47 | 16 |
| 43.3 | 263 | 0.07 | 10.00 | 11.46 | 47 | 15 |
| 45.0 | 261 | 0.07 | 10.00 | 11.43 | 47 | 15 |
| 46.7 | 283 | 0.07 | 10.00 | 11.49 | 47 | 15 |
| 48.3 | 280 | 0.07 | 10.01 | 11.46 | 47 | 15 |
| 50.0 | 302 | 0.07 | 10.01 | 11.47 | 47 | 14 |
| 51.7 | 298 | 0.07 | 10.01 | 11.47 | 47 | 14 |
| 53.3 | 323 | 0.07 | 10.01 | 11.47 | 47 | 14 |
| 55.0 | 318 | 0.07 | 10.01 | 11.49 | 47 | 14 |
| 56.7 | 343 | 0.07 | 10.01 | 11.49 | 47 | 13 |
| 58.3 | 339 | 0.07 | 10.01 | 11.49 | 47 | 13 |
| 60.0 | 362 | 0.07 | 10.01 | 11.48 | 47 | 13 |
| 61.7 | 362 | 0.07 | 10.01 | 11.46 | 47 | 13 |

**Table 3: Example 1 GC analysis of product stream**

| **Time** | **GC FID area%** | | | | |
|---|---|---|---|---|---|
| **Hr** | **1233xf %** | **C4H6ClF %** | **1232xf %** | **1232 isomer %** | **1231 isomer %** |
| 1.7 | 6.40 | 1.40 | 78.09 | 0.34 | 3.77 |
| 3.3 | 6.71 | 1.70 | 75.91 | 0.37 | 4.27 |
| 5.0 | 6.36 | 1.54 | 74.13 | 0.33 | 4.41 |
| 6.7 | 6.21 | 1.47 | 75.38 | 0.34 | 3.95 |
| 8.3 | 6.14 | 1.51 | 78.44 | 0.36 | 3.55 |
| 10.0 | 6.00 | 1.37 | 73.05 | 0.35 | 4.77 |
| 11.7 | 5.95 | 1.56 | 72.78 | 0.37 | 4.50 |
| 13.3 | 5.96 | 1.40 | 73.30 | 0.34 | 4.75 |
| 15.0 | 5.96 | 1.23 | 75.34 | 0.33 | 3.94 |
| 16.7 | 6.03 | 1.30 | 73.57 | 0.35 | 4.52 |
| 18.3 | 6.24 | 1.32 | 76.68 | 0.34 | 3.90 |
| 20.0 | 6.15 | 1.62 | 74.91 | 0.38 | 4.56 |
| 21.7 | 5.89 | 1.29 | 72.87 | 0.35 | 4.69 |
| 23.3 | 5.71 | 1.47 | 72.72 | 0.35 | 4.46 |
| 25.0 | 5.70 | 1.46 | 71.95 | 0.37 | 4.69 |
| 26.7 | 5.71 | 1.54 | 71.93 | 0.37 | 4.83 |
| 28.3 | 6.04 | 1.21 | 77.27 | 0.34 | 3.92 |
| 30.0 | 6.66 | 1.61 | 77.91 | 0.37 | 3.52 |
| 33.3 | 7.66 | 1.19 | 78.68 | 0.23 | 3.57 |
| 35.0 | 7.76 | 1.15 | 80.27 | 0.33 | 2.78 |
| 36.7 | 7.82 | 1.23 | 79.51 | 0.37 | 3.06 |
| 38.3 | 8.53 | 1.01 | 81.46 | 0.21 | 2.22 |
| 40.0 | 8.02 | 1.14 | 81.26 | 0.33 | 2.44 |
| 41.7 | 8.45 | 1.08 | 81.95 | 0.22 | 2.14 |
| 43.3 | 7.57 | 1.11 | 84.60 | 0.00 | 2.37 |
| 45.0 | 7.41 | 1.08 | 82.69 | 0.26 | 3.21 |
| 46.7 | 7.74 | 1.03 | 82.93 | 0.26 | 2.88 |
| 48.3 | 7.74 | 1.03 | 82.93 | 0.26 | 2.88 |
| 50.0 | 8.29 | 0.91 | 83.75 | 0.22 | 2.27 |
| 51.7 | 7.59 | 1.05 | 83.98 | 0.25 | 2.65 |
| 53.3 | 7.74 | 1.02 | 83.61 | 0.25 | 2.84 |
| 55.0 | 7.65 | 0.96 | 85.13 | 0.24 | 2.28 |
| 56.7 | 9.32 | 0.83 | 84.00 | 0.24 | 1.92 |
| 58.3 | 8.26 | 1.01 | 83.72 | 0.23 | 2.37 |
| 60.0 | 8.17 | 0.85 | 85.26 | 0.23 | 1.94 |
| 61.7 | 8.64 | 0.83 | 84.32 | 0.21 | 2.28 |

**Table 3 Example 1 GC analysis of product stream (Continued)**

| **Time** | **GC FID area%** | | | | | |
|---|---|---|---|---|---|---|
| **Hr** | **1231 isomer %** | **1231 isomer %** | **1232 isomer %** | **1110 %** | **1230xa %** | **Others %** |
| 1.7 | 2.12 | 2.19 | 1.29 | 0.00 | 0.00 | 4.42 |
| 3.3 | 2.10 | 2.20 | 1.27 | 0.00 | 0.00 | 5.47 |
| 5.0 | 2.86 | 2.66 | 1.37 | 0.00 | 0.29 | 6.05 |
| 6.7 | 2.69 | 2.45 | 1.24 | 0.00 | 0.29 | 5.97 |
| 8.3 | 1.95 | 2.03 | 0.96 | 0.00 | 0.24 | 4.83 |
| 10.0 | 3.08 | 2.99 | 1.35 | 0.00 | 0.29 | 6.75 |
| 11.7 | 2.94 | 2.83 | 1.27 | 0.00 | 0.32 | 7.48 |
| 13.3 | 3.07 | 2.97 | 1.30 | 0.00 | 0.30 | 6.62 |
| 15.0 | 2.67 | 2.51 | 1.18 | 0.00 | 0.28 | 6.57 |
| 16.7 | 2.93 | 2.81 | 1.25 | 0.00 | 0.27 | 6.97 |
| 18.3 | 2.09 | 2.21 | 1.09 | 0.00 | 0.29 | 5.84 |
| 20.0 | 2.18 | 2.32 | 0.99 | 0.00 | 0.32 | 6.57 |
| 21.7 | 3.03 | 2.95 | 1.24 | 0.42 | 0.33 | 6.93 |
| 23.3 | 2.89 | 2.78 | 1.15 | 0.41 | 0.31 | 7.75 |
| 25.0 | 3.09 | 3.00 | 1.26 | 0.46 | 0.27 | 7.76 |
| 26.7 | 3.10 | 3.03 | 1.23 | 0.43 | 0.31 | 7.53 |
| 28.3 | 1.97 | 2.06 | 0.96 | 0.00 | 0.28 | 5.94 |
| 30.0 | 1.80 | 1.88 | 1.02 | 0.00 | 0.26 | 4.98 |
| 33.3 | 1.58 | 1.62 | 1.12 | 0.00 | 0.00 | 4.35 |
| 35.0 | 1.33 | 1.32 | 0.97 | 0.00 | 0.00 | 4.09 |
| 36.7 | 1.36 | 1.37 | 0.98 | 0.00 | 0.00 | 4.29 |
| 38.3 | 1.02 | 1.00 | 0.92 | 0.00 | 0.00 | 3.65 |
| 40.0 | 1.17 | 1.15 | 0.91 | 0.00 | 0.00 | 3.59 |
| 41.7 | 1.07 | 1.03 | 0.93 | 0.00 | 0.00 | 3.13 |
| 43.3 | 0.89 | 0.52 | 0.55 | 0.07 | 0.18 | 2.15 |
| 45.0 | 1.20 | 0.77 | 0.56 | 0.07 | 0.25 | 2.51 |
| 46.7 | 0.96 | 0.67 | 0.61 | 0.08 | 0.28 | 2.55 |
| 48.3 | 0.96 | 0.67 | 0.61 | 0.08 | 0.28 | 2.55 |
| 50.0 | 0.63 | 0.49 | 0.63 | 0.09 | 0.25 | 2.48 |
| 51.7 | 0.98 | 0.76 | 0.57 | 0.09 | 0.18 | 1.90 |
| 53.3 | 0.87 | 0.58 | 0.61 | 0.09 | 0.18 | 2.21 |
| 55.0 | 0.62 | 0.51 | 0.52 | 0.08 | 0.18 | 1.82 |
| 56.7 | 0.54 | 0.46 | 0.72 | 0.08 | 0.00 | 1.88 |
| 58.3 | 0.76 | 0.60 | 0.68 | 0.07 | 0.17 | 2.12 |
| 60.0 | 0.62 | 0.41 | 0.61 | 0.10 | 0.15 | 1.66 |
| 61.7 | 0.71 | 0.34 | 0.63 | 0.08 | 0.12 | 1.85 |

### Example 2: Hydrofluorination of HCO-1230xa to

### HCFO-1233xf and HCFO-1232xf without a catalyst

An empty 12-inches long Monel (0.5 0.5" OD and 043" ID) tube was used as a reactor with a 10-inch heating zone. The test conditions are provided in Table 2 and the product analysis is provided in Table 3

An empty 12-inches long Monel (0.5" OD and 0.43" ID) tube with a 10-inch heating zone was used as a reactor. The HCO-1230xa-HF reaction was tested at/under the conditions provided in Table 2. HCO-1230xa was fed by a pump and passed through a vaporizer at 200°C with N₂ and was then mixed with HF, and the mixture flowed through the reactor. The reactor effluent was analyzed by a GC-MS-FID. The analysis shows that HCFO-1233xf and HCFO-1232xf were co-produced at high concentrations at various conditions. All of the tests were done at atmospheric pressure and with longer contact times compared to Example 1. The test conditions are provided in Table 4 and the product analysis is provided in Table 5. Comparing similar conditions in Examples 1 and 2 shows that the longer contact time in Example 2 longer contact time increased HCFO-1232xf selectivity.

**Table 4: Example 2 Reaction Conditions**

| **Time Hr** | **T C** | **1230xa ml/hr** | **N2 sccm** | **HF sccm** | **HF/1230xa mol ratio** | **Contact time Sec** |
|---|---|---|---|---|---|---|
| 63.3 | 201 | 0.06 | 6.96 | 5.98 | 29 | 28 |
| 65.0 | 199 | 0.06 | 7.04 | 5.98 | 29 | 28 |
| 66.7 | 204 | 0.06 | 6.99 | 6.11 | 29 | 28 |
| 68.3 | 220 | 0.06 | 6.99 | 6.13 | 29 | 27 |
| 70.0 | 218 | 0.06 | 6.99 | 6.00 | 29 | 27 |
| 71.7 | 243 | 0.06 | 6.96 | 6.00 | 29 | 26 |
| 73.3 | 239 | 0.06 | 6.99 | 5.97 | 29 | 26 |
| 75.0 | 263 | 0.06 | 6.98 | 5.97 | 29 | 25 |
| 76.7 | 260 | 0.06 | 6.97 | 5.97 | 29 | 25 |
| 78.3 | 283 | 0.06 | 6.98 | 5.97 | 29 | 24 |
| 80.0 | 281 | 0.06 | 7.02 | 5.97 | 29 | 24 |
| 81.7 | 302 | 0.06 | 6.96 | 5.97 | 29 | 23 |
| 83.3 | 298 | 0.06 | 7.01 | 5.97 | 29 | 23 |
| 85.0 | 323 | 0.06 | 6.96 | 5.97 | 29 | 22 |
| 86.7 | 318 | 0.06 | 6.98 | 5.97 | 29 | 23 |
| 88.3 | 343 | 0.06 | 6.98 | 5.97 | 29 | 22 |
| 90.0 | 343 | 0.06 | 6.98 | 5.97 | 29 | 22 |
| 91.7 | 359 | 0.06 | 6.98 | 6.13 | 29 | 21 |
| 93.3 | 358 | 0.06 | 6.98 | 5.96 | 29 | 21 |
| 95.0 | 198 | 0.04 | 7.09 | 5.99 | 43 | 28 |
| 96.7 | 202 | 0.04 | 7.01 | 5.99 | 43 | 28 |
| 98.3 | 203 | 0.04 | 6.99 | 5.99 | 43 | 28 |
| 100.0 | 219 | 0.04 | 7.02 | 5.99 | 43 | 27 |
| 101.7 | 223 | 0.04 | 7.01 | 5.96 | 43 | 27 |
| 103.3 | 250 | 0.04 | 6.95 | 5.98 | 43 | 26 |
| 105.0 | 248 | 0.04 | 6.97 | 5.98 | 43 | 26 |
| 106.7 | 283 | 0.04 | 7.01 | 5.98 | 43 | 24 |
| 108.3 | 283 | 0.04 | 7.01 | 5.98 | 43 | 24 |
| 110.0 | 313 | 0.04 | 7.02 | 5.98 | 43 | 23 |
| 111.7 | 313 | 0.04 | 6.96 | 5.98 | 43 | 23 |
| 113.3 | 343 | 0.04 | 7.02 | 5.98 | 43 | 22 |
| 115.0 | 342 | 0.04 | 7.02 | 5.98 | 43 | 22 |
| 116.7 | 323 | 0.04 | 7.02 | 5.98 | 43 | 22 |

**Table 5: Example 2 GC analysis of product stream**

| **Time** | **GC FID area** | | | | |
|---|---|---|---|---|---|
| **hr** | **1233xf** | **C4H6ClF** | **1232xf** | **1232 isomer** | **1231 isomer** |
| 63.3 | 7.08 | 1.15 | 81.96 | 0.29 | 4.08 |
| 65.0 | 6.92 | 1.17 | 84.06 | 0.30 | 3.23 |
| 66.7 | 7.11 | 1.01 | 84.21 | 0.29 | 3.18 |
| 68.3 | 6.86 | 0.99 | 85.11 | 0.26 | 2.71 |
| 70.0 | 7.25 | 1.37 | 85.04 | 0.24 | 2.59 |
| 71.7 | 6.51 | 1.02 | 82.77 | 0.29 | 3.71 |
| 73.3 | 6.42 | 1.10 | 83.45 | 0.28 | 3.65 |
| 75.0 | 6.37 | 1.14 | 81.68 | 0.41 | 4.09 |
| 76.7 | 6.28 | 1.00 | 83.80 | 0.27 | 3.69 |
| 78.3 | 6.06 | 0.88 | 83.30 | 0.28 | 3.81 |
| 80.0 | 5.82 | 1.02 | 76.16 | 0.27 | 4.50 |
| 81.7 | 6.58 | 1.07 | 82.17 | 0.29 | 3.70 |
| 83.3 | 6.50 | 1.09 | 83.42 | 0.30 | 3.65 |
| 85.0 | 6.62 | 1.05 | 83.54 | 0.29 | 3.85 |
| 86.7 | 6.52 | 1.06 | 84.60 | 0.26 | 3.30 |
| 88.3 | 6.51 | 1.01 | 84.39 | 0.28 | 3.66 |
| 90.0 | 6.83 | 1.13 | 82.45 | 0.28 | 4.02 |
| 91.7 | 6.90 | 0.97 | 84.04 | 0.29 | 3.56 |
| 93.3 | 6.54 | 0.97 | 83.47 | 0.30 | 3.73 |
| 95.0 | 4.85 | 0.85 | 73.03 | 0.22 | 10.31 |
| 96.7 | 5.55 | 0.79 | 77.41 | 0.18 | 6.84 |
| 98.3 | 7.13 | 0.72 | 80.17 | 0.18 | 5.07 |
| 100.0 | 7.77 | 0.60 | 82.77 | 0.22 | 4.04 |
| 101.7 | 9.16 | 0.69 | 83.18 | 0.25 | 3.06 |
| 103.3 | 10.40 | 0.64 | 83.58 | 0.16 | 2.14 |
| 105.0 | 10.96 | 0.63 | 83.33 | 0.31 | 2.07 |
| 106.7 | 12.15 | 0.63 | 82.99 | 0.00 | 1.61 |
| 108.3 | 13.30 | 0.67 | 82.35 | 0.13 | 1.27 |
| 110.0 | 14.05 | 0.66 | 82.21 | 0.12 | 1.09 |
| 111.7 | 14.40 | 0.74 | 81.64 | 0.11 | 1.15 |
| 113.3 | 20.69 | 0.44 | 76.08 | 0.00 | 0.45 |
| 115.0 | 16.52 | 0.55 | 79.57 | 0.11 | 0.85 |
| 116.7 | 16.18 | 0.74 | 80.45 | 0.14 | 0.73 |

**Table 5: Example 2 GC analysis of product stream (continued)**

| **Time** | **GC FID area** | | | | | |
|---|---|---|---|---|---|---|
| **hr** | **1231 isomer** | **1231 isomer** | **1232 isomer** | **1110** | **1230xa** | **Others** |
| 63.3 | 1.29 | 1.00 | 0.79 | 0.09 | 0.19 | 2.08 |
| 65.0 | 1.08 | 0.74 | 0.53 | 0.10 | 0.13 | 1.77 |
| 66.7 | 0.85 | 0.81 | 0.53 | 0.08 | 0.15 | 1.78 |
| 68.3 | 1.06 | 0.68 | 0.46 | 0.09 | 0.09 | 1.69 |
| 70.0 | 0.76 | 0.55 | 0.35 | 0.05 | 0.05 | 1.71 |
| 71.7 | 1.54 | 1.14 | 0.49 | 0.07 | 0.13 | 2.33 |
| 73.3 | 1.39 | 1.01 | 0.45 | 0.10 | 0.10 | 2.04 |
| 75.0 | 1.42 | 0.98 | 0.50 | 0.08 | 0.12 | 3.21 |
| 76.7 | 1.31 | 0.96 | 0.39 | 0.10 | 0.13 | 2.05 |
| 78.3 | 1.74 | 1.23 | 0.41 | 0.10 | 0.14 | 2.05 |
| 80.0 | 3.70 | 2.85 | 1.18 | 0.41 | 0.14 | 3.94 |
| 81.7 | 1.72 | 1.40 | 0.54 | 0.00 | 0.13 | 2.41 |
| 83.3 | 1.38 | 1.09 | 0.52 | 0.08 | 0.00 | 1.97 |
| 85.0 | 1.47 | 0.96 | 0.46 | 0.07 | 0.00 | 1.68 |
| 86.7 | 1.23 | 0.84 | 0.46 | 0.10 | 0.00 | 1.63 |
| 88.3 | 1.09 | 0.97 | 0.47 | 0.09 | 0.00 | 1.53 |
| 90.0 | 1.46 | 1.06 | 0.49 | 0.08 | 0.00 | 2.21 |
| 91.7 | 1.25 | 0.86 | 0.51 | 0.08 | 0.00 | 1.55 |
| 93.3 | 1.31 | 1.37 | 0.55 | 0.07 | 0.00 | 1.69 |
| 95.0 | 2.78 | 2.81 | 0.60 | 0.08 | 0.00 | 4.47 |
| 96.7 | 1.46 | 1.72 | 0.54 | 0.00 | 0.00 | 5.50 |
| 98.3 | 1.31 | 1.29 | 0.65 | 0.00 | 0.00 | 3.47 |
| 100.0 | 1.34 | 0.88 | 0.51 | 0.00 | 0.00 | 1.87 |
| 101.7 | 0.86 | 0.74 | 0.68 | 0.06 | 0.00 | 1.32 |
| 103.3 | 0.75 | 0.43 | 0.73 | 0.06 | 0.00 | 1.11 |
| 105.0 | 0.62 | 0.36 | 0.71 | 0.05 | 0.00 | 0.97 |
| 106.7 | 0.49 | 0.45 | 0.80 | 0.00 | 0.00 | 0.88 |
| 108.3 | 0.40 | 0.16 | 0.83 | 0.00 | 0.00 | 0.89 |
| 110.0 | 0.24 | 0.00 | 0.85 | 0.06 | 0.00 | 0.73 |
| 111.7 | 0.30 | 0.00 | 0.81 | 0.00 | 0.00 | 0.84 |
| 113.3 | 0.00 | 0.00 | 1.41 | 0.00 | 0.00 | 0.92 |
| 115.0 | 0.33 | 0.19 | 1.05 | 0.06 | 0.00 | 0.77 |
| 116.7 | 0.21 | 0.00 | 0.89 | 0.07 | 0.00 | 0.59 |

### Example 3: Hyrofluorination of HCO-1230xa to

### HCFO-1233xf and HCFO-1232xf with 8ml Hastelloy C packing

8 ml Hastelloy C packing (9.4g) was loaded into a 12 inches long Monel (0.5" OD and 0.43" ID) tube with 10 inches heating zone, which was used as a reactor. HCO-1230xa was fed by a pump and passed through a vaporizer at 200°C with N₂ and was then mixed with HF, and the mixture flowed through the reactor with the catalyst bed. The reactor effluent was analyzed by a GC-MS-FID. The analysis shows that HCFO-1233xf and HCFO-1232xf were co-produced at high concentrations at various conditions. All of the tests were done at atmospheric pressure. The test conditions are provided in Table 6 and the product analysis is provided in Table 7.

**Table 6: Example 3 Reaction Conditions**

| **Time Hr** | **T C** | **1230xa sccm** | **N2 sccm** | **HF sccm** | **HF/1230xa mol ratio** | **Contact Time Sec** |
|---|---|---|---|---|---|---|
| 1.7 | 204 | 0.38 | 40.18 | 6.02 | 16 | 6.3 |
| 3.3 | 204 | 0.38 | 40.57 | 5.96 | 16 | 6.3 |
| 5.0 | 204 | 0.38 | 40.54 | 6.00 | 16 | 6.3 |
| 6.7 | 224 | 0.38 | 40.52 | 6.02 | 16 | 6.0 |
| 8.3 | 224 | 0.38 | 40.50 | 6.02 | 16 | 6.0 |
| 10.0 | 222 | 0.38 | 40.42 | 6.02 | 16 | 6.1 |
| 11.7 | 243 | 0.38 | 40.11 | 6.03 | 16 | 5.9 |
| 13.3 | 238 | 0.38 | 40.26 | 6.04 | 16 | 5.9 |
| 15.0 | 242 | 0.38 | 40.11 | 6.04 | 16 | 5.9 |
| 16.7 | 259 | 0.38 | 40.21 | 6.04 | 16 | 5.7 |
| 18.3 | 261 | 0.38 | 40.12 | 6.09 | 16 | 5.7 |
| 20.0 | 264 | 0.38 | 40.13 | 6.15 | 16 | 5.6 |
| 21.7 | 280 | 0.38 | 40.09 | 6.13 | 16 | 5.5 |
| 23.3 | 279 | 0.38 | 40.15 | 6.10 | 16 | 5.5 |
| 25.0 | 284 | 0.38 | 40.08 | 6.11 | 16 | 5.4 |
| 26.7 | 198 | 0.59 | 40.12 | 6.01 | 10 | 6.4 |
| 28.3 | 203 | 0.59 | 40.09 | 6.01 | 10 | 6.3 |
| 30.0 | 201 | 0.59 | 40.13 | 6.01 | 10 | 6.4 |
| 31.7 | 220 | 0.59 | 40.14 | 6.01 | 10 | 6.1 |
| 33.3 | 222 | 0.59 | 40.07 | 6.06 | 10 | 6.1 |
| 35.0 | 220 | 0.59 | 40.12 | 6.02 | 10 | 6.1 |
| 36.7 | 238 | 0.59 | 40.07 | 6.02 | 10 | 5.9 |
| 38.3 | 243 | 0.59 | 40.10 | 6.02 | 10 | 5.8 |
| 40.0 | 242 | 0.59 | 40.26 | 6.02 | 10 | 5.8 |
| 41.7 | 262 | 0.59 | 40.53 | 6.02 | 10 | 5.6 |
| 43.3 | 258 | 0.59 | 40.49 | 6.02 | 10 | 5.6 |
| 45.0 | 258 | 0.59 | 40.53 | 6.02 | 10 | 5.6 |
| 46.7 | 283 | 0.62 | 40.14 | 6.08 | 10 | 5.4 |
| 48.3 | 278 | 0.62 | 40.11 | 6.15 | 10 | 5.4 |
| 50.0 | 283 | 0.62 | 40.10 | 6.11 | 10 | 5.4 |
| 51.7 | 199 | 1.18 | 40.19 | 6.02 | 5 | 6.3 |
| 53.3 | 198 | 1.18 | 40.37 | 6.02 | 5 | 6.3 |
| 55.0 | 199 | 1.18 | 40.25 | 6.02 | 5 | 6.3 |
| 56.7 | 221 | 1.18 | 40.38 | 6.02 | 5 | 6.0 |
| 60.0 | 219 | 1.18 | 40.50 | 6.04 | 5 | 6.0 |
| 61.7 | 238 | 1.18 | 40.30 | 6.04 | 5 | 5.8 |
| 65.0 | 240 | 1.18 | 40.15 | 6.04 | 5 | 5.8 |
| 66.7 | 260 | 1.18 | 40.40 | 6.04 | 5 | 5.5 |
| 68.3 | 262 | 1.18 | 40.09 | 6.03 | 5 | 5.6 |
| 70.0 | 279 | 1.18 | 40.08 | 6.03 | 5 | 5.4 |
| 71.7 | 282 | 1.18 | 40.43 | 6.03 | 5 | 5.3 |
| 73.3 | 278 | 1.18 | 40.39 | 6.03 | 5 | 5.4 |
| 75.0 | 278 | 1.18 | 40.39 | 6.03 | 5 | 5.4 |

**Table 7: Example 3 GC analysis of product stream**

| **Time** | **GC FID area%** | | | | |
|---|---|---|---|---|---|
| **Hr** | **1233xf** | **1232xf** | **1231 isomer** | **1230xa** | **others** |
| 1.7 | 30.64% | 63.70% | 0.00% | 0.00% | 5.66% |
| 3.3 | 26.01% | 70.41% | 0.30% | 0.00% | 3.28% |
| 5.0 | 26.15% | 70.90% | 0.00% | 0.00% | 2.95% |
| 6.7 | 24.85% | 71.83% | 0.00% | 0.00% | 3.33% |
| 8.3 | 24.22% | 72.46% | 0.00% | 0.00% | 3.33% |
| 10.0 | 24.58% | 72.30% | 0.00% | 0.00% | 3.13% |
| 11.7 | 22.76% | 73.20% | 0.33% | 0.00% | 3.71% |
| 13.3 | 22.89% | 73.85% | 0.00% | 0.00% | 3.26% |
| 15.0 | 22.27% | 74.49% | 0.00% | 0.00% | 3.24% |
| 16.7 | 22.80% | 74.03% | 0.00% | 0.00% | 3.17% |
| 18.3 | 21.70% | 74.62% | 0.36% | 0.00% | 3.32% |
| 20.0 | 20.81% | 75.39% | 0.37% | 0.00% | 3.43% |
| 21.7 | 20.77% | 75.26% | 0.42% | 0.00% | 3.56% |
| 23.3 | 20.92% | 75.57% | 0.00% | 0.00% | 3.52% |
| 25.0 | 20.68% | 75.86% | 0.00% | 0.00% | 3.46% |
| 26.7 | 20.08% | 75.73% | 0.46% | 0.00% | 3.72% |
| 28.3 | 18.30% | 77.09% | 0.57% | 0.00% | 4.03% |
| 30.0 | 17.96% | 77.42% | 0.54% | 0.00% | 4.08% |
| 31.7 | 17.34% | 78.01% | 0.54% | 0.00% | 4.10% |
| 33.3 | 16.69% | 78.61% | 0.59% | 0.00% | 4.12% |
| 35.0 | 16.34% | 78.89% | 0.57% | 0.00% | 4.20% |
| 36.7 | 16.33% | 78.76% | 0.67% | 0.00% | 4.23% |
| 38.3 | 15.87% | 78.76% | 0.75% | 0.00% | 4.61% |
| 40.0 | 15.54% | 79.55% | 0.68% | 0.00% | 4.22% |
| 41.7 | 15.20% | 79.60% | 0.68% | 0.00% | 4.52% |
| 43.3 | 14.92% | 79.57% | 0.86% | 0.00% | 4.66% |
| 45.0 | 14.77% | 80.00% | 0.87% | 0.00% | 4.35% |
| 46.7 | 15.37% | 79.60% | 0.77% | 0.00% | 4.26% |
| 48.3 | 14.57% | 80.30% | 0.86% | 0.00% | 4.27% |
| 50.0 | 14.51% | 80.36% | 0.81% | 0.00% | 4.31% |
| 51.7 | 15.82% | 79.63% | 0.61% | 0.00% | 3.95% |
| 53.3 | 13.56% | 79.82% | 1.03% | 0.00% | 5.59% |
| 55.0 | 12.51% | 79.96% | 1.13% | 0.00% | 6.41% |
| 56.7 | 11.83% | 79.62% | 1.54% | 0.00% | 7.02% |
| 60.0 | 11.27% | 79.20% | 1.87% | 0.00% | 7.66% |
| 61.7 | 10.20% | 79.31% | 2.50% | 0.00% | 7.99% |
| 65.0 | 9.52% | 80.00% | 2.39% | 0.00% | 8.08% |
| 66.7 | 9.52% | 79.92% | 2.45% | 0.00% | 8.11% |
| 68.3 | 9.28% | 79.73% | 2.78% | 0.00% | 8.21% |
| 70.0 | 9.47% | 79.22% | 3.28% | 0.00% | 8.02% |
| 71.7 | 9.17% | 80.38% | 2.54% | 0.00% | 7.90% |
| 73.3 | 9.22% | 79.44% | 3.33% | 0.00% | 8.01% |
| 75.0 | 8.88% | 79.67% | 3.06% | 0.00% | 8.39% |

### Example 4: Hyrofluorination of HCO-1230xa to

### HCFO-1233xf and HCFO-1232xf with 8ml Hastelloy C packing

8 ml Hastelloy C packing (9.4g) was loaded into a 12 inches long Monel (0.5" OD and 0.43" ID) tube with 10 inches heating zone, which was used as a reactor. The HCO-1230xa-HF reaction was tested at conditions listed table 8 below, with a relatively higher HF/1230xa ratio as compared with Example 3. HCO-1230xa was fed by a pump and passed through a vaporizer at 200°C with N₂ and was then mixed with HF, and the mixture flowed through the reactor with the catalyst bed. The reactor effluent was analyzed by a GC-MS-FID. The analysis shows that HCFO-1233xf and HCFO-1232xf were co-produced at high concentrations at various conditions. All of the tests were done at atmospheric pressure. The test conditions are provided in Table 8 and the product analysis is provided in Table 9.

**Table 8: Example 4 Reaction Conditions**

| **Time Hr** | **T C** | **1230xa cc/hr** | **1230xa sccm** | **N2 sccm** | **HF sccm** | **HF/1230xa mol ratio** | **Contact Time Sec** |
|---|---|---|---|---|---|---|---|
| 1.7 | 202 | 0.11 | 0.38 | 40 | 11.52 | 30 | 5.7 |
| 3.3 | 198 | 0.11 | 0.38 | 40 | 11.52 | 30 | 5.7 |
| 5.0 | 218 | 0.11 | 0.38 | 40 | 11.52 | 30 | 5.5 |
| 6.7 | 220 | 0.11 | 0.38 | 40 | 11.57 | 30 | 5.5 |
| 8.3 | 238 | 0.11 | 0.38 | 40 | 11.61 | 30 | 5.3 |
| 10.0 | 239 | 0.11 | 0.38 | 40 | 11.66 | 31 | 5.3 |
| 11.7 | 259 | 0.11 | 0.38 | 40 | 11.52 | 30 | 5.1 |
| 13.3 | 259 | 0.11 | 0.38 | 40 | 11.52 | 30 | 5.1 |
| 15.0 | 281 | 0.11 | 0.38 | 40 | 11.53 | 30 | 4.9 |
| 16.7 | 280 | 0.11 | 0.38 | 40 | 11.53 | 30 | 4.9 |
| 18.3 | 299 | 0.11 | 0.38 | 40 | 11.47 | 30 | 4.7 |
| 20.0 | 300 | 0.11 | 0.38 | 40 | 11.53 | 30 | 4.7 |
| 21.7 | 320 | 0.11 | 0.38 | 40 | 11.53 | 30 | 4.6 |
| 23.3 | 320 | 0.11 | 0.38 | 40 | 11.53 | 30 | 4.6 |
| 25.0 | 340 | 0.11 | 0.38 | 40 | 11.54 | 30 | 4.4 |
| 26.7 | 340 | 0.11 | 0.38 | 40 | 11.54 | 30 | 4.4 |
| 28.3 | 202 | 0.11 | 0.38 | 40 | 8.52 | 22 | 6.0 |
| 30.0 | 200 | 0.11 | 0.38 | 40 | 8.52 | 22 | 6.0 |
| 31.7 | 198 | 0.11 | 0.38 | 40 | 8.52 | 22 | 6.1 |
| 33.3 | 218 | 0.11 | 0.38 | 40 | 8.52 | 22 | 5.8 |
| 35.0 | 218 | 0.11 | 0.38 | 40 | 8.54 | 22 | 5.8 |
| 36.7 | 240 | 0.11 | 0.38 | 40 | 8.59 | 23 | 5.6 |
| 38.3 | 239 | 0.11 | 0.38 | 40 | 8.68 | 23 | 5.6 |
| 40.0 | 261 | 0.11 | 0.38 | 40 | 8.61 | 23 | 5.4 |
| 41.7 | 262 | 0.11 | 0.38 | 40 | 8.54 | 22 | 5.3 |
| 43.3 | 279 | 0.11 | 0.38 | 40 | 8.54 | 22 | 5.2 |
| 45.0 | 279 | 0.11 | 0.38 | 40 | 8.54 | 22 | 5.2 |
| 46.7 | 299 | 0.11 | 0.38 | 40 | 8.54 | 22 | 5.0 |
| 48.3 | 299 | 0.11 | 0.38 | 40 | 8.54 | 22 | 5.0 |
| 50.0 | 321 | 0.11 | 0.38 | 40 | 8.54 | 22 | 4.8 |
| 51.7 | 321 | 0.11 | 0.38 | 40 | 8.54 | 22 | 4.8 |
| 53.3 | 340 | 0.11 | 0.38 | 40 | 8.48 | 22 | 4.7 |
| 55.0 | 340 | 0.11 | 0.38 | 40 | 8.54 | 22 | 4.7 |
| 56.7 | 202 | 0.11 | 0.38 | 32 | 18.95 | 50 | 5.7 |
| 58.3 | 200 | 0.11 | 0.38 | 32 | 19.00 | 50 | 5.8 |
| 60.0 | 198 | 0.11 | 0.38 | 32 | 18.88 | 49 | 5.8 |
| 61.7 | 220 | 0.11 | 0.38 | 32 | 18.99 | 50 | 5.6 |
| 63.3 | 220 | 0.11 | 0.38 | 32 | 18.89 | 50 | 5.6 |
| 65.0 | 242 | 0.11 | 0.38 | 32 | 19.04 | 50 | 5.3 |
| 66.7 | 242 | 0.11 | 0.38 | 32 | 19.04 | 50 | 5.3 |
| 68.3 | 262 | 0.11 | 0.38 | 32 | 19.06 | 50 | 5.1 |
| 70.0 | 258 | 0.11 | 0.38 | 32 | 19.02 | 50 | 5.2 |
| 71.7 | 281 | 0.11 | 0.38 | 32 | 19.02 | 50 | 5.0 |
| 73.3 | 280 | 0.11 | 0.38 | 32 | 19.02 | 50 | 4.9 |
| 75.0 | 299 | 0.11 | 0.38 | 32 | 19.02 | 50 | 4.8 |
| 76.7 | 301 | 0.11 | 0.38 | 32 | 19.02 | 50 | 4.8 |
| 78.3 | 319 | 0.11 | 0.38 | 32 | 19.02 | 50 | 4.6 |
| 80.0 | 320 | 0.11 | 0.38 | 32 | 19.03 | 50 | 4.6 |
| 81.7 | 340 | 0.11 | 0.38 | 32 | 19.06 | 50 | 4.5 |
| 83.3 | 340 | 0.11 | 0.38 | 32 | 19.06 | 50 | 4.5 |
| 85.0 | 360 | 0.11 | 0.38 | 32 | 18.93 | 50 | 4.3 |
| 86.7 | 360 | 0.11 | 0.38 | 32 | 19.01 | 50 | 4.3 |
| 88.3 | 201 | 0.11 | 0.38 | 10 | 19.04 | 50 | 10.0 |
| 90.0 | 202 | 0.11 | 0.38 | 10 | 18.89 | 50 | 10.1 |
| 91.7 | 198 | 0.11 | 0.38 | 10 | 18.99 | 50 | 10.2 |
| 93.3 | 219 | 0.11 | 0.38 | 10 | 19.00 | 50 | 9.7 |
| 95.0 | 219 | 0.11 | 0.38 | 10 | 19.09 | 50 | 9.7 |
| 96.7 | 241 | 0.11 | 0.38 | 10 | 19.21 | 50 | 9.2 |
| 98.3 | 241 | 0.11 | 0.38 | 10 | 19.35 | 51 | 9.2 |
| 100.0 | 262 | 0.11 | 0.38 | 10 | 19.19 | 50 | 8.9 |
| 101.7 | 262 | 0.11 | 0.38 | 10 | 19.05 | 50 | 8.9 |
| 103.3 | 280 | 0.11 | 0.38 | 10 | 19.01 | 50 | 8.6 |
| 105.0 | 281 | 0.11 | 0.38 | 10 | 19.02 | 50 | 8.6 |
| 106.7 | 299 | 0.11 | 0.38 | 10 | 19.01 | 50 | 8.3 |
| 108.3 | 300 | 0.11 | 0.38 | 10 | 18.99 | 50 | 8.3 |
| 110.0 | 321 | 0.11 | 0.38 | 10 | 19.00 | 50 | 8.1 |
| 111.7 | 320 | 0.11 | 0.38 | 10 | 19.14 | 50 | 8.0 |
| 113.3 | 339 | 0.11 | 0.38 | 10 | 19.27 | 51 | 7.7 |
| 115.0 | 340 | 0.11 | 0.38 | 10 | 19.23 | 50 | 7.7 |
| 116.7 | 360 | 0.11 | 0.38 | 10 | 19.15 | 50 | 7.5 |
| 118.3 | 360 | 0.11 | 0.38 | 10 | 19.00 | 50 | 7.6 |
| 120.0 | 201 | 0.11 | 0.38 | 10 | 11.46 | 30 | 13.5 |
| 121.7 | 202 | 0.11 | 0.38 | 10 | 11.51 | 30 | 13.5 |
| 123.3 | 200 | 0.11 | 0.38 | 10 | 11.44 | 30 | 13.6 |
| 125.0 | 218 | 0.11 | 0.38 | 10 | 11.50 | 30 | 13.0 |
| 126.7 | 219 | 0.11 | 0.38 | 10 | 11.40 | 30 | 13.1 |
| 128.3 | 241 | 0.11 | 0.38 | 10 | 11.52 | 30 | 12.4 |
| 130.0 | 242 | 0.11 | 0.38 | 10 | 11.52 | 30 | 12.5 |
| 131.7 | 261 | 0.11 | 0.38 | 10 | 11.52 | 30 | 12.0 |
| 133.3 | 258 | 0.11 | 0.38 | 10 | 11.52 | 30 | 12.1 |
| 135.0 | 280 | 0.11 | 0.38 | 10 | 11.53 | 30 | 11.6 |
| 136.7 | 279 | 0.11 | 0.38 | 10 | 11.53 | 30 | 11.6 |
| 138.3 | 299 | 0.11 | 0.38 | 10 | 11.43 | 30 | 11.2 |
| 140.0 | 300 | 0.11 | 0.38 | 10 | 11.53 | 30 | 11.2 |

**Table 9: Example 4 GC analysis of product stream**

| **Time Hr** | **GC FID area%** | | | | |
|---|---|---|---|---|---|
| | **1233xf** | **1232xf** | **1231 isomer** | **1230xa** | **others** |
| 1.7 | 60.83% | 29.17% | 2.70% | 0.00% | 7.31% |
| 3.3 | 54.88% | 40.82% | 1.81% | 0.00% | 2.50% |
| 5.0 | 45.01% | 51.14% | 1.57% | 0.00% | 2.28% |
| 6.7 | 38.63% | 57.89% | 1.39% | 0.00% | 2.09% |
| 8.3 | 35.35% | 61.42% | 1.21% | 0.00% | 2.03% |
| 10.0 | 32.31% | 64.58% | 1.12% | 0.00% | 1.99% |
| 11.7 | 30.52% | 66.62% | 1.00% | 0.00% | 1.87% |
| 13.3 | 27.35% | 69.72% | 0.94% | 0.00% | 1.99% |
| 15.0 | 27.58% | 69.83% | 0.86% | 0.00% | 1.73% |
| 16.7 | 20.37% | 75.61% | 1.29% | 0.00% | 2.73% |
| 18.3 | 19.63% | 76.40% | 1.28% | 0.00% | 2.69% |
| 20.0 | 20.00% | 75.83% | 1.38% | 0.00% | 2.79% |
| 21.7 | 19.61% | 76.39% | 1.26% | 0.00% | 2.74% |
| 23.3 | 19.39% | 76.34% | 1.49% | 0.00% | 2.79% |
| 25.0 | 19.65% | 75.86% | 1.53% | 0.00% | 2.96% |
| 26.7 | 19.26% | 76.18% | 1.60% | 0.00% | 2.96% |
| 28.3 | 22.08% | 74.51% | 1.11% | 0.00% | 2.29% |
| 30.0 | 20.95% | 75.52% | 1.27% | 0.00% | 2.26% |
| 31.7 | 20.76% | 75.58% | 1.34% | 0.00% | 2.32% |
| 33.3 | 20.38% | 75.66% | 1.51% | 0.00% | 2.45% |
| 35.0 | 20.02% | 75.46% | 1.54% | 0.00% | 2.99% |
| 36.7 | 19.87% | 76.16% | 1.48% | 0.00% | 2.50% |
| 38.3 | 19.47% | 76.53% | 1.48% | 0.00% | 2.52% |
| 40.0 | 19.22% | 76.13% | 1.57% | 0.00% | 3.08% |
| 41.7 | 19.39% | 75.70% | 1.70% | 0.00% | 3.21% |
| 43.3 | 19.55% | 76.18% | 1.64% | 0.00% | 2.64% |
| 45.0 | 19.44% | 76.26% | 1.58% | 0.00% | 2.73% |
| 46.7 | 19.16% | 76.63% | 1.54% | 0.00% | 2.67% |
| 48.3 | 18.57% | 77.26% | 1.47% | 0.00% | 2.70% |
| 50.0 | 18.96% | 76.84% | 1.46% | 0.00% | 2.75% |
| 51.7 | 19.20% | 76.31% | 1.69% | 0.00% | 2.79% |
| 53.3 | 19.27% | 76.87% | 1.26% | 0.00% | 2.60% |
| 55.0 | 19.15% | 76.56% | 1.54% | 0.00% | 2.74% |
| 56.7 | 24.82% | 70.89% | 1.85% | 0.00% | 2.44% |
| 58.3 | 24.87% | 71.47% | 1.49% | 0.00% | 2.17% |
| 60.0 | 23.78% | 72.79% | 1.22% | 0.00% | 2.21% |
| 61.7 | 23.99% | 73.05% | 1.10% | 0.00% | 1.85% |
| 63.3 | 26.06% | 71.83% | 0.70% | 0.00% | 1.42% |
| 65.0 | 23.19% | 74.03% | 0.96% | 0.00% | 1.82% |
| 66.7 | 22.97% | 74.36% | 0.80% | 0.00% | 1.87% |
| 68.3 | 22.55% | 74.69% | 0.88% | 0.00% | 1.88% |
| 70.0 | 22.69% | 74.54% | 0.89% | 0.00% | 1.87% |
| 71.7 | 21.95% | 75.20% | 0.91% | 0.00% | 1.94% |
| 73.3 | 20.88% | 75.83% | 0.98% | 0.00% | 2.32% |
| 75.0 | 20.62% | 76.55% | 0.82% | 0.00% | 2.01% |
| 76.7 | 20.73% | 76.44% | 0.87% | 0.00% | 1.96% |
| 78.3 | 20.96% | 75.64% | 0.87% | 0.00% | 2.53% |
| 80.0 | 20.71% | 76.18% | 0.79% | 0.00% | 2.32% |
| 81.7 | 20.47% | 76.36% | 0.81% | 0.00% | 2.36% |
| 83.3 | 20.15% | 76.61% | 0.78% | 0.00% | 2.46% |
| 85.0 | 20.26% | 76.31% | 0.92% | 0.00% | 2.51% |
| 86.7 | 20.46% | 76.23% | 0.89% | 0.00% | 2.42% |
| 88.3 | 21.93% | 74.22% | 1.06% | 0.00% | 2.79% |
| 90.0 | 21.50% | 74.88% | 0.89% | 0.00% | 2.73% |
| 91.7 | 21.54% | 74.89% | 0.88% | 0.00% | 2.69% |
| 93.3 | 20.98% | 75.46% | 0.91% | 0.00% | 2.65% |
| 95.0 | 20.35% | 76.12% | 0.85% | 0.00% | 2.68% |
| 96.7 | 19.88% | 76.57% | 0.87% | 0.00% | 2.68% |
| 98.3 | 20.15% | 76.09% | 1.00% | 0.00% | 2.76% |
| 100.0 | 19.77% | 76.40% | 1.06% | 0.00% | 2.77% |
| 101.7 | 19.77% | 76.57% | 0.93% | 0.00% | 2.72% |
| 103.3 | 19.03% | 77.28% | 0.99% | 0.00% | 2.69% |
| 105.0 | 18.48% | 77.83% | 0.97% | 0.00% | 2.72% |
| 106.7 | 18.29% | 78.04% | 0.91% | 0.00% | 2.76% |
| 108.3 | 17.81% | 78.57% | 0.88% | 0.00% | 2.73% |
| 110.0 | 18.34% | 77.92% | 0.95% | 0.00% | 2.78% |
| 111.7 | 18.42% | 77.92% | 0.91% | 0.00% | 2.74% |
| 113.3 | 19.00% | 77.21% | 1.01% | 0.00% | 2.79% |
| 115.0 | 18.86% | 77.42% | 1.01% | 0.00% | 2.71% |
| 116.7 | 19.13% | 77.18% | 0.97% | 0.00% | 2.72% |
| 118.3 | 18.86% | 77.56% | 0.88% | 0.00% | 2.70% |
| 120.0 | 24.80% | 70.40% | 1.19% | 0.00% | 3.61% |
| 121.7 | 22.80% | 73.07% | 1.02% | 0.00% | 3.12% |
| 123.3 | 22.66% | 73.05% | 1.02% | 0.00% | 3.27% |
| 125.0 | 21.89% | 74.07% | 0.93% | 0.00% | 3.10% |
| 126.7 | 21.62% | 74.33% | 0.97% | 0.00% | 3.08% |
| 128.3 | 20.66% | 75.23% | 1.06% | 0.00% | 3.05% |
| 130.0 | 19.86% | 76.06% | 1.04% | 0.00% | 3.04% |
| 131.7 | 19.71% | 76.06% | 1.13% | 0.00% | 3.10% |
| 133.3 | 19.08% | 77.04% | 0.98% | 0.00% | 2.90% |
| 135.0 | 18.71% | 77.21% | 1.06% | 0.00% | 3.02% |
| 136.7 | 18.89% | 76.79% | 1.16% | 0.00% | 3.15% |
| 138.3 | 18.44% | 77.16% | 1.20% | 0.00% | 3.21% |
| 140.0 | 17.74% | 77.55% | 1.22% | 0.00% | 3.49% |

### Example 5: Hydrochlorination of HCFO-1233xf to HCFO-1232xf

4ml 12-20mesh of chromia catalyst is loaded into a 12 inches long Inconel (0.5-inch OD) tube reactor. The catalyst is activated by HF starting from 150°C to 450°C. HCFO-1233xf is fed by a pump at a 0.5ml/hr rate through a vaporizer at 100°C with 9 sccm N2 and then is mixed with 15 sccm HCl passed through the reactor catalyst bed at 250°C. The reactor effluent is analyzed by a GC-MS-FID and shows 40% HCFO-1232xf present in the organic product stream.

### Example 6: Hydrogenation of HCFO-1232xf

### to HCFC-252dc with 0.5% Pd/C and a solvent

5g 0.5% Pd/C catalyst, 25g 1232xf and 7g isopropanol was added into a 400ml Hastelloy C shaker tube. After shaker tube is sealed, it was chilled to 0°C and evacuated. Then, the mixture was heated to 60°C and H₂ was added to 150 psig with agitation, the pressure drop of reactor indicating progress of reaction. After reactor pressure was dropped to 118 psig, the reactor was pressured up with H₂ again to 150 psig, and reactor pressure dropped to 143 psig in 2 hrs. Then, the reactor was chilled to 0°C and pressure was released. The product recovered was analyzed by GC, and GC analysis showed formation of HCFC-252dc, as shown in Table 10.

**Table 10**

| **Compounds** | **FID area%** |
|---|---|
| 1233xf | 1.3677% |
| 1242xc (CF₂=CCl-CH₃) | 5.7855% |
| isopropanol | 34.0366% |
| 1232xf | 46.7582% |
| 252dc | 7.8488% |
| others | 4.2032% |

### Example 7: Hydrogenation of HCFO-1232xf

### to HCFC-252dc with 0.5% Pd/C and w/o solvent

8 g 0.5% Pd/C catalyst and 200 g HCFO-1232xf was added into a 400ml Hastelloy C shaker tube. After shaker tube was sealed, it was chilled to -30°C and evacuated. Then, the mixture was heated to 90°C and H₂ was added to 200 psig with agitation, the pressure drop of reactor indicating progress of reaction. After reactor pressure was dropped to 145 psig, the reactor was pressured up with H₂ again to 200 psig. This process was repeated multiple times until the pressure did not drop any more. Then, the reactor was chilled to -30°C and pressure was released. The product recovered was analyzed by GC, and GC analysis showed formation of HCFC-252dc and HFO-1252zc, as shown in Table 11.

**Table 11**

| **Compounds** | | **FID GC area%** |
|---|---|---|
| 1252zc | CF₂=CHCH₃ | 1.2761% |
| 272fb | HCF₂CH₂CH₃ | 1.4588% |
| 1233xf | CF₃CCl=CH₂ | 1.5427% |
| 253db | CF₃CHClCH₃ | 1.1468% |
| 1242xc | CF₂=CClCH₃ | 5.4282% |
| 263fb | CF₃CH₂CH₃ | 3.3335% |
| 262fc | CClF₂CH₂CH₃ | 0.3736% |
| 243 isomer | C₃H₃Cl₂F₃ | 1.0163% |
| 1223xd | CF₃CCl=CHCl | 0.1157% |
| 1232xf | CClF₂CCl=CH₂ | 21.0833% |
| 252dc | CClF₂CHClCH₃ | 62.2155% |

### Example 8: Hydrogenation of HCFO-1232xf to HCFC-252dc with 1% Pd/C

4ml 12-20mesh of 1% Pd/C catalyst is loaded into a 12 inches long Inconel (0.5-inch OD) tube reactor. The catalyst is activated by 30 sccm H₂ at 200°C for 30 min. HCFO-1233xf is fed by a pump at a 0.5ml/hr rate passes through a vaporizer at 100°C with 9 sccm N₂ and then is mixed with 20 sccm H₂ and flows through the reactor at 200°C with catalyst bed. The reactor effluent is analyzed by a GC-MS-FID and shows 35% HCFC-252dc present in the organic product stream.

### Example 9: Hydrogenation of HCFO-1232xf

### to HCFC-252dc by 0.02% Pd/Al₂O₃ catalyst

4 ml 0.1% Pd/Al₂O₃ catalyst was loaded into a 12 inches long Inconel (0.5 inch OD) tube reactor. The catalyst was activated by 15 sccm H₂ at 120°C for 120 min. HCFO-1232xf was fed by a pump and passed through a vaporizer at 100°C. The tests were done at conditions listed in the Table 12 below. The reactor effluent was analyzed by a GC-MS-FID. The analysis is summarized in Table 13 and shows HCFC-252dc was formed during the tests.

**Table 12**

| **Time** | **T** | **P** | **1232xf** | **N2** | **H2** |
|---|---|---|---|---|---|
| **hr** | **C** | **psig** | **cc/hr** | **sccm** | **sccm** |
| 1 | 39 | 2.9 | 1.00 | 5.00 | 7.06 |
| 2 | 40 | 2.8 | 1.00 | 5.00 | 7.06 |
| 3 | 50 | 2.9 | 1.00 | 5.00 | 7.07 |
| 4 | 50 | 2.9 | 1.00 | 5.00 | 7.07 |
| 5 | 59 | 2.9 | 1.00 | 5.00 | 7.07 |
| 6 | 59 | 2.9 | 1.00 | 5.00 | 7.06 |
| 7 | 71 | 2.9 | 1.00 | 5.00 | 7.09 |
| 8 | 71 | 2.8 | 1.00 | 5.00 | 7.09 |
| 9 | 81 | 2.8 | 1.00 | 5.00 | 7.05 |
| 10 | 80 | 2.8 | 1.00 | 5.00 | 7.05 |
| 11 | 90 | 5.0 | 1.00 | 0.00 | 10.01 |
| 12 | 90 | 5.0 | 1.00 | 0.00 | 10.01 |
| 13 | 90 | 5.0 | 1.00 | 0.00 | 10.00 |
| 14 | 101 | 5.0 | 1.00 | 0.00 | 9.99 |
| 15 | 100 | 5.0 | 1.00 | 0.00 | 9.99 |
| 16 | 110 | 5.0 | 1.00 | 0.00 | 10.00 |
| 17 | 110 | 5.0 | 1.00 | 0.00 | 10.00 |
| 18 | 120 | 5.0 | 1.00 | 0.00 | 10.00 |
| 19 | 120 | 5.0 | 1.00 | 0.00 | 10.00 |

**Table 13**

| **Time hr** | **GC FID Area% of product stream** | | | | | |
|---|---|---|---|---|---|---|
| | **Propane** | **1243zf** | **1252zc** | **263fb** | **1233xf** | **1242zf** |
| 1 | 0.00% | 0.09% | 0.04% | 0.07% | 3.99% | 0.10% |
| 2 | 0.00% | 0.10% | 0.03% | 0.07% | 4.05% | 0.08% |
| 3 | 0.00% | 0.15% | 0.07% | 0.14% | 3.91% | 0.16% |
| 4 | 0.00% | 0.12% | 0.08% | 0.12% | 3.74% | 0.16% |
| 5 | 0.00% | 0.14% | 0.21% | 0.19% | 3.73% | 0.27% |
| 6 | 0.00% | 0.11% | 0.18% | 0.14% | 3.65% | 0.24% |
| 7 | 0.03% | 0.11% | 0.40% | 0.20% | 3.69% | 0.37% |
| 8 | 0.03% | 0.10% | 0.36% | 0.16% | 3.62% | 0.33% |
| 9 | 0.07% | 0.10% | 0.47% | 0.13% | 3.63% | 0.43% |
| 10 | 0.08% | 0.10% | 0.34% | 0.10% | 3.53% | 0.37% |
| 11 | 0.17% | 0.12% | 0.53% | 0.12% | 3.51% | 0.59% |
| 12 | 0.17% | 0.14% | 0.36% | 0.11% | 3.59% | 0.50% |
| 13 | 0.15% | 0.14% | 0.24% | 0.11% | 4.74% | 0.42% |
| 14 | 0.25% | 0.16% | 0.38% | 0.00% | 4.15% | 0.54% |
| 15 | 0.23% | 0.14% | 0.36% | 0.00% | 3.32% | 0.52% |
| 16 | 0.36% | 0.21% | 0.47% | 0.00% | 3.22% | 0.69% |
| 17 | 0.33% | 0.20% | 0.47% | 0.00% | 3.77% | 0.66% |
| 18 | 0.50% | 0.31% | 0.77% | 0.00% | 3.24% | 0.91% |
| 19 | 0.46% | 0.28% | 0.92% | 0.00% | 2.86% | 0.94% |

**Table 13 (Continued)**

| **Time hr** | **GC FID Area% of product stream** | | | | | | |
|---|---|---|---|---|---|---|---|
| | **1242xf** | **1251** | **253db** | **1232xf** | **252dc** | **1241xb** | **others** |
| 1 | 0.31% | 0.00% | 1.11% | 84.75% | 3.81% | 0.23% | 5.52% |
| 2 | 0.34% | 0.00% | 1.11% | 85.56% | 2.03% | 0.26% | 6.36% |
| 3 | 0.33% | 0.00% | 1.11% | 84.44% | 3.40% | 0.29% | 6.00% |
| 4 | 0.33% | 0.00% | 1.11% | 85.81% | 3.57% | 0.24% | 4.73% |
| 5 | 0.32% | 0.04% | 1.10% | 82.67% | 6.41% | 0.27% | 4.66% |
| 6 | 0.31% | 0.04% | 1.10% | 83.36% | 5.63% | 0.28% | 4.96% |
| 7 | 0.28% | 0.06% | 1.11% | 80.38% | 8.19% | 0.32% | 4.87% |
| 8 | 0.28% | 0.07% | 1.10% | 81.09% | 8.28% | 0.30% | 4.29% |
| 9 | 0.22% | 0.11% | 1.12% | 80.67% | 8.02% | 0.37% | 4.65% |
| 10 | 0.21% | 0.12% | 1.12% | 82.77% | 6.40% | 0.40% | 4.46% |
| 11 | 0.16% | 0.13% | 1.10% | 77.57% | 9.94% | 0.63% | 5.42% |
| 12 | 0.16% | 0.14% | 1.11% | 80.19% | 7.43% | 0.65% | 5.44% |
| 13 | 0.17% | 0.15% | 1.08% | 82.21% | 5.64% | 0.61% | 4.35% |
| 14 | 0.12% | 0.23% | 1.11% | 81.88% | 4.85% | 0.93% | 5.39% |
| 15 | 0.12% | 0.21% | 1.12% | 83.63% | 4.62% | 0.86% | 4.86% |
| 16 | 0.10% | 0.31% | 1.13% | 81.84% | 4.77% | 1.21% | 5.68% |
| 17 | 0.11% | 0.30% | 1.16% | 82.32% | 4.80% | 1.09% | 4.79% |
| 18 | 0.09% | 0.41% | 1.16% | 79.70% | 5.66% | 1.46% | 5.79% |
| 19 | 0.10% | 0.39% | 1.22% | 79.66% | 6.68% | 1.26% | 5.23% |

### Example 10: Hydrogenation of HCFO-1232xf

### to HCFC-252dc by 0.1% Pd/Al₂O₃ catalyst

6 ml 0.1% Pd/Al₂O₃ catalyst was loaded into a 12 inches long Inconel (0.5-inch OD) tube reactor. The catalyst was activated by 15 sccm H₂ at 120°C for 120 minutes. HCFO-1232xf was fed by a pump and passed through a vaporizer at 100°C. The tests were done at conditions listed in the Table 14 below. The reactor effluent was analyzed by a GC-MS-FID. The analysis is summarized in Table 15 and shows HCFC-252dc was formed with high selectivity.

**Table 14**

| **Time** | **T** | **P** | **1232xf** | **H2** |
|---|---|---|---|---|
| **hr** | **C** | **psig** | **cc/hr** | **sccm** |
| 1 | 60 | 2.7 | 1.00 | 10.01 |
| 2 | 60 | 2.6 | 1.00 | 10.01 |
| 3 | 60 | 2.6 | 1.00 | 10.01 |
| 4 | 71 | 2.6 | 1.00 | 10.19 |
| 5 | 71 | 2.6 | 1.00 | 10.19 |
| 6 | 81 | 2.6 | 1.00 | 10.19 |
| 7 | 81 | 2.6 | 1.00 | 10.00 |
| 8 | 91 | 2.6 | 1.00 | 10.00 |
| 9 | 91 | 2.6 | 1.00 | 10.00 |
| 10 | 101 | 2.7 | 1.00 | 10.00 |
| 11 | 101 | 2.7 | 1.00 | 10.00 |
| 12 | 109 | 25.0 | 1.00 | 19.97 |
| 13 | 110 | 25.0 | 1.00 | 19.97 |
| 14 | 121 | 25.0 | 1.00 | 19.97 |
| 15 | 120 | 25.0 | 1.00 | 20.00 |
| 16 | 120 | 35.0 | 1.50 | 30.06 |
| 17 | 130 | 35.0 | 1.50 | 30.03 |
| 18 | 130 | 35.0 | 1.50 | 30.03 |
| 19 | 140 | 35.0 | 1.50 | 30.03 |
| 20 | 140 | 35.0 | 1.50 | 30.03 |

**Table 15**

| **Time hr** | **GC FID Area% of product stream** | | | | | | |
|---|---|---|---|---|---|---|---|
| | **propane** | **1243zf** | **1252zc** | **272fb** | **253 isomer** | **1233xf** | **1242zf** |
| 1 | 0.00% | 0.06% | 0.00% | 0.00% | 0.21% | 2.86% | 0.31% |
| 2 | 0.00% | 0.09% | 0.00% | 0.00% | 0.00% | 3.16% | 0.30% |
| 3 | 0.00% | 0.07% | 0.00% | 0.00% | 0.00% | 3.42% | 0.28% |
| 4 | 0.00% | 0.09% | 1.26% | 0.05% | 0.25% | 2.69% | 0.35% |
| 5 | 0.00% | 0.08% | 0.89% | 0.03% | 0.23% | 2.99% | 0.31% |
| 6 | 0.02% | 0.08% | 1.27% | 0.07% | 0.27% | 3.04% | 0.49% |
| 7 | 0.02% | 0.07% | 0.95% | 0.05% | 0.24% | 2.92% | 0.45% |
| 8 | 0.04% | 0.08% | 1.54% | 0.10% | 0.25% | 3.01% | 0.69% |
| 9 | 0.04% | 0.07% | 1.29% | 0.09% | 0.24% | 2.92% | 0.68% |
| 10 | 0.06% | 0.08% | 1.68% | 0.12% | 0.26% | 2.97% | 0.94% |
| 11 | 0.06% | 0.08% | 1.65% | 0.12% | 0.24% | 2.98% | 0.97% |
| 12 | 0.18% | 0.19% | 2.60% | 0.00% | 0.00% | 0.23% | 2.55% |
| 13 | 0.02% | 0.04% | 0.00% | 0.00% | 0.30% | 2.58% | 2.28% |
| 14 | 0.09% | 0.12% | 2.48% | 0.00% | 0.00% | 0.00% | 2.88% |
| 15 | 0.11% | 0.14% | 2.76% | 5.24% | 0.22% | 0.00% | 3.47% |
| 16 | 0.13% | 0.24% | 0.00% | 7.24% | 0.37% | 0.00% | 3.12% |
| 17 | 0.07% | 0.15% | 3.70% | 5.76% | 0.35% | 0.00% | 4.63% |
| 18 | 0.05% | 0.12% | 4.23% | 5.27% | 0.35% | 0.00% | 5.27% |
| 19 | 0.08% | 0.13% | 5.35% | 5.69% | 0.36% | 0.00% | 6.81% |
| 20 | 0.06% | 0.11% | 5.78% | 4.68% | 0.35% | 0.00% | 7.47% |

**Table 15 (Continued)**

| **Time hr** | **GC FID Area% of product stream** | | | | | | |
|---|---|---|---|---|---|---|---|
| | **1242xf** | **1251** | **253db** | **1232xf** | **252dc** | **1241xb** | **others** |
| 1 | 0.38% | 0.00% | 1.20% | 86.07% | 4.35% | 0.17% | 4.39% |
| 2 | 0.39% | 0.00% | 1.12% | 85.88% | 4.51% | 0.21% | 4.34% |
| 3 | 0.38% | 0.00% | 1.11% | 85.99% | 4.74% | 0.19% | 3.81% |
| 4 | 0.27% | 0.00% | 1.11% | 76.11% | 14.47% | 0.17% | 3.19% |
| 5 | 0.35% | 0.00% | 1.10% | 79.86% | 10.77% | 0.17% | 3.22% |
| 6 | 0.27% | 0.02% | 1.10% | 76.51% | 13.65% | 0.18% | 3.04% |
| 7 | 0.34% | 0.02% | 1.10% | 79.40% | 11.43% | 0.17% | 2.83% |
| 8 | 0.26% | 0.03% | 1.10% | 75.54% | 14.21% | 0.19% | 2.95% |
| 9 | 0.33% | 0.03% | 1.09% | 77.58% | 12.57% | 0.19% | 2.88% |
| 10 | 0.25% | 0.04% | 1.10% | 76.05% | 12.98% | 0.22% | 3.26% |
| 11 | 0.26% | 0.04% | 1.11% | 76.64% | 12.63% | 0.21% | 3.02% |
| 12 | 0.36% | 0.00% | 1.16% | 0.00% | 84.73% | 0.00% | 8.00% |
| 13 | 0.14% | 0.02% | 1.12% | 52.70% | 34.78% | 0.11% | 5.91% |
| 14 | 0.00% | 0.00% | 1.13% | 21.27% | 65.07% | 0.08% | 6.90% |
| 15 | 0.00% | 0.00% | 1.14% | 9.19% | 74.84% | 0.00% | 2.89% |
| 16 | 0.61% | 0.00% | 1.13% | 0.10% | 81.59% | 0.00% | 5.48% |
| 17 | 0.00% | 0.00% | 1.09% | 1.58% | 78.47% | 0.04% | 4.18% |
| 18 | 0.00% | 0.00% | 1.09% | 1.22% | 78.10% | 0.04% | 4.27% |
| 19 | 0.00% | 0.00% | 1.09% | 1.31% | 74.46% | 0.04% | 4.68% |
| 20 | 0.00% | 0.00% | 1.09% | 2.14% | 73.63% | 0.04% | 4.65% |

### Example 11: Hydrogenation of HCFC-252dc to HFO-1252zc with 8% Cu/C

4ml 12-20mesh of 8%Cu/C catalyst is loaded into a 12 inches long Inconel (0.5-inch OD) tube reactor. The catalyst is activated by 30 sccm H₂ at 200°C for 30 min. HCFC-252dc is fed by a pump at a 0.5 ml/hr rate and goes through a vaporizer at 105°C with 9 sccm N₂, and then is mixed with 20 sccm H₂ and flows through the reactor at 400°C with catalyst bed. The reactor effluent is analyzed by a GC-MS-FID and the analysis shows 30% HFO-1252zc present in the organic product stream.

### Example 12: Reaction of HCFC-252dc with a Zinc Compound to HFO-1252zc

10g Zn powder previously activated by aqueous HCl and dried under N₂ is added into an autoclave together with 20g HCFC-252dc and 20g methanol. After the autoclave is sealed, the reaction mixture is heated to 100°C with agitation and agitated at 100°C for 2 hours. The increase of pressure indicated the progress of reaction. At end of 2 hours, a sample from vapor side of reactor is collected into a sample bag and analyzed by GC-MS-FID. The GC result shows 50% of HFO-1252zc in the vapor sample.

### Example 13: Hydrodechlorination of

### HCFC-252dc to HFO-1252zc with 10% Cu/C

6 ml 10%Cu/C catalyst was loaded into a 12 inches long Inconel (0.5-inch OD) tube reactor. The catalyst was activated by 20 sccm H₂ at 450°C for 120 minutes. HCFC-252dc was fed by a pump and passed through a vaporizer at 100°C. The tests were done at conditions listed in the Table 16 below. The reactor effluent was analyzed by a GC-MS-FID, and the analysis (summarized in Tables 17 and 18) shows HFO-1252zc was formed with high selectivity.

**Table 16**

| **Time hr** | **Temp C** | **Pressure psi** | **252dc cc/hr** | **N2 sccm** | **H2 sccm** |
|---|---|---|---|---|---|
| 1 | 180 | 20.0 | 0.17 | 10.05 | 6.08 |
| 2 | 180 | 20.0 | 0.17 | 10.02 | 6.08 |
| 3 | 180 | 20.0 | 0.17 | 10.02 | 6.07 |
| 4 | 200 | 20.0 | 0.17 | 10.02 | 6.07 |
| 5 | 200 | 20.0 | 0.17 | 10.02 | 6.05 |
| 6 | 200 | 20.0 | 0.17 | 10.02 | 6.05 |
| 7 | 220 | 20.0 | 0.17 | 10.02 | 6.05 |
| 8 | 220 | 20.0 | 0.17 | 10.02 | 6.05 |
| 9 | 220 | 20.0 | 0.17 | 10.02 | 6.05 |
| 10 | 241 | 20.0 | 0.17 | 10.02 | 6.07 |
| 11 | 241 | 20.0 | 0.17 | 10.02 | 6.05 |
| 12 | 241 | 20.0 | 0.17 | 10.02 | 6.05 |
| 13 | 260 | 20.0 | 0.17 | 10.02 | 6.06 |
| 14 | 260 | 20.0 | 0.17 | 10.02 | 6.06 |
| 15 | 260 | 20.0 | 0.17 | 10.02 | 6.06 |
| 16 | 260 | 20.0 | 0.17 | 10.02 | 6.06 |

**Table 17**

| **Time hr** | **GC-FID area% of product stream** | | | | |
|---|---|---|---|---|---|
| | **1252zc** | **1243zf** | **262fc** | **E-1251 zb** | **Z-1251 zb** |
| 1 | 16.77% | 0.03% | 0.13% | 0.07% | 0.01% |
| 2 | 13.34% | 0.03% | 0.07% | 0.07% | 0.00% |
| 3 | 11.52% | 0.02% | 0.05% | 0.05% | 0.00% |
| 4 | 15.17% | 0.04% | 0.06% | 0.06% | 0.00% |
| 5 | 12.78% | 0.02% | 0.06% | 0.07% | 0.00% |
| 6 | 10.88% | 0.02% | 0.05% | 0.07% | 0.00% |
| 7 | 16.96% | 0.03% | 0.20% | 0.10% | 0.02% |
| 8 | 16.02% | 0.03% | 0.23% | 0.10% | 0.02% |
| 9 | 13.83% | 0.03% | 0.21% | 0.09% | 0.02% |
| 10 | 25.78% | 0.05% | 1.29% | 0.18% | 0.04% |
| 11 | 26.06% | 0.06% | 1.40% | 0.20% | 0.05% |
| 12 | 25.62% | 0.05% | 1.47% | 0.20% | 0.05% |
| 13 | 40.67% | 0.08% | 6.08% | 0.52% | 0.16% |
| 14 | 45.14% | 0.08% | 6.86% | 0.56% | 0.16% |
| 15 | 45.93% | 0.09% | 6.94% | 0.59% | 0.16% |
| 16 | 45.99% | 0.09% | 7.13% | 0.60% | 0.15% |

**Table 17 (Continued)**

| **Time hr** | **GC-FID area% of product stream** | | | | | |
|---|---|---|---|---|---|---|
| | **272fb** | **252dc** | **1241xb** | **1241xb** | **262db** | **others** |
| 1 | 0.00% | 82.21% | 0.08% | 0.06% | 0.05% | 0.5875% |
| 2 | 0.00% | 85.71% | 0.08% | 0.08% | 0.05% | 0.5681% |
| 3 | 0.00% | 87.56% | 0.09% | 0.08% | 0.04% | 0.5897% |
| 4 | 0.00% | 84.17% | 0.08% | 0.08% | 0.06% | 0.2955% |
| 5 | 0.00% | 86.59% | 0.08% | 0.07% | 0.04% | 0.3033% |
| 6 | 0.00% | 88.50% | 0.08% | 0.06% | 0.04% | 0.3105% |
| 7 | 0.00% | 82.37% | 0.08% | 0.06% | 0.06% | 0.1240% |
| 8 | 0.00% | 83.24% | 0.08% | 0.05% | 0.05% | 0.1698% |
| 9 | 0.00% | 85.48% | 0.08% | 0.05% | 0.05% | 0.1645% |
| 10 | 0.02% | 72.39% | 0.07% | 0.04% | 0.03% | 0.1045% |
| 11 | 0.03% | 71.94% | 0.07% | 0.04% | 0.03% | 0.1180% |
| 12 | 0.03% | 72.33% | 0.07% | 0.04% | 0.02% | 0.1239% |
| 13 | 0.13% | 52.06% | 0.06% | 0.03% | 0.03% | 0.1860% |
| 14 | 0.14% | 46.83% | 0.06% | 0.03% | 0.00% | 0.1466% |
| 15 | 0.14% | 45.90% | 0.06% | 0.02% | 0.00% | 0.1552% |
| 16 | 0.12% | 45.67% | 0.06% | 0.03% | 0.00% | 0.1532% |

**Table 18: GC analysis of reaction product at 13 hours**

| **Compounds** | **GC-FID area%** |
|---|---|
| methane | 0.0011% |
| 1261ze | 0.0056% |
| 1252zc | 40.6943% |
| 1243zf | 0.0138% |
| 262fc | 6.0905% |
| 1242zf | 0.4957% |
| 1251zb | 0.1329% |
| 1242xf | 0.0222% |
| 272fb | 0.1273% |
| 253db | 0.0816% |
| 1232xf | 0.0473% |
| 252dc | 52.2432% |
| 1241xb | 0.0117% |
| 1241xb | 0.0118% |
| 262db | 0.0183% |
| others | 0.0028% |

### Example 14: Hydrodechlorination of

### HCFC-252dc to HFO-1252zc with 1% Au/Al₂O₃

5 ml 1%Au/Al₂O₃ catalyst was loaded into a 12 inches long Inconel (0.5-inch OD) tube reactor. The catalyst was activated by 20 sccm H₂ at 350°C for 120 minutes. HCFC-252dc was fed by a pump and passed through a vaporizer at 100°C. The tests were done at conditions listed in the Table 19 below. The reactor effluent was analyzed by a GC-MS-FID, and the analysis shows HFO-1252zc was formed in these tests (see Table 20).

**Table 19**

| **Time hr** | **T C** | **P psig** | **252dc cc/hr** | **N2 sccm** | **H2 sccm** |
|---|---|---|---|---|---|
| 1 | 160 | 20.0 | 0.30 | 12.22 | 10.01 |
| 2 | 160 | 20.0 | 0.30 | 12.22 | 10.00 |
| 3 | 160 | 20.0 | 0.30 | 12.22 | 10.00 |
| 4 | 180 | 20.0 | 0.30 | 12.22 | 10.00 |
| 5 | 180 | 20.0 | 0.30 | 12.22 | 10.00 |
| 6 | 200 | 20.0 | 0.30 | 12.22 | 10.01 |
| 7 | 200 | 20.0 | 0.30 | 12.22 | 9.96 |
| 8 | 220 | 20.0 | 0.30 | 12.22 | 9.96 |
| 9 | 220 | 20.0 | 0.30 | 12.22 | 9.96 |

**Table 20**

| **Time hr** | **FID area%** | | | | | | | | |
|---|---|---|---|---|---|---|---|---|---|
| | **1252zc** | **1261ze** | **1260** | **1251zd** | **252dc** | **1241xb** | **1241xb** | **1250xd** | **others** |
| 1 | 0.00% | 0.00% | 0.00% | 0.46% | 96.11% | 0.34% | 0.39% | 0.15% | 2.55% |
| 2 | 0.00% | 0.00% | 0.00% | 0.47% | 96.02% | 0.33% | 0.37% | 0.17% | 2.64% |
| 3 | 0.00% | 0.00% | 0.00% | 0.44% | 96.23% | 0.31% | 0.36% | 0.13% | 2.53% |
| 4 | 3.82% | 0.00% | 0.00% | 0.39% | 93.47% | 0.78% | 0.97% | 0.07% | 0.50% |
| 5 | 3.96% | 0.00% | 0.15% | 0.39% | 92.82% | 0.82% | 1.02% | 0.09% | 0.75% |
| 6 | 5.70% | 1.93% | 0.13% | 0.45% | 86.46% | 2.02% | 2.63% | 0.08% | 0.60% |
| 7 | 4.81% | 1.74% | 0.12% | 0.41% | 87.45% | 1.95% | 2.58% | 0.00% | 0.94% |
| 8 | 8.84% | 6.19% | 0.18% | 0.00% | 67.87% | 6.10% | 8.13% | 0.00% | 2.69% |
| 9 | 6.14% | 5.00% | 0.12% | 0.55% | 75.30% | 5.07% | 6.77% | 0.00% | 1.06% |

### Example 15: Hydrodechlorination of HCFC-252dc to HFO-1252zc with 4% Au/C

4ml 4% Au/C catalyst was loaded into a 12 inches long Inconel (0.5-inch OD) tube reactor. The catalyst was activated by 20 sccm H₂ at 350°C for 120 minutes. HCFC-252dc was fed by a pump and passes through a vaporizer at 100°C. The tests were done at conditions listed in the Table 21 below. The reactor effluent was analyzed by a GC-MS-FID, and the analysis shows HFO-1252zc was formed with high selectivity (see Tables 21 and 22).

**Table 21**

| **Time hr** | **T C** | **P psig** | **252dc cc/hr** | **N2 sccm** | **H2 sccm** |
|---|---|---|---|---|---|
| 1 | 180 | 20.0 | 0.30 | 12 | 10 |
| 2 | 200 | 20.0 | 0.30 | 12 | 10 |
| 3 | 200 | 20.0 | 0.30 | 12 | 10 |
| 4 | 220 | 20.0 | 0.30 | 12 | 10 |
| 5 | 220 | 20.0 | 0.30 | 12 | 10 |
| 6 | 210 | 20.0 | 0.30 | 20 | 10 |
| 7 | 220 | 20.0 | 0.30 | 20 | 10 |
| 8 | 220 | 20.0 | 0.30 | 20 | 10 |
| 9 | 230 | 20.0 | 0.30 | 20 | 10 |
| 10 | 230 | 20.0 | 0.30 | 20 | 10 |
| 11 | 215 | 20.0 | 0.30 | 20 | 10 |
| 12 | 215 | 20.0 | 0.30 | 20 | 10 |
| 13 | 215 | 20.0 | 0.30 | 20 | 10 |
| 14 | 215 | 20.0 | 0.30 | 20 | 10 |
| 31 | 225 | 20.0 | 0.30 | 20 | 10 |
| 32 | 225 | 20.0 | 0.30 | 20 | 10 |
| 33 | 225 | 20.0 | 0.30 | 20 | 10 |
| 86 | 240 | 20.0 | 0.20 | 13 | 10 |
| 87 | 240 | 20.0 | 0.20 | 13 | 10 |
| 88 | 240 | 20.1 | 0.20 | 13 | 10 |
| 89 | 240 | 20.0 | 0.20 | 13 | 10 |
| 90 | 240 | 20.0 | 0.20 | 13 | 10 |
| 91 | 240 | 20.0 | 0.20 | 13 | 10 |
| 92 | 240 | 20.0 | 0.20 | 13 | 10 |
| 93 | 240 | 20.0 | 0.20 | 13 | 10 |
| 94 | 240 | 20.0 | 0.20 | 13 | 10 |
| 95 | 240 | 20.0 | 0.20 | 13 | 10 |
| 96 | 240 | 20.0 | 0.20 | 13 | 10 |
| 97 | 240 | 20.0 | 0.20 | 13 | 10 |
| 98 | 240 | 20.0 | 0.20 | 13 | 10 |
| 99 | 240 | 20.0 | 0.20 | 13 | 10 |
| 100 | 240 | 20.0 | 0.20 | 13 | 10 |
| 101 | 240 | 20.0 | 0.20 | 13 | 10 |
| 102 | 240 | 20.0 | 0.20 | 13 | 10 |

**Table 21 (Continued)**

| **Time hr** | **Mole Percents** | | | | | | | |
|---|---|---|---|---|---|---|---|---|
| | **propane** | **Propylene** | **1252zc** | **1260** | **1251** | **252dc** | **1250xd** | **others** |
| 1 | 0.15% | 0.00% | 9.01% | 0.23% | 0.36% | 89.49% | 0.16% | 0.61% |
| 2 | 0.19% | 0.00% | 18.33% | 0.45% | 0.54% | 79.57% | 0.10% | 0.82% |
| 3 | 0.18% | 0.00% | 17.66% | 0.40% | 0.56% | 80.35% | 0.07% | 0.78% |
| 4 | 0.24% | 0.94% | 36.28% | 1.17% | 1.29% | 60.00% | 0.00% | 0.07% |
| 5 | 0.26% | 1.15% | 38.77% | 1.19% | 1.53% | 56.86% | 0.00% | 0.24% |
| 6 | 0.00% | 0.00% | 26.61% | 0.00% | 0.66% | 72.51% | 0.00% | 0.23% |
| 7 | 0.00% | 0.00% | 36.72% | 0.00% | 1.31% | 60.91% | 0.00% | 1.06% |
| 8 | 0.00% | 0.00% | 35.19% | 0.00% | 1.32% | 62.45% | 0.00% | 1.04% |
| 9 | 0.00% | 0.00% | 33.91% | 0.56% | 1.94% | 62.88% | 0.00% | 0.72% |
| 10 | 0.00% | 0.00% | 42.01% | 0.65% | 2.27% | 54.30% | 0.00% | 0.77% |
| 11 | 0.00% | 0.00% | 20.59% | 0.00% | 0.87% | 78.03% | 0.18% | 0.34% |
| 12 | 0.00% | 0.00% | 14.28% | 0.00% | 0.89% | 84.62% | 0.12% | 0.09% |
| 13 | 0.00% | 0.00% | 13.37% | 0.00% | 0.98% | 85.35% | 0.00% | 0.29% |
| 14 | 0.00% | 0.00% | 13.56% | 0.00% | 1.08% | 85.06% | 0.00% | 0.30% |
| 31 | 0.00% | 0.00% | 18.08% | 0.00% | 2.08% | 79.84% | 0.00% | 0.00% |
| 32 | 0.00% | 0.00% | 21.97% | 0.00% | 2.31% | 75.72% | 0.00% | 0.00% |
| 33 | 0.00% | 0.00% | 19.18% | 0.00% | 2.10% | 78.72% | 0.00% | 0.00% |
| 86 | 0.00% | 0.00% | 34.37% | 0.00% | 2.75% | 61.37% | 0.00% | 1.50% |
| 87 | 0.00% | 0.00% | 34.28% | 0.00% | 3.04% | 61.98% | 0.00% | 0.70% |
| 88 | 0.00% | 0.00% | 33.35% | 0.00% | 3.36% | 62.66% | 0.00% | 0.63% |
| 89 | 0.00% | 0.00% | 29.87% | 0.00% | 3.50% | 65.79% | 0.00% | 0.85% |
| 90 | 0.00% | 0.00% | 33.31% | 0.00% | 3.78% | 62.16% | 0.00% | 0.74% |
| 91 | 0.00% | 0.00% | 32.01% | 0.00% | 3.82% | 63.41% | 0.00% | 0.76% |
| 92 | 0.00% | 0.00% | 28.26% | 0.00% | 3.93% | 67.09% | 0.00% | 0.72% |
| 93 | 0.00% | 0.00% | 31.40% | 0.00% | 3.78% | 64.56% | 0.00% | 0.26% |
| 94 | 0.00% | 0.00% | 44.44% | 0.00% | 3.94% | 51.28% | 0.00% | 0.34% |
| 95 | 0.00% | 0.00% | 45.82% | 0.00% | 3.17% | 51.02% | 0.00% | 0.00% |
| 96 | 0.00% | 0.00% | 21.59% | 0.00% | 2.61% | 75.68% | 0.00% | 0.13% |
| 97 | 0.00% | 0.00% | 28.34% | 0.00% | 4.06% | 66.97% | 0.00% | 0.63% |
| 98 | 0.00% | 0.00% | 25.66% | 0.00% | 3.80% | 69.75% | 0.00% | 0.80% |
| 99 | 0.00% | 0.00% | 24.82% | 0.00% | 3.76% | 70.65% | 0.00% | 0.76% |
| 100 | 0.00% | 0.00% | 26.28% | 0.00% | 3.90% | 68.93% | 0.00% | 0.88% |
| 101 | 0.00% | 0.00% | 25.08% | 0.00% | 3.94% | 70.12% | 0.00% | 0.85% |
| 102 | 0.00% | 0.00% | 24.49% | 0.00% | 3.64% | 71.34% | 0.00% | 0.54% |

**Table 22: GC analysis of product at 88 hours**

| **Compounds** | **FID area%** |
|---|---|
| 1261ze | 0.0317% |
| 1252zc | 33.1413% |
| 1243zf | 0.0111% |
| 262fc | 0.3783% |
| 1242zf | 3.3672% |
| 253 isomer | 0.0121% |
| 253fb | 0.0796% |
| 252dc | 62.7460% |
| 1241xb | 0.0100% |
| others | 0.2227% |

### Example 16: Reaction of HCFC-252dc with a Zinc

### Compound to form HFO-1252zc with ZnCl₂ as catalyst

0.81g Zn powder previously activated by aqueous HCl and dried under N₂ was added into an autoclave together with 1.33 g HCFC-252dc, 0.3 g ZnCl₂, 3.9 g dried methanol. After the autoclave was sealed, it was chilled to -40°C and then full vacuum was pulled to remove air. Then, the reaction mixture was heated to 100°C with agitation and agitated at 100°C for 16 hours. The pressure of autoclave increased to 468 psig at the end of the test which indicated the progress of reaction. After the reaction mixture was cooled to room temperature, both the vapor phase and liquid phase of the reaction mixture were analyzed by GC-MS-FID. The results are provided in Tables 23 and 24.

**Table 23: GC analysis of Vapor phase of reactor**

| **Compounds** | **GC-FID area%** |
|---|---|
| propylene | 0.0355% |
| 1252zc | 48.8204% |
| methanol | 40.4500% |
| 262fc | 0.5280% |
| 252dc | 8.6450% |

**Table 24: GC analysis of liquid phase of reactor**

| **Compounds** | **GC-FID area%** |
|---|---|
| Propylene | 0.0242% |
| propane | 0.0104% |
| 1252zc | 4.3681 % |
| E-1261ze | 0.0064% |
| methanol | 79.2087% |
| 253db | 0.0136% |
| 262fc | 0.0471% |
| acetone | 0.0349% |
| methyl acetate | 2.1540% |
| 1232xf | 0.0459% |
| 1,1-dimethoxyethane | 0.0096% |
| 252dc | 13.9872% |
| others | 0.0898% |

### Example 17: Reaction of HCFC-252dc with a Zinc

### Compound to form HFO-1252zc with TBAB as catalyst

0.9 g Zn powder previously activated by aqueous HCl and dried under N₂ was added into an autoclave together with 1.39 g HCFC-252dc, 0.14 g tetrabutylammonium bromide (TBAB), and 4.2 g dried methanol. After the autoclave was sealed, it was chilled to -40°C and then full vacuum was pulled to remove air. Then, the reaction mixture was heated to 100°C with agitation and agitated at 100°C for 10 hours. The pressure of the autoclave increased to 293 psig at the end of the test, which indicated the progress of reaction. After the reaction mixture was cooled to room temperature, both the vapor phase and liquid phase of the reaction mixture were analyzed by GC-MS-FID, and results are provided in Tables 25 and 26.

**Table 25: GC analysis of vapor phase of reactor**

| **Compounds** | **GC-FID area%** |
|---|---|
| ethane | 0.0016% |
| butene | 0.0009% |
| 1243zf | 0.0271% |
| propene | 0.1281% |
| propane | 0.3552% |
| 1252zc | 74.1391% |
| E-1261ze | 0.0417% |
| Z-1261ze | 0.0089% |
| methanol | 23.0273% |
| C3H7F | 0.0232% |
| butene | 0.0014% |
| 1233xf | 0.0355% |
| 253db | 0.0181% |
| 1242xc | 0.0928% |
| 262fc | 0.1613% |
| E-1251zb | 0.0068% |
| Z-1251zb | 0.0030% |
| 1251xe | 0.0037% |
| 252dc | 1.3772% |
| others | 0.5471% |

**Table 26: GC analysis of liquid phase of reactor**

| **Compounds** | **GC-FID area%** |
|---|---|
| 1243zf | 0.0021% |
| propene | 0.0040% |
| propane | 0.0064% |
| 1252zc | 3.5846% |
| E-1261ze | 0.0045% |
| Z-1261ze | 0.0013% |
| methanol | 93.3449% |
| 262fc | 0.0635% |
| 1251zb | 0.0108% |
| 1251xe | 0.0017% |
| 252dc | 2.7344% |
| methyl propionate | 0.1190% |
| others | 0.1228% |

### Example 18: Reaction of HCFC-252dc with a

### Zinc Compound to form HFO-1252zc with Zinc acetate as catalyst

0.8 g Zn powder previously activated by aqueous HCl and dried under N₂ was added into an autoclave together with 1.3 g HCFC-252dc, 0.23 g zinc acetate, and 3.9 g dried methanol. After the autoclave was sealed, it was chilled to -40°C and then full vacuum was pulled to remove air. Then, the reaction mixture was heated to 100°C with agitation and agitated at 100°C for 16 hours. The pressure of the autoclave increased to 255 psig at the end of the test, which indicated the progress of reaction. After the reaction mixture was cooled to room temperature, both the vapor phase and liquid phase of the reaction mixture were analyzed by GC-MS-FID, and the results are provided in Tables 27 and 28.

**Table 27: GC analysis of Vapor phase of reactor**

| **Compounds** | **GC-FID area%** |
|---|---|
| 1243zf | 0.1270% |
| propylene | 0.4968% |
| propane | 0.2529% |
| 1252zc | 60.0510% |
| E-1261ze | 0.0580% |
| methanol | 13.6227% |
| 1242xc | 1.8375% |
| 262fc | 0.1291% |
| acetone | 6.1918% |
| methyl acetate | 2.7506% |
| 252dc | 14.2252% |
| pentane | 0.1702% |
| others | 0.0872% |

**Table 28: GC analysis of liquid phase of reactor**

| **Compounds** | **GC-FID area%** |
|---|---|
| Propylene | 0.0298% |
| propane | 0.0125% |
| 1252zc | 4.7762% |
| E-1261ze | 0.0076% |
| Z-1261ze | 0.0022% |
| methanol | 77.7457% |
| 1242xc | 0.0106% |
| 262fc | 0.0303% |
| acetone | 0.0332% |
| Z-1251zb | 0.0025% |
| 1251xe | 0.0008% |
| methyl acetate | 2.2784% |
| 1233xf | 0.0567% |
| 1,1-dimethoxyethane | 0.0099% |
| 252dc | 14.9457% |
| pentane | 0.0063% |
| others | 0.0516% |

### Additional Embodiments:

Embodiment A compositions comprising, consisting essentially of, or consisting of HCFC-252dc and
(i) one or more of HCFO-1233xf, HCFO-1232xf, HFO-1243zf, HCFO-1242zf, HCFO-1242xc, HCFO-1242xf, HCFO-1241xb, HFO-1252zc, HCFO-1251zb (Z and E isomers), HCFO-1223xd, HCFC-262fc, HFC-263fb, HCFC-253 isomer, HCFC-253db, HFC-272fb, HCFC-243 isomer, propane and isopropanol; or
(ii) one or more of HCFO-1233xf, HCFO-1232xf, HCFO-1242xc and isopropanol; or
(iii) one or more of HCFO-1233xf, HCFO-1232xf, HFO-1252zc, HFC-272fb, HCFC-253db, HCFO-1242xc, HFC-263fb, HCFC-262fc, HCFC-243 isomer and HCFO-1223xd; or
(iv) one or more of propane, HFO-1243zf, HFO-1252zc, HFC-263fb, HCFO-1233xf, HCFO-1242zf, HCFO-1251zb (Z and E isomers), HCFC-253db, HCFO-1232xf and HCFO-1241xb; or
(v) one or more of propane, HFO-1243zf, HFO-1252zc, HFC-272fb, HCFC-253 isomer, HCFO-1233xf, HCFO-1242zf, HCFO-1242xf, HCFO-1251zb (Z and E isomers), HCFC-253db, HCFO-1232xf and HCFO-1241xb.

Embodiment B compositions comprising, consisting essentially of, or consisting of HFO-1252zc and
(i) one or more of E-HFO-1261ze, Z-HFO-1261ze, HCFO-1233xf, HCFO-1232xf, HFO-1243zf, HCFO-1242zf, HCFO-1242xc, HCFO-1242xf, HCFO-1241xb, HCFO-1250xd, E-HCFO-1251zb, Z-HCFO-1251zb, HCFO-1251xe, HCFO-1251zd, HCFC-262fc, HCFC-253 isomer, HCFC-253fb, HCFC-252dc, HCFC-253db, HCFC-262db, HFC-272fb, HFC-281, HCO-1260, ethane, butene, methane, pentane, propane, propylene, acetone, methanol, 1,1-dimethoxyethane, methyl propionate and methyl acetate; or
(ii) one or more of HFO-1243zf, HCFC-262fc, E-HCFO-1251zb, Z-HCFO-1251zb, HFC-272fb, HCFC-252dc, HCFO-1241xb, HCFC-262db, E-HFO-1261ze, Z-HFO-1261ze, methane, HCFO-1242zf, HCFO-1242xf, HCFC-253db and HCFO-1232xf; or
(iii) one or more of E-HFO-1261ze, Z-HFO-1261ze, HCO-1260, HCFO-1251zd, HCFC-252dc, HCFO-1241xb and HCFO-1250xd; or
(iv) one or more of propane, propylene, HCO-1260, HCFO-1250xd, E-HCFO-1251zb, Z-HCFO-1251zb, HCFO-1251xe, HCFO-1251zd, E-HFO-1261ze, Z-HFO-1261ze, HFO-1243zf, HCFO-1242zf, HCFC-253 isomer, HCFC-253fb, HCFC-252dc and HCFO-1241xb; or
(v) one or more of propylene, methanol, propane, acetone, methyl acetate, 1,1-dimethoxyethane, HCFC-262fc, HCFC-252dc, E-HFO-1261ze, HCFC-253db and HCFO-1232xf; or
(vi) one or more of ethane, butene, propene, propane, methanol, methyl propionate, HFO-1243zf, E-HFO-1261ze, Z-HFO-1261ze, HFC-281 (C₃H₇F), HCFO-1233xf, HCFC-253db, HCFO-1242xc, HCFC-262fc, E-HCFO-1251zb, Z-HCFO-1251zb and HCFO-1251xe; or
(vii) one or more of propylene, propane, methanol, acetone, methyl acetate, pentane, 1,1-dimethoxyethane, HFO-1243zf, E-HFO-1261ze, Z-HFO-1261ze, HCFO-1242xc, HCFC-262fc, Z-HCFO-1251zb, HCFO-1251xe and HCFO-1233xf.

The compositions of Embodiment A wherein the main components comprise at least two of HCFO-1232xf and HCFC-252dc.

The compositions of Embodiment B wherein the main components comprise at least two of HFO-1252zc and HFO-1261ze which is one of E-HFO-1261ze, Z-HFO-1261ze or mixtures of E-HFO-1261ze and Z-HFO-1261ze.

The compositions of Embodiment B wherein the main components comprise at least two of HFO-1252zc and HFO-1243zf.

Process Embodiment C wherein the HCFO-1232xf conversion to HCFC-252dc reaction temperature is in the range of about 20°C to about 200°C, and more preferably in the range about 40°C to about 160°C.

Process Embodiment D wherein the temperature for conversion of the HCFC-252dc to HFO-1252zc in the liquid phase by reaction with a metal is in the range of about 80°C to about 160°, and more preferably in the range of about 90° to about 140°C.

Process Embodiment E wherein the temperature for conversion of the HCFC-252dc to HFO-1252zc by a vapor phase hydrogenation reaction is in the range of about 160°C to about 300°C, and more preferably about 200°C to about 260°C, preferably about 200°C to about 240°C.

Process Embodiment F wherein the conversion of HCFO-1232xf to HCFC-252dc is conducted in the presence of a metal catalyst supported on one of carbon and Al₂O₃. Metal catalyst is selected from Cu, Ni, Pd and Pt. In one embodiment, the catalyst is preferably Pd. In some embodiments, the catalyst is Pd/C or Pd/Al₂O₃. In some embodiments, the catalyst is selected from 0.5% Pd/C to 5% Pd/C, 0.02% Pd/Al₂O₃ to 0.1% Pd/Al₂O₃.

Process Embodiment G wherein the conversion of HCFC-252dc to HFO-1252zc by reaction with a metal, such as zinc, is conducted in the presence of a catalyst which is selected from zinc salt or a phase transfer catalyst, such as TBAB.

Process Embodiment H wherein the conversion of HCFC-252dc to HFO-1252zc with H₂ is conducted in the presence of a metal catalyst which is selected from Cu, Au, preferably supported on one of carbon and Al₂O₃. In some embodiments, the catalyst is Cu/C or Au/Al₂O₃.

### Other Embodiments

Embodiment 1: A process of making 2,3-dichloro-3,3-difluoropropene (HCFO-1232xf) or co-produced HCFO-1232xf and 2-chloro-3,3,3-trifluoropropene (HCFO-1233xf), the process comprising: contacting 1,1,2,3-tetrachloro-1-propene (HCO-1230xa) and HF in the absence or presence of a catalyst.

Embodiment 2: The process of Embodiment 1, wherein the catalyst is selected from a supported or unsupported partially fluorinated metal oxide, a nickel-based alloy and a nickel-chromium-based alloy, preferably selected from fluorinated Al2O3, Hastelloy^{®} packing and Inconel^{™} packing.

Embodiment 3: The process of Embodiment 1 or Embodiment 2, wherein HCFO-1232xf and HCFO-1233xf are co-produced at a HCFO-1232xf:HCFO-1233xf ratio of greater than 1:1.

Embodiment 4: The process of Embodiment 1, wherein the catalyst comprises a supported or unsupported partially fluorinated metal oxide, preferably wherein the catalyst is selected from Cr2O3, CrCl3/C, Cr2O3/Al2O3, Cr2O3/AlF3, Cr2O3/carbon, CoCl2/Cr2O3/Al2O3, NiCl2/Cr2O3/Al2O3, CoCl2/AlF3, Zn/Al2O3, or NiCl2/AlF3.

Embodiment 5: The process of Embodiment 4, wherein HCFO-1232xf and HCFO-1233xf are co-produced at a HCFO-1232xf:HCFO-1233xf ratio of less than or equal to 1:1.

Embodiment 6: The process of any of Embodiments 1 to 5, wherein the process is in the vapor phase.

Embodiment 7: The process of any of Embodiments 1 to 6, wherein the contacting is at a temperature of from about 150°C to about 350°C, preferably from about 180°C to about 300°C.

Embodiment 8: The process of any of Embodiments 1 to 7, wherein a molar ratio of HF to HCO-1230xa is from about 10:1 to about 60:1, preferably from about 15:1 to about 50:1.

Embodiment 9: A process of making 2,3-dichloro-3,3-difluoropropene (HCFO-1232xf) comprising: contacting 2-chloro-3,3,3-trifluoropropene (HCFO-1233xf) and HCl in the presence of a catalyst.

Embodiment 10: The process of Embodiment 9, wherein the process is in the vapor phase.

Embodiment 11: The process of any of Embodiments 9 to 10, wherein a molar ratio of HCl to HCFO-1233xf is from about 10:1 to about 40:1, preferably from about 15:1 to about 35:1.

Embodiment 12: The process of any of Embodiments 9 to 11, wherein the contacting is at a temperature of from about 180°C to about 350°C, preferably from about 200°C to about 320°C.

Embodiment 13: The process of any of Embodiments 9 to 12, wherein the catalyst is selected from a fluorinated transition metal oxide or a transition metal halide catalyst.

Embodiment 14: A process comprising contacting (1) 2,3-dichloro-3,3-difluoropropene (HCFO-1232xf) or (2) a mixture of co-produced HCFO-1232xf and 2-chloro-3,3,3-trifluoropropene (HCFO-1233xf) with hydrogen in the presence of a catalyst to produce a reaction mixture comprising 1,2-dichloro-1,1-difluoropropane (HCFC-252dc).

Embodiment 15: The process of Embodiment 14, wherein the reaction mixture further comprises 2-chloro-1,1,1-trifluoropropane (HCFC-253db).

Embodiment 16: The process of any of Embodiments 14 or 15, wherein the process is in the vapor phase.

Embodiment 17: The process of Embodiment 16, wherein a molar ratio of H2 to HCFO-1232xf is from about 1:1 to about 15:1, preferably from about 1.5:1 to about 10:1.

Embodiment 18: The process of any of Embodiments 16 to 17, wherein the contacting is at a temperature of between about 20°C and about 200°C, preferably between about 20°C and about 150°C.

Embodiment 19: The process of any of Embodiments 16 to 18, wherein the catalyst comprises a transition metal preferably on a support, preferably wherein the catalyst is selected from the group consisting of Cu, Ni, Pd, Pd and combinations thereof, preferably on a support.

Embodiment 20: The process of Embodiment 19, wherein the catalyst loading is in the range of 0.005 wt.% to 3 wt.%.

Embodiment 21: The process of Embodiment 19, wherein the catalyst is Pd/C, preferably 0.5% Pd/C to 5% Pd/C.

Embodiment 22: The process of Embodiment 19, wherein the catalyst is Pd/Al2O3, preferably 0.02% Pd/Al2O3 to 0.1% Pd/Al2O3.

Embodiment 23: The process of any of Embodiments 14 or 15, wherein the process is in the liquid phase.

Embodiment 24: The process of Embodiment 23, wherein a molar ratio of H2 to HCFO-1232xf is from about 1:1 to about 5:1, preferably from about 1.2:1 to about 3:1.

Embodiment 25: The process of any of Embodiments 23 to 24, wherein the contacting is at a temperature of between about 20°C and about 150°C, preferably between about 40°C and about 120°C.

Embodiment 26: The process of any of Embodiments 23 to 25, wherein the catalyst comprises a transition metal preferably on a support, preferably wherein the catalyst is selected from the group consisting of Cu, Ni, Pd, Pd and combinations thereof, preferably on a support.

Embodiment 27: The process of Embodiment 26, wherein the catalyst loading is in the range of 0.005 wt.% to 3 wt.%.

Embodiment 28: The process of any of Embodiments 23 to 27, wherein the process is conducted in the presence of a solvent.

Embodiment 29: The process of Embodiment 28, wherein the solvent is a protic or aprotic organic solvent.

Embodiment 30: The process of Embodiment 29, wherein the solvent is selected from the group consisting of selected from an alcohol, dimethyl sulfoxide (DMSO), dimethyl acetamide (DMAC), dimethyl formamide (DMF), tetrahydrofuran (THF) and dioxan.

Embodiment 31: A process of making 1,2-dichloro-1,1-difluoropropane (HCFC-252dc) comprising:
contacting 2-chloro-3,3,3-trifluoropropene (HCFO-1233xf) and hydrogen chloride in the presence of a catalyst to form 2,3-dichloro-3,3-difluoropropene (HCFO-1232xf), and
contacting the HCFO-1232xf and hydrogen in the presence of another catalyst to make HCFC-252dc.

Embodiment 32: A process of making 1,1-difluoropropene (HFO-1252zc) comprising contacting 1,2-dichloro-1,1-difluoropropane (HCFC-252dc) with hydrogen in the vapor phase in the presence of a catalyst.

Embodiment 33: The process of Embodiment 32, wherein the catalyst comprises a metal selected from the group consisting of Cu, Au, and combinations thereof, the metal catalyst preferably being supported on one of carbon, silicon carbide and Al2O3.

Embodiment 34: The process of Embodiment 33, wherein the catalyst loading ranges from about 0.1% to about 10%.

Embodiment 35: The process of any of Embodiments 33 to 34, wherein the reaction temperature is in the range of about 160°C to about 500°C, preferably about 250°C to about 450°C.

Embodiment 36: The process of any of Embodiments 32 to 35, wherein the process is conducted at a pressure of between ≥ 0 to ≤ 250psig.

Embodiment 37: The process of any of Embodiments 32 to 36, wherein the process is conducted in the presence of a diluent.

Embodiment 38: A process of making 1,1-difluoropropene (HFO-1252zc) comprising contacting 1,2-dichloro-1,1-difluoropropane (HCFC-252dc) with a metal, in the liquid phase in the presence of a catalyst.

Embodiment 39: The process of Embodiment 38, wherein the metal is a reactive metal.

Embodiment 40: The process of any of Embodiments 38 to 39, wherein the metal is selected from the group consisting of Zn, Mg, Cu, Fe and combinations thereof.

Embodiment 41: The process of any of Embodiments 38 to 40, wherein the reaction is conducted in the presence of a protic or aprotic solvent, acetic acid or acetic anhydride.

Embodiment 42: The process of Embodiment 41, wherein the solvent is selected from the group consisting of an alcohol, or one of dimethyl sulfoxide (DMSO), dimethyl acetamide (DMAC), dimethyl formamide (DMF), tetrahydrofuran (THF), pyridine and dioxane.

Embodiment 43: The process of Embodiment 42, wherein the alcohol is selected from the group consisting of methanol, ethanol, isopropanol.

Embodiment 44: The process of any of Embodiments 38 to 43, wherein the liquid phase reaction is conducted under autogenous pressure.

Embodiment 45: The process of any of Embodiments 38 to 44, wherein the liquid phase reaction is conducted at temperatures between about 50°C to about 160°, preferably in the range of about 90° to about 140°C.

Embodiment 46: The process of any of Embodiments 38 to 45, wherein the catalyst is selected from the group consisting of a metal halide, a zinc salt, a phase transfer catalyst and combinations thereof.

Embodiment 47: The process of Embodiment 46, wherein the catalyst is selected from the group consisting of zinc chloride, zinc acetate, ammonium salt, phosphonium salts, quaternary ammonium salts such as tetrabutylammonium salt or Aliquat 336.

Embodiment 48: A process of making 1,1-difluoropropene (HFO-1252zc) comprising:
contacting 1,1,2,3-tetrachloro-1-propene (HCO-1230xa) and HF in the presence of a first catalyst to form 2,3-dichloro-3,3-difluoropropene (HCFO-1232xf);
contacting the HCFO-1232xf and hydrogen in the presence of a second catalyst to make 1,2-dichloro-1,1-difluoropropane (HCFC-252dc); and
contacting the HCFC-252dc with hydrogen in the presence of a third catalyst to make HFO-1252zc, or contacting the HCFC-252dc and a metal in the presence of a fourth catalyst to make HFO-1252zc.

Embodiment 49: A process of making 1,1-difluoropropene (HFO-1252zc) comprising:
providing a 2-chloro-3,3,3-trifluoropropene (HCFO-1233xf) feed,
contacting the HCFO-1233xf feed and HCl in the presence of a first catalyst to make 2,3-dichloro-3,3-difluoropropene (HCFO-1232xf),
contacting the HCFO-1232xf and hydrogen in the presence of a second catalyst to make 1,2-dichloro-1,1-difluoropropane (HCFC-252dc), and
contacting the HCFC-252dc with hydrogen in the presence of third catalyst to make HFO-1252zc, or contacting HCFC-252dc and a metal in the presence of a fourth catalyst to make HFO-1252zc.

Embodiment 50: The process of Embodiment 49, further comprising contacting 1,1,2,3-tetrachloro-1-propene (HCO-1230xa) and HF in the presence of a catalyst to form a reaction mixture comprising the HCFO-1233xf feed.

Embodiment 51: A composition comprising: (i) one or more compounds selected from the group consisting of of HFO-1252zc, HCFC-252dc and HCFC-262fc, and (ii) one or more additional compounds selected from the group consisting of HFO-1252zc, HCFC-252dc, HFO- HFC-263fb, HFC-253db, HCFO-1233xf, HFC-272fb, HFC-152a and HFO-1242xc.

Embodiment 52: The composition of Embodiment 51, the composition comprising HFO-1252zc, HCFC-252dc and one or more additional compounds selected from the group consisting of HFC-263fb, HFC-253db, 1233xf, HFC-272fb, HCFC-262fc, HFC-152a and HFO-1242xc.

Embodiment 53: A composition comprising HCFC-252dc and one or more additional compounds selected from the group consisting of HCFO-1233xf, HCFO-1232xf, HFO-1243zf, HCFO-1242zf, HCFO-1242xc, HCFO-1242xf, HCFO-1241xb, HFO-1252zc, HCFO-1251zb (Z and E isomers), HCFO-1251xe, HCFO-1223xd, HCFC-262fc, HFC-263fb, HCFC-253 isomer, HCFC-253fb, HCFC-252dc, HCFC-253db, HCFC-262db, HFC-272fb, HCFC-243 isomer, propane and isopropanol.

Embodiment 54: A composition comprising HFO-1252zc and one or more additional compounds selected from the group consisting of E-HFO-1261ze, Z-HFO-1261ze, HCFO-1233xf, HCFO-1232xf, HFO-1243zf, HCFO-1242zf, HCFO-1242xc, HCFO-1242xf, HCFO-1241xb , HFO-1252zc, E-HCFO-1251zb, Z-HCFO-1251zb, HCFO-1251xe, HCFO-1223xd, HCFC-262fc, HFC-263fb, HCFC-253 isomer, HCFC-253fb, HCFC-252dc, HCFC-253db, HCFC-262db, HFC-272fb, HCFC-243 isomer,HFC-281, propane, propylene, acetone, methanol, isopropanol and methyl acetate.

Embodiment 55: A composition comprising HCFC-252dc and one or more additional compounds selected from the group consisting of HCFO-1233xf, HCFO-1232xf, HFO-1243zf, HCFO-1242zf, HCFO-1242xc, HCFO-1242xf, HCFO-1241xb, HFO-1252zc, HCFO-1251zb (Z and E isomers), HCFO-1223xd, HCFC-262fc, HFC-263fb, HCFC-253 isomer, HCFC-253db, HFC-272fb, HCFC-243 isomer, propane and isopropanol.

Embodiment 56: A composition comprising HCFC-252dc and one or more additional compounds selected from the group consisting of HCFO-1233xf, HCFO-1232xf, HCFO-1242xc and isopropanol.

Embodiment 57: A composition comprising HCFC-252dc and one or more additional compounds selected from the group consisting of HCFO-1233xf, HCFO-1232xf, HFO-1252zc, HFC-272fb, HCFC-253db, HCFO-1242xc, HFC-263fb, HCFC-262fc, HCFC-243 isomer and HCFO-1223xd.

Embodiment 58: A composition comprising HCFC-252dc and one or more additional compounds selected from the group consisting of propane, HFO-1243zf, HFO-1252zc, HFC-263fb, HCFO-1233xf, HCFO-1242zf, HCFO-1251zb (Z and E isomers), HCFC-253db, HCFO-1232xf and HCFO-1241xb.

Embodiment 59: A composition comprising HCFC-252dc and one or more additional compounds selected from the group consisting of propane, HFO-1243zf, HFO-1252zc, HFC-272fb, HCFC-253 isomer, HCFO-1233xf, HCFO-1242zf, HCFO-1242xf, HCFO-1251zb (Z and E isomers), HCFC-253db, HCFO-1232xf and HCFO-1241xb.

Embodiment 60: A composition comprising HFO-1252zc and one or more additional compounds selected from the group consisting of E-HFO-1261ze, Z-HFO-1261ze, HCFO-1233xf, HCFO-1232xf, HFO-1243zf, HCFO-1242zf, HCFO-1242xc, HCFO-1242xf, HCFO-1241xb, HCFO-1250xd, E-HCFO-1251zb, Z-HCFO-1251zb, HCFO-1251xe, HCFO-1251zd, HCFC-262fc, HCFC-253 isomer, HCFC-253fb, HCFC-252dc, HCFC-253db, HCFC-262db, HFC-272fb, HFC-281, HCO-1260, methanol, ethane, butene, methane, pentane, propane, propylene, acetone, methanol, 1,1-dimethoxyethane, methyl propionate and methyl acetate.

Embodiment 61: A composition comprising HFO-1252zc and one or more additional compounds selected from the group consisting of HFO-1243zf, HCFC-262fc, E-HCFO-1251zb, Z-HCFO-1251zb, HFC-272fb, HCFC-252dc, HCFO-1241xb, HCFC-262db, E-HFO-1261ze, Z-HFO-1261ze, methane, HCFO-1242zf, HCFO-1242xf, HCFC-253db and HCFO-1232xf.

Embodiment 62: A composition comprising HFO-1252zc and one or more additional compounds selected from the group consisting of E-HFO-1261ze, Z-HFO-1261ze, HCO-1260, HCFO-1251zd, HCFC-252dc, HCFO-1241xb and HCFO-1250xd.

Embodiment 63: A composition comprising HFO-1252zc and one or more additional compounds selected from the group consisting of propane, propylene, HCO-1260, HCFO-1250xd, E-HCFO-1251zb, Z-HCFO-1251zb, HCFO-1251xe, HCFO-1251zd, E-HFO-1261ze, Z-HFO-1261ze, HFO-1243zf, HCFO-1242zf, HCFC-253 isomer, HCFC-253fb, HCFC-252dc and HCFO-1241xb.

Embodiment 64: A composition comprising HFO-1252zc and one or more additional compounds selected from the group consisting of propylene, methanol, propane, acetone, methyl acetate, 1,1-dimethoxyethane, HCFC-262fc, HCFC-252dc, E-HFO-1261ze, HCFC-253db and HCFO-1232xf.

Embodiment 65: A composition comprising HFO-1252zc and one or more additional compounds selected from the group consisting of ethane, butene, propene, propane, methanol, methyl propionate, HFO-1243zf, E-HFO-1261ze, Z-HFO-1261ze, HFC-281 (C3H7F), HCFO-1233xf, HCFC-253db, HCFO-1242xc, HCFC-262fc, E-HCFO-1251zb, Z-HCFO-1251zb and HCFO-1251xe.

Embodiment 66: A composition comprising HFO-1252zc and one or more additional compounds selected from the group consisting of propylene, propane, methanol, acetone, methyl acetate, pentane, 1,1-dimethoxyethane, HFO-1243zf, E-HFO-1261ze, Z-HFO-1261ze, HCFO-1242xc, HCFC-262fc, Z-HCFO-1251zb, HCFO-1251xe and HCFO-1233xf.

Although certain aspects, embodiments and principals have been described above, it is understood that this description is made only way of example and not as limitation of the scope of the invention or appended claims. The foregoing various aspects, embodiments and principals can be used alone and in combinations with each other.
The following numbered clauses also form part of the description
Clause 1. A process of making 2,3-dichloro-3,3-difluoropropene (HCFO-1232xf) or co-produced HCFO-1232xf and 2-chloro-3,3,3-trifluoropropene (HCFO-1233xf), the process comprising: contacting 1,1,2,3-tetrachloro-1-propene (HCO-1230xa) and HF in the absence or presence of a catalyst.
Clause 2. The process of clause 1, wherein the catalyst is selected from a supported or unsupported partially fluorinated metal oxide, a nickel-based alloy and a nickel-chromium-based alloy, preferably selected from fluorinated Al₂O₃, Hastelloy^{®} packing and Inconel^{™} packing.
Clause 3. The process of clause 1 or clause 2, wherein HCFO-1232xf and HCFO-1233xf are co-produced at a HCFO-1232xf:HCFO-1233xf ratio of greater than 1:1.
Clause 4. The process of clause 1, wherein the catalyst comprises a supported or unsupported partially fluorinated metal oxide, preferably wherein the catalyst is selected from Cr₂O₃, CrCl₃/C, Cr₂O₃/Al₂O₃, Cr₂O₃/AlF₃, Cr₂O₃/carbon, CoCl₂/Cr₂O₃/Al₂O₃, NiCl₂/Cr₂O₃/Al₂O₃, CoCl₂/AlF₃, Zn/Al₂O₃, or NiCl₂/AlF₃.
Clause 5. The process of clause 4, wherein HCFO-1232xf and HCFO-1233xf are co-produced at a HCFO-1232xf:HCFO-1233xf ratio of less than or equal to 1:1.
Clause 6. The process of any of clauses 1 to 5, wherein the process is in the vapor phase.
Clause 7. The process of any of clauses 1 to 6, wherein the contacting is at a temperature of from about 150°C to about 350°C, preferably from about 180°C to about 300°C.
Clause 8. The process of any of clauses 1 to 7, wherein a molar ratio of HF to HCO-1230xa is from about 10:1 to about 60:1, preferably from about 15:1 to about 50:1.
Clause 9. A process of making 2,3-dichloro-3,3-difluoropropene (HCFO-1232xf) comprising: contacting 2-chloro-3,3,3-trifluoropropene (HCFO-1233xf) and HCl in the presence of a catalyst.
Clause 10. The process of clause 9, wherein the process is in the vapor phase.
Clause 11. The process of any of clauses 9 to 10, wherein a molar ratio of HCl to HCFO-1233xf is from about 10:1 to about 40:1, preferably from about 15:1 to about 35:1.
Clause 12. The process of any of clauses 9 to 11, wherein the contacting is at a temperature of from about 180°C to about 350°C, preferably from about 200°C to about 320°C.
Clause 13. The process of any of clauses 9 to 12, wherein the catalyst is selected from a fluorinated transition metal oxide or a transition metal halide catalyst.
Clause 14. A process comprising contacting (1) 2,3-dichloro-3,3-difluoropropene (HCFO-1232xf) or (2) a mixture of co-produced HCFO-1232xf and 2-chloro-3,3,3-trifluoropropene (HCFO-1233xf) with hydrogen in the presence of a catalyst to produce a reaction mixture comprising 1,2-dichloro-1,1-difluoropropane (HCFC-252dc).
Clause 15. The process of clause 14, wherein the reaction mixture further comprises 2-chloro-1,1,1-trifluoropropane (HCFC-253db).
Clause 16. The process of any of clauses 14 or 15, wherein the process is in the vapor phase.
Clause 17. The process of clause 16, wherein a molar ratio of H₂ to HCFO-1232xf is from about 1:1 to about 15:1, preferably from about 1.5:1 to about 10:1.
Clause 18. The process of any of clauses 16 to 17, wherein the contacting is at a temperature of between about 20°C and about 200°C, preferably between about 20°C and about 150°C.
Clause 19. The process of any of clauses 16 to 18, wherein the catalyst comprises a transition metal preferably on a support, preferably wherein the catalyst is selected from the group consisting of Cu, Ni, Pd, Pd and combinations thereof, preferably on a support.
Clause 20. The process of clause 19, wherein the catalyst loading is in the range of 0.005 wt.% to 3 wt.%.
Clause 21. The process of clause 19, wherein the catalyst is Pd/C, preferably 0.5% Pd/C to 5% Pd/C.
Clause 22. The process of clause 19, wherein the catalyst is Pd/Al₂O₃, preferably 0.02% Pd/Al₂O₃ to 0.1% Pd/Al₂O₃.
Clause 23. The process of any of clauses 14 or 15, wherein the process is in the liquid phase.
Clause 24. The process of clause 23, wherein a molar ratio of H₂ to HCFO-1232xf is from about 1:1 to about 5:1, preferably from about 1.2:1 to about 3:1.
Clause 25. The process of any of clauses 23 to 24, wherein the contacting is at a temperature of between about 20°C and about 150°C, preferably between about 40°C and about 120°C.
Clause 26. The process of any of clauses 23 to 25, wherein the catalyst comprises a transition metal preferably on a support, preferably wherein the catalyst is selected from the group consisting of Cu, Ni, Pd, Pd and combinations thereof, preferably on a support.
Clause 27. The process of clause 26, wherein the catalyst loading is in the range of 0.005 wt.% to 3 wt.%.
Clause 28. The process of any of clauses 23 to 27, wherein the process is conducted in the presence of a solvent.
Clause 29. The process of clause 28, wherein the solvent is a protic or aprotic organic solvent.
Clause 30. The process of clause 29, wherein the solvent is selected from the group consisting of selected from an alcohol, dimethyl sulfoxide (DMSO), dimethyl acetamide (DMAC), dimethyl formamide (DMF), tetrahydrofuran (THF) and dioxan.
Clause 31. A process of making 1,2-dichloro-1,1-difluoropropane (HCFC-252dc) comprising:
   a. contacting 2-chloro-3,3,3-trifluoropropene (HCFO-1233xf) and hydrogen chloride in the presence of a catalyst to form 2,3-dichloro-3,3-difluoropropene (HCFO-1232xf), and
   b. contacting the HCFO-1232xf and hydrogen in the presence of another catalyst to make HCFC-252dc.
Clause 32. A process of making 1,1-difluoropropene (HFO-1252zc) comprising contacting 1,2-dichloro-1,1-difluoropropane (HCFC-252dc) with hydrogen in the vapor phase in the presence of a catalyst.
Clause 33. The process of clause 32, wherein the catalyst comprises a metal selected from the group consisting of Cu, Au, and combinations thereof, the metal catalyst preferably being supported on one of carbon, silicon carbide and Al2O3.
Clause 34. The process of clause 33, wherein the catalyst loading ranges from about 0.1% to about 10%.
Clause 35. The process of any of clauses 33 to 34, wherein the reaction temperature is in the range of about 160°C to about 500°C, preferably about 250°C to about 450°C.
Clause 36. The process of any of clauses 32 to 35, wherein the process is conducted at a pressure of between ≥ 0 to ≤ 250psig.
Clause 37. The process of any of clauses 32 to 36, wherein the process is conducted in the presence of a diluent.
Clause 38. A process of making 1,1-difluoropropene (HFO-1252zc) comprising contacting 1,2-dichloro-1,1-difluoropropane (HCFC-252dc) with a metal, in the liquid phase in the presence of a catalyst.
Clause 39. The process of clause 38, wherein the metal is a reactive metal.
Clause 40. The process of any of clauses 38 to 39, wherein the metal is selected from the group consisting of Zn, Mg, Cu, Fe and combinations thereof.
Clause 41. The process of any of clauses 38 to 40, wherein the reaction is conducted in the presence of a protic or aprotic solvent, acetic acid or acetic anhydride.
Clause 42. The process of clause 41, wherein the solvent is selected from the group consisting of an alcohol, or one of dimethyl sulfoxide (DMSO), dimethyl acetamide (DMAC), dimethyl formamide (DMF), tetrahydrofuran (THF), pyridine and dioxane.
Clause 43. The process of clause 42, wherein the alcohol is selected from the group consisting of methanol, ethanol, isopropanol.
Clause 44. The process of any of clauses 38 to 43, wherein the liquid phase reaction is conducted under autogenous pressure.
Clause 45. The process of any of clauses 38 to 44, wherein the liquid phase reaction is conducted at temperatures between about 50°C to about 160°, preferably in the range of about 90° to about 140°C.
Clause 46. The process of any of clauses 38 to 45, wherein the catalyst is selected from the group consisting of a metal halide, a zinc salt, a phase transfer catalyst and combinations thereof.
Clause 47. The process of clause 46, wherein the catalyst is selected from the group consisting of zinc chloride, zinc acetate, ammonium salt, phosphonium salts, quaternary ammonium salts such as tetrabutylammonium salt or Aliquat 336.
Clause 48. A process of making 1,1-difluoropropene (HFO-1252zc) comprising:
   i. contacting 1,1,2,3-tetrachloro-1-propene (HCO-1230xa) and HF in the presence of a first catalyst to form 2,3-dichloro-3,3-difluoropropene (HCFO-1232xf);
   ii. contacting the HCFO-1232xf and hydrogen in the presence of a second catalyst to make 1,2-dichloro-1,1-difluoropropane (HCFC-252dc); and
   iii. contacting the HCFC-252dc with hydrogen in the presence of a third catalyst to make HFO-1252zc, or contacting the HCFC-252dc and a metal in the presence of a fourth catalyst to make HFO-1252zc.
Clause 49. A process of making 1,1-difluoropropene (HFO-1252zc) comprising:
   i. providing a 2-chloro-3,3,3-trifluoropropene (HCFO-1233xf) feed,
   ii. contacting the HCFO-1233xf feed and HCl in the presence of a first catalyst to make 2,3-dichloro-3,3-difluoropropene (HCFO-1232xf),
   iii. contacting the HCFO-1232xf and hydrogen in the presence of a second catalyst to make 1,2-dichloro-1,1-difluoropropane (HCFC-252dc), and
   iv. contacting the HCFC-252dc with hydrogen in the presence of third catalyst to make HFO-1252zc, or contacting HCFC-252dc and a metal in the presence of a fourth catalyst to make HFO-1252zc.
Clause 50. The process of clause 49, further comprising contacting 1,1,2,3-tetrachloro-1-propene (HCO-1230xa) and HF in the presence of a catalyst to form a reaction mixture comprising the HCFO-1233xf feed.
Clause 51. A composition comprising: (i) one or more compounds selected from the group consisting of HFO-1252zc, HCFC-252dc and HCFC-262fc, and (ii) one or more additional compounds selected from the group consisting of HFO-1252zc, HCFC-252dc, HFO- HFC-263fb, HFC-253db, HCFO-1233xf, HFC-272fb, HFC-152a and HFO-1242xc.
Clause 52. The composition of clause 51, the composition comprising HFO-1252zc, HCFC-252dc and one or more additional compounds selected from the group consisting of HFC-263fb, HFC-253db, 1233xf, HFC-272fb, HCFC-262fc, HFC-152a and HFO-1242xc.
Clause 53. A composition comprising HCFC-252dc and one or more additional compounds selected from the group consisting of HCFO-1233xf, HCFO-1232xf, HFO-1243zf, HCFO-1242zf, HCFO-1242xc, HCFO-1242xf, HCFO-1241xb, HFO-1252zc, HCFO-1251zb (Z and E isomers), HCFO-1251xe, HCFO-1223xd, HCFC-262fc, HFC-263fb, HCFC-253 isomer, HCFC-253fb, HCFC-252dc, HCFC-253db, HCFC-262db, HFC-272fb, HCFC-243 isomer, propane and isopropanol.
Clause 54. A composition comprising HFO-1252zc and one or more additional compounds selected from the group consisting of E-HFO-1261ze, Z-HFO-1261ze, HCFO-1233xf, HCFO-1232xf, HFO-1243zf, HCFO-1242zf, HCFO-1242xc, HCFO-1242xf, HCFO-1241xb , HFO-1252zc, E-HCFO-1251zb, Z-HCFO-1251zb, HCFO-1251xe, HCFO-1223xd, HCFC-262fc, HFC-263fb, HCFC-253 isomer, HCFC-253fb, HCFC-252dc, HCFC-253db, HCFC-262db, HFC-272fb, HCFC-243 isomer,HFC-281, propane, propylene, acetone, methanol, isopropanol and methyl acetate.
Clause 55. A composition comprising HCFC-252dc and one or more additional compounds selected from the group consisting of HCFO-1233xf, HCFO-1232xf, HFO-1243zf, HCFO-1242zf, HCFO-1242xc, HCFO-1242xf, HCFO-1241xb, HFO-1252zc, HCFO-1251zb (Z and E isomers), HCFO-1223xd, HCFC-262fc, HFC-263fb, HCFC-253 isomer, HCFC-253db, HFC-272fb, HCFC-243 isomer, propane and isopropanol.
Clause 56. A composition comprising HCFC-252dc and one or more additional compounds selected from the group consisting of HCFO-1233xf, HCFO-1232xf, HCFO-1242xc and isopropanol.
Clause 57. A composition comprising HCFC-252dc and one or more additional compounds selected from the group consisting of HCFO-1233xf, HCFO-1232xf, HFO-1252zc, HFC-272fb, HCFC-253db, HCFO-1242xc, HFC-263fb, HCFC-262fc, HCFC-243 isomer and HCFO-1223xd.
Clause 58. A composition comprising HCFC-252dc and one or more additional compounds selected from the group consisting of propane, HFO-1243zf, HFO-1252zc, HFC-263fb, HCFO-1233xf, HCFO-1242zf, HCFO-1251zb (Z and E isomers), HCFC-253db, HCFO-1232xf and HCFO-1241xb.
Clause 59. A composition comprising HCFC-252dc and one or more additional compounds selected from the group consisting of propane, HFO-1243zf, HFO-1252zc, HFC-272fb, HCFC-253 isomer, HCFO-1233xf, HCFO-1242zf, HCFO-1242xf, HCFO-1251zb (Z and E isomers), HCFC-253db, HCFO-1232xf and HCFO-1241xb.
Clause 60. A composition comprising HFO-1252zc and one or more additional compounds selected from the group consisting of E-HFO-1261ze, Z-HFO-1261ze, HCFO-1233xf, HCFO-1232xf, HFO-1243zf, HCFO-1242zf, HCFO-1242xc, HCFO-1242xf, HCFO-1241xb, HCFO-1250xd, E-HCFO-1251zb, Z-HCFO-1251zb, HCFO-1251xe, HCFO-1251zd, HCFC-262fc, HCFC-253 isomer, HCFC-253fb, HCFC-252dc, HCFC-253db, HCFC-262db, HFC-272fb, HFC-281, HCO-1260, methanol, ethane, butene, methane, pentane, propane, propylene, acetone, methanol, 1,1-dimethoxyethane, methyl propionate and methyl acetate.
Clause 61. A composition comprising HFO-1252zc and one or more additional compounds selected from the group consisting of HFO-1243zf, HCFC-262fc, E-HCFO-1251zb, Z-HCFO-1251zb, HFC-272fb, HCFC-252dc, HCFO-1241xb, HCFC-262db, E-HFO-1261ze, Z-HFO-1261ze, methane, HCFO-1242zf, HCFO-1242xf, HCFC-253db and HCFO-1232xf.
Clause 62. A composition comprising HFO-1252zc and one or more additional compounds selected from the group consisting of E-HFO-1261ze, Z-HFO-1261ze, HCO-1260, HCFO-1251zd, HCFC-252dc, HCFO-1241xb and HCFO-1250xd.
Clause 63. A composition comprising HFO-1252zc and one or more additional compounds selected from the group consisting of propane, propylene, HCO-1260, HCFO-1250xd, E-HCFO-1251zb, Z-HCFO-1251zb, HCFO-1251xe, HCFO-1251zd, E-HFO-1261ze, Z-HFO-1261ze, HFO-1243zf, HCFO-1242zf, HCFC-253 isomer, HCFC-253fb, HCFC-252dc and HCFO-1241xb.
Clause 64. A composition comprising HFO-1252zc and one or more additional compounds selected from the group consisting of propylene, methanol, propane, acetone, methyl acetate, 1,1-dimethoxyethane, HCFC-262fc, HCFC-252dc, E-HFO-1261ze, HCFC-253db and HCFO-1232xf.
Clause 65. A composition comprising HFO-1252zc and one or more additional compounds selected from the group consisting of ethane, butene, propene, propane, methanol, methyl propionate, HFO-1243zf, E-HFO-1261ze, Z-HFO-1261ze, HFC-281 (C₃H₇F), HCFO-1233xf, HCFC-253db, HCFO-1242xc, HCFC-262fc, E-HCFO-1251zb, Z-HCFO-1251zb and HCFO-1251xe.
Clause 66. A composition comprising HFO-1252zc and one or more additional compounds selected from the group consisting of propylene, propane, methanol, acetone, methyl acetate, pentane, 1,1-dimethoxyethane, HFO-1243zf, E-HFO-1261ze, Z-HFO-1261ze, HCFO-1242xc, HCFC-262fc, Z-HCFO-1251zb, HCFO-1251xe and HCFO-1233xf.

## Claims

1. A process of making 1,1-difluoropropene (HFO-1252zc) comprising contacting 1,2-dichloro-1,1-difluoropropane (HCFC-252dc) with either:
(a) hydrogen in the vapor phase in the presence of a catalyst; or
(b) a metal, in the liquid phase in the presence of a catalyst.

2. The process of claim 1, wherein the process is according to option (a) and wherein the catalyst comprises a metal selected from the group consisting of Cu, Au, and combinations thereof, the metal catalyst preferably being supported on one of carbon, silicon carbide and Al₂O₃.

3. The process of claim 2, wherein the catalyst loading ranges from about 0.1% to about 10%.

4. The process of any of claims 1 to 3, wherein the process is according to option (a) and wherein the reaction temperature is in the range of about 160°C to about 500°C, preferably about 250°C to about 450°C.

5. The process of any of claims 1 to 4, wherein the process is according to option (a) and wherein the process is conducted at a pressure of between ≥ 0 to ≤ 1724 kPa gauge (≥ 0 to ≤ 250 psig).

6. The process of any of claims 1 to 5, wherein the process is according to option (a) and wherein the process is conducted in the presence of a diluent.

7. The process of claim 1, wherein the process is according to option (b) and wherein the metal is a reactive metal.

8. The process of claim 1 or 7, wherein the process is according to option (b) and wherein the metal is selected from the group consisting of Zn, Mg, Cu, Fe and combinations thereof.

9. The process of any of claims 1, 7 or 8, wherein the process is according to option (b) and wherein the reaction is conducted in the presence of a protic or aprotic solvent, acetic acid or acetic anhydride;
optionally wherein the solvent is selected from the group consisting of an alcohol, or one of dimethyl sulfoxide (DMSO), dimethyl acetamide (DMAC), dimethyl formamide (DMF), tetrahydrofuran (THF), pyridine and dioxane;
optionally wherein the alcohol is selected from the group consisting of methanol, ethanol, isopropanol.

10. The process of any of claims 1, or 7 to 9, wherein the process is according to option (b) and wherein the liquid phase reaction is conducted under autogenous pressure.

11. The process of any of claims 1, or 7 to 10, wherein the process is according to option (b) and wherein the liquid phase reaction is conducted at temperatures between about 50°C to about 160°C, preferably in the range of about 90°C to about 140°C.

12. The process of any of claims 1, or 7 to 11, wherein the process is according to option (b) and wherein the catalyst is selected from the group consisting of a metal halide, a zinc salt, a phase transfer catalyst and combinations thereof;
optionally wherein the catalyst is selected from the group consisting of zinc chloride, zinc acetate, ammonium salt, phosphonium salts, quaternary ammonium salts such as tetrabutylammonium salt or Aliquat 336.

13. A process of making 1,1-difluoropropene (HFO-1252zc) comprising:
i. contacting 1,1,2,3-tetrachloro-1-propene (HCO-1230xa) and HF in the presence of a first catalyst to form 2,3-dichloro-3,3-difluoropropene (HCFO-1232xf);
ii. contacting the HCFO-1232xf and hydrogen in the presence of a second catalyst to make 1,2-dichloro-1,1-difluoropropane (HCFC-252dc); and
iii. contacting the HCFC-252dc with hydrogen in the presence of a third catalyst to make HFO-1252zc, or contacting the HCFC-252dc and a metal in the presence of a fourth catalyst to make HFO-1252zc.

14. A process of making 1,1-difluoropropene (HFO-1252zc) comprising:
i. providing a 2-chloro-3,3,3-trifluoropropene (HCFO-1233xf) feed,
ii. contacting the HCFO-1233xf feed and HCl in the presence of a first catalyst to make 2,3-dichloro-3,3-difluoropropene (HCFO-1232xf),
iii. contacting the HCFO-1232xf and hydrogen in the presence of a second catalyst to make 1,2-dichloro-1,1-difluoropropane (HCFC-252dc), and
iv. contacting the HCFC-252dc with hydrogen in the presence of third catalyst to make HFO-1252zc, or contacting HCFC-252dc and a metal in the presence of a fourth catalyst to make HFO-1252zc.

15. The process of claim 14, further comprising contacting 1,1,2,3-tetrachloro-1-propene (HCO-1230xa) and HF in the presence of a catalyst to form a reaction mixture comprising the HCFO-1233xf feed.
